# EUROPEAN PATENT SPECIFICATION

(11) **EP 2 937 417 B1**
(45) Date of publication and mention of the grant of the patent: **20.02.2019**
(21) Application number: 14166041.5
(22) Date of filing: 25.04.2014
(51) Int. Cl.: C12N 15/11, C12N 15/113, C12N 15/67, C12N 15/85

(54) **MiRNAs enhancing cell productivity**
miRNAs zur Erhöhung der Zellproduktivität
Productivité de cellules améliorant des miARN

(43) Date of publication of application: 28.10.2015
(73) Proprietor: Hochschule Biberach, 88400 Biberach (DE)
(72) Inventor: Otte, Kerstin, 89073 Ulm (DE); Handrick, René, 88400 Biberach (DE); Fischer, Simon, 89195 Staig (DE)
(74) Representative: PATERIS Patentanwälte PartmbB

(56) References cited:
- WO-A1-2010/129919
- WO-A2-2013/182553
- MICHAELA STROTBEK ET AL: "Stable microRNA expression enhances therapeutic antibody productivity of Chinese hamster ovary cells", METABOLIC ENGINEERING, vol. 20, 18 October 2013 (2013-10-18), pages 157-166, XP055143395, ISSN: 1096-7176, DOI: 10.1016/j.ymben.2013.10.005
- JADHAV VAIBHAV ET AL: "Stable overexpression of miR-17 enhances recombinant protein production of CHO cells", JOURNAL OF BIOTECHNOLOGY, vol. 175, 8 February 2014 (2014-02-08), pages 38-44, XP028631072, ISSN: 0168-1656, DOI: 10.1016/J.JBIOTEC.2014.01.032
- A. GERRITS ET AL: "Genetic screen identifies microRNA cluster 99b/let-7e/125a as a regulator of primitive hematopoietic cells", BLOOD, vol. 119, no. 2, 28 November 2011 (2011-11-28), pages 377-387, XP055144534, ISSN: 0006-4971, DOI: 10.1182/blood-2011-01-331686
- DONG JU SON ET AL: "The atypical mechanosensitive microRNA-712 derived from pre-ribosomal RNA induces endothelial inflammation and atherosclerosis", NATURE COMMUNICATIONS, vol. 4, 18 December 2013 (2013-12-18), XP055162844, DOI: 10.1038/ncomms4000
- MULLER D ET AL: "MicroRNAs as targets for engineering of CHO cell factories", TRENDS IN BIOTECHNOLOGY, ELSEVIER PUBLICATIONS, CAMBRIDGE, GB, vol. 26, no. 7, 28 May 2008 (2008-05-28), pages 359-365, XP022757292, ISSN: 0167-7799, DOI: 10.1016/J.TIBTECH.2008.03.010 [retrieved on 2008-05-28]

## Description

### Field of the Invention

The present disclosure relates to a nucleic acid construct comprising at least two different regions each encoding for at least one miRNA or miRNA-inhibitor having distinct functions such as stimulating cellular production of a biomolecule, regulating cell survival and/or regulating proliferation. The disclosure further relates to a cell comprising such a nucleic acid construct. Moreover, the disclosure relates to a method for increasing the yield of a biomolecule produced by a cell cultured *in vitro.*

### Background of the Invention

Since the first pharmaceuticals, such as insulin, were biotechnologically produced, i.e. in living cells, the field of biopharmaceutical manufacturing has grown enormously. Nowadays a large variety of pharmaceutical compounds is produced using biotechnological methods. Such compounds comprise antibodies, cytokines, hormones as well as anticoagulants. Whereas most compounds are produced in lower organisms such as bacteria and yeast, an increasing number of interesting pharmaceuticals, in particular large and complex proteins, need to be expressed in cells derived from higher order organisms such as mammals. This is due to the need of specific enzymes or other molecules involved in the synthesis of the desired compounds, which mostly developed late during evolution. For example, most post-translational modifications of proteins are mediated by enzymes, which are expressed by most mammalian cells but not in bacteria or yeast. Accordingly, biopharmaceuticals are nowadays extensively produced in mammalian cell factories. Due to the rapidly growing demand of biopharmaceuticals, in particular recombinant proteins, various strategies are pursued to achieve higher product titers while maintaining maximal product quality. However, moderate product titers and low stress tolerance in bioreactors are still considerable challenges compared to prokaryotic expression systems. Overcoming limitations of mammalian manufacturing cell lines has been addressed by different cell line engineering approaches to steadily increase production efficiency (e.g. Kramer et al., 2010). Apart from gene knockouts mediated e.g. by zinc finger nucleases, mega nucleases or more recently by using the CRISPR/Cas9 system, the introduction of beneficial genes such as Bcl-XL or AVEN was frequently applied to engineer mammalian cell factories. However, the vast majority of those techniques is complex, labour intensive or requires a substantial amount of time for establishment. Moreover, traditional cell engineering strategies usually rely on the overexpression of one or few secretion enhancing genes. The constitutive overexpression, however, adds additional translational burden to the production cell and, thus, lowers its capacity for producing the biopharmaceutical of interest.

Therefore, there is a need in the art to provide tools and methods to increase production efficiency in biopharmaceutical manufacturing, in particular to increase the productivity while maintaining or even minimizing the cells consumption of energy and nutrients not directed to the production of the compound of interest.

WO 2013/182553 A2 discloses that increasing the level of miRNAs miR-557 and miR-1287 increases the yield of a biomolecule produced by CHO cells.

Strotbek et al., Metabolic Engineering, vol. 20 (2013), pages 157-166 discloses that increasing the level of miRNAs miR-557 and miR-1287 increases the yield of IgG1 produced by CHO cells.

Jadhav et al., Journal of Biotechnology, vol. 175, no. 100 (2014), pages 38-44 discloses that increasing the level of miRNA miR-17 increases the yield of EpoFc protein produced by CHO cells.

Gerrits et al., Blood, vol. 119, no. 2 (2014), pages 377-387 discloses that overexpression of miR cluster 99b/let-7a/125a in mouse hematopoietic cells increases stem cell activity.

Muller et al., Trends in Biotechnology, vol. 26, no. 7 (2008), pages 359-365 discloses the use of miRNAs for engineering CHO cells for producing biopharmaceuticals.

WO 2010/129919 A1 discloses the use of miR-712 for inhibiting a target gene in a cell.

Son et al., Nature Communications, vol. 4 (2013), discloses that miR-712 is a mechanosensitive miRNA upregulated by disturbed flow in endothelial cells and that miR-712 induces atherosclerosis.

### Summary of the Invention

The present invention relates to a nucleic acid construct for increasing the yield of a biomolecule produced by a Chinese hamster ovary cell cultured *in vitro,* the nucleic acid construct comprising at least two different regions, wherein the regions are
- a first region encoding for at least one miRNA stimulating cellular production of a biomolecule in a cell, the miRNA is selected from group 1, or
- a second region encoding for at least one miRNA and/or miRNA inhibitor suppressing cell death, the miRNA is selected from group 2 and the miRNA-inhibitor inhibits a miRNA selected from group 3, and
- a third region encoding for at least one miRNA regulating cell proliferation, wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

The present invention further relates to a cell comprising a construct according to the invention, wherein the cell is a Chinese hamster ovary cell.

The present invention further relates to a method for increasing the yield of a biomolecule produced by a cell cultured *in vitro* comprising at least two steps selected of
- stimulating cellular production of the biomolecule by increasing the level of at least one miRNA selected from group 1 in the cell, or
- reducing cell death by increasing the level of at least one miRNA selected from group 2 in the cell and/or decreasing the level of at least one miRNA selected from group 3, and
- regulating proliferation of the cell by increasing the level of at least one miRNA in the cell, wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the cell is a Chinese hamster ovary cell.

The present invention further relates to a use of a combination of a miRNA of SEQ ID NO.: 160 and at least one miRNA selected from group 1, at least one miRNA selected from group 2 and/or at least one miRNA-inhibitor inhibiting a miRNA selected from group 3 for increasing the yield of a biomolecule produced by a Chinese hamster ovary cell cultured *in vitro,* wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

### Aspects of the Disclosure

In a first aspect, disclosed is a nucleic acid construct comprising at least two different regions, wherein the regions are selected of a first region encoding for at least one miRNA stimulating cellular production of a biomolecule in a cell, the miRNA is selected from group 1, a second region encoding for at least one miRNA and/or miRNA-inhibitor suppressing cell death, the miRNA is selected from group 2 and the miRNA-inhibitor inhibits a miRNA selected from group 3, and a third region encoding for at least one miRNA and/or miRNA-inhibitor regulating cell proliferation, the miRNA is selected from group 4 or 5 and the miRNA-inhibitor inhibits a miRNA selected from group 4 or 5, wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294; group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295; group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471,472, 473, 474, 475,476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609; group 4 consists
of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 160, 162, 164, 173, 177, 187, 189, 193, 198, 202, 215, 218, 232, 234, 235, 244, 246, 249, 250, 261, 263, 264, 267, 270, 275, 281, 283, 287, 288, and 291; and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553,554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606.

In a further aspect, disclosed is a cell comprising the nucleic acid construct as disclosed.

In a further aspect, disclosed is a method for increasing the yield of a biomolecule produced by a cell cultured *in vitro* comprising at least two steps selected of stimulating cellular production of the biomolecule by increasing the level of at least one miRNA selected from group 1 in the cell, reducing cell death by increasing the level of at least one miRNA selected from group 2 in the cell and/or decreasing the level of at least one miRNA selected from group 3, and regulating proliferation of the cell by increasing the level of at least one miRNA selected from group 4 or 5 in the cell and/or decreasing the level of at least one miRNA selected from group 4 or 5, wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294; group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295; group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444,445, 446, 447, 448, 449, 450, 451, 452, 453, 454, 455, 456, 457, 458, 459, 460, 461,462, 463, 464, 465, 466, 467, 468, 469, 470, 471, 472, 473, 474, 475, 476, 477, 478, 479, 480, 481, 482, 483,484, 485, 486, 487, 488, 489, 490, 491, 492, 493,494, 495, 496, 497, 498, 499, 500, 501, 502, 503, 504, 505, 506, 507, 508, 509, 510, 511, 512, 513, 514, 515, 605, 607, 608, and 609; group 4 consists of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 160, 162, 164, 173, 177, 187, 189, 193, 198, 202, 215, 218, 232, 234, 235, 244, 246, 249, 250, 261, 263, 264, 267, 270, 275, 281, 283, 287, 288, and 291; and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606.

In a further aspect, disclosed is a method for producing a biomolecule in a cell comprising the steps propagating the cell in a cell culture, increasing the level of at least one miRNA selected from the group consisting of SEQ ID NO.: 1, 2, 3, 4, 5, 6, 7, 8, 9, 10, 11, 12, 13, 14, 15, 16, 17, 18, 19, and 20, and isolating the biomolecule from the cell culture.

In a further aspect, disclosed is the use of a combination of at least one miRNA selected from group 1, at least one miRNA selected from group 2 and/or at least one miRNA-inhibitor inhibiting a miRNA selected from group 3, and at least one miRNA selected from group 4 or 5 and/or at least one miRNA-inhibitor inhibiting a miRNA selected from group 4 or 5, wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294; group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273,
274, 277, 280, 282, 284, 289, 293, and 295; group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609; group 4 consists of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 160, 162, 164, 173, 177, 187, 189, 193, 198, 202, 215, 218, 232, 234, 235, 244, 246, 249, 250, 261, 263, 264, 267, 270, 275, 281, 283, 287, 288, and 291; and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606.

### Brief Description of the Drawings

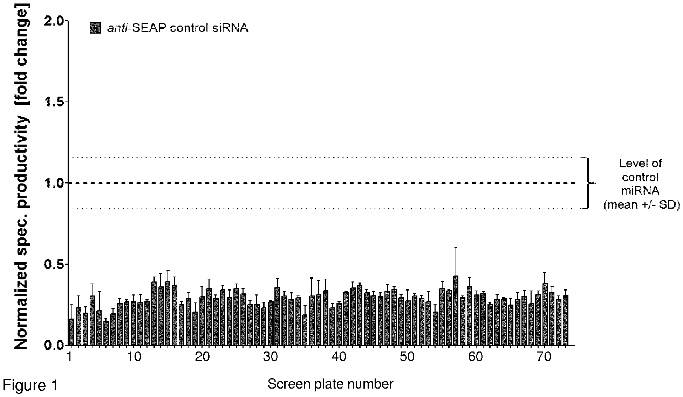

Figure 1 shows the normalized specific SEAP productivity of CHO-SEAP control cells transfected with a functional anti-SEAP control siRNA for all 73 screen plates. Each column represents the mean value of the indicated screen plate. Data was normalized to the mean value of the respective non-targeting control miRNA. Error bars indicate the standard deviation (SD) of three independent transfections.

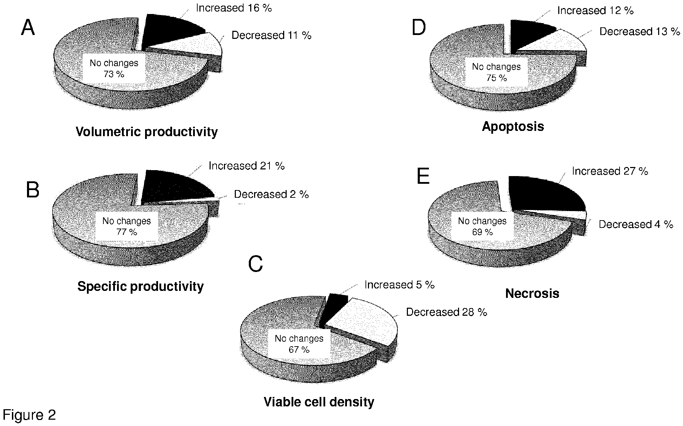

Figure 2 shows an overview on the numbers of miRNAs mimics from the primary screen in CHO-SEAP cells, which induced significant changes (p<0.05), as percentage of the miRNA library. Cake charts are given for each considered bioprocess relevant parameter.

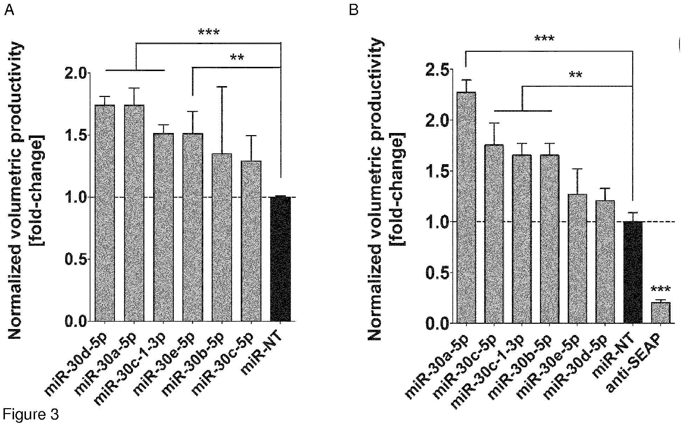

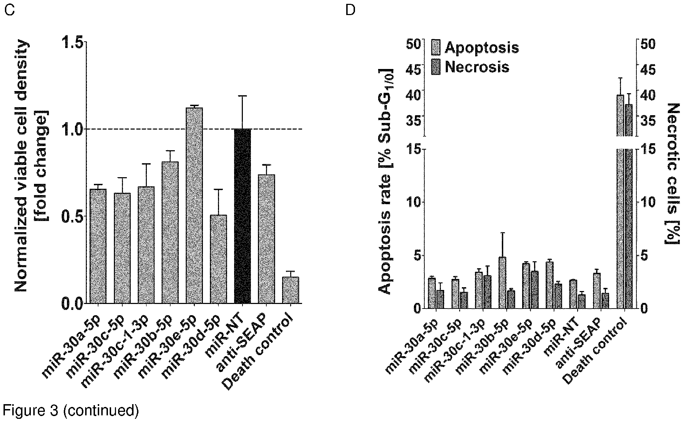

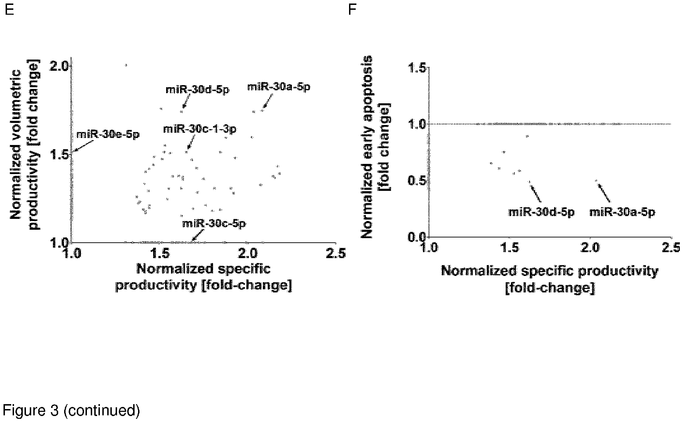

Figure 3 shows that the entire miR-30 family contributes to enhanced culture performance of CHO-SEAP cells. (A) Normalized volumetric SEAP productivity for all miR-30 miRNAs exhibiting increased SEAP productivity in the primary miRNA screen (A) and in the secondary (validation) miRNA screen (B). Normalized viable cell density for all miR-30 miRNAs exhibiting increased SEAP under agitated culture conditions (C). Influence of miR-30 miRNAs on apoptosis and necrosis under agitated culture conditions. Error bars represent the SD of three independent transfections. For statistical analysis an unpaired two-tailed t-test was applied (** p<0.01; *** p<0.001). Normalized increase in volumetric (y-axis) against specific SEAP productivity (x-axis) shown for miRNAs significantly influencing both parameters. (E) Normalized decrease in apoptosis (y-axis) against increase in specific SEAP productivity (x-axis) shown for miRNAs significantly influencing both parameters (F). Respective miR-30 family members are indicated.

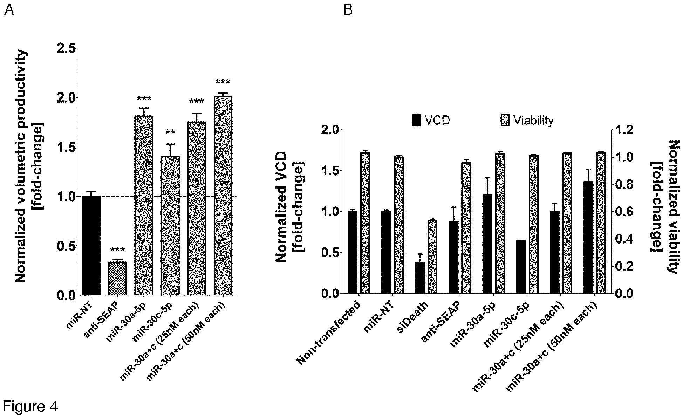

Figure 4 shows the results of a scale-up transfection of miR-30 family members for screen validation in CHO-SEAP cells. Influence on normalized volumetric SEAP productivity (A) and viable cell density (VCD) and viability (B) following introduction of either single miR-30a-5p and miR-30c-5p mimics or combinations of both miRNAs after 72 h following transient introduction. Values were normalized to the miR-NT control and error bars represent the SD of three independent transfections. For statistical analysis an unpaired two-tailed t-test was applied (* p<0.05; ** p<0.01; *** p<0.001).

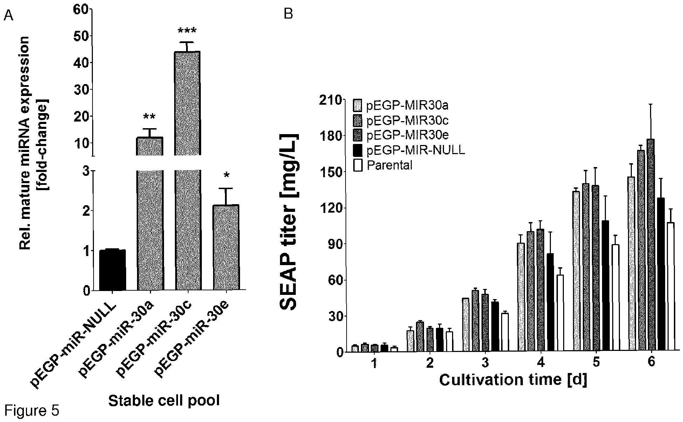

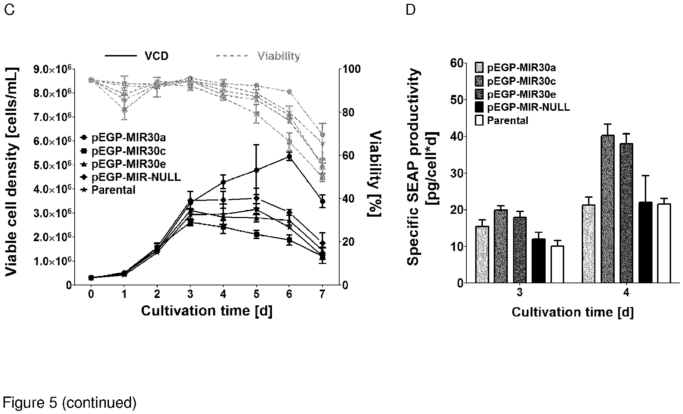

Figure 5 shows a characterization of stable miR-30 overexpressing CHO-SEAP cell pools. miRNA overexpression in stable cell pools (A). Mature miR-30 levels are expressed relative to U6 snoRNA. miRNA overexpression is presented as fold-change value relative to endogenous miRNA level represented by the pEGP-MIR-Null control pool. Determination of volumetric SEAP productivity (B), viable cell density / viability (C), and specific SEAP productivity (D) during batch cultivation of MIR30a, MIR30c and MIR30e overexpressing cell pools compared to negative control and parental CHO-SEAP cells. Error bars represent the SD of three replicates. Statistical analysis: unpaired two-tailed t-test comparing each miR-30 overexpressing pool with the parental CHO-SEAP cell line (* p<0.05; ** p<0.01; *** p<0.001).

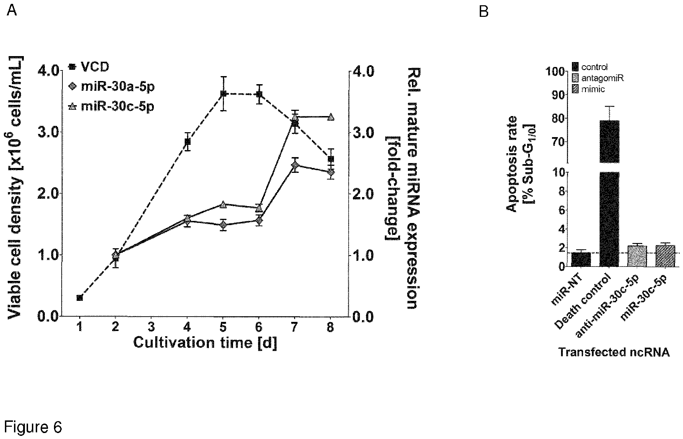

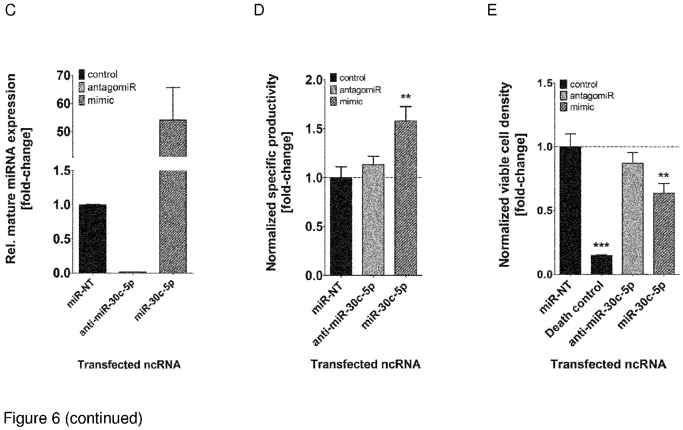

Figure 6 shows (A) an analysis of endogenous miR-30a-5p (diamond) and miR-30c-5p (triangle) expression level during batch cultivation of CHO-SEAP cells. Analysis of miRNA expression level and viable cell density (dotted line) was performed at indicated days post seeding and changes in miRNA expression were calculated relative to the level at 48 h. (B) Analysis of apoptosis in CHO-SEAP cells after miR-30c-5p mimics/antagomiR (anti-miR-30c-5p) transfection by means of Nicoletti staining. DNA content of transfected cells was determined using flow cytometry and cells exhibiting DNA content less than 2n (Sub-G1/0) were quantified as percentage of the whole cell population. Error bars represent the SD of three independent transfections. (C) Relative mature miR-30c-5p abundance in CHO-SEAP cells after miR-30c-5p mimics/antagomiR (anti-miR-30c-5p) transfection. miRNA expression is presented as fold-change value relative to endogenous miRNA level represented by miR-NT transfected control cells (black column). Total RNA was isolated 72 h post transfection and RNA samples of triplicate transfections were pooled for reverse transcription. Mature miR-30c-5p levels are expressed relative to U6 snoRNA and error bars represent the SD of three technical replicates. Normalized (D) specific SEAP productivity and (E) viable cell density of CHO-SEAP cells 72 h following miR-30c-5p mimics/antagomiR (anti-miR-30c-5p) introduction. Data was normalized to values of the miR-NT transfected control cells (black column) and error bars represent the SD of three independent transfections. For statistical analysis an unpaired two-tailed t-test was applied (** p<0.01; *** p<0.001).

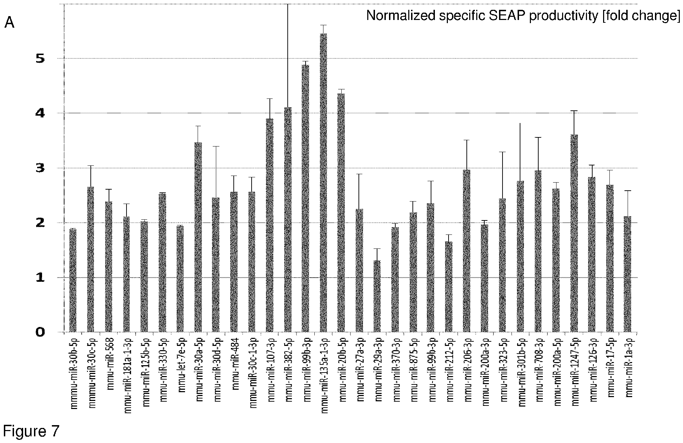

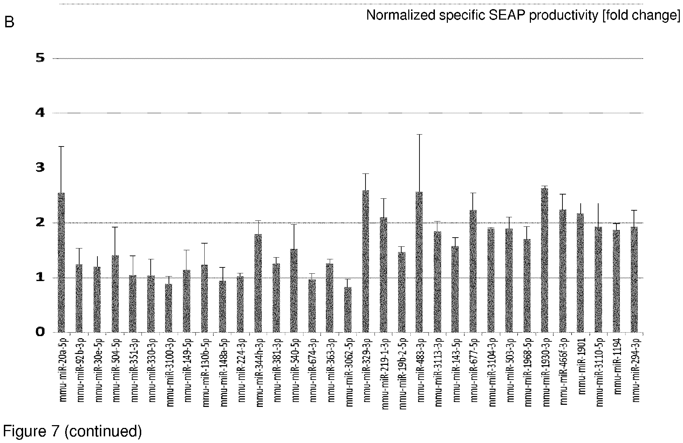

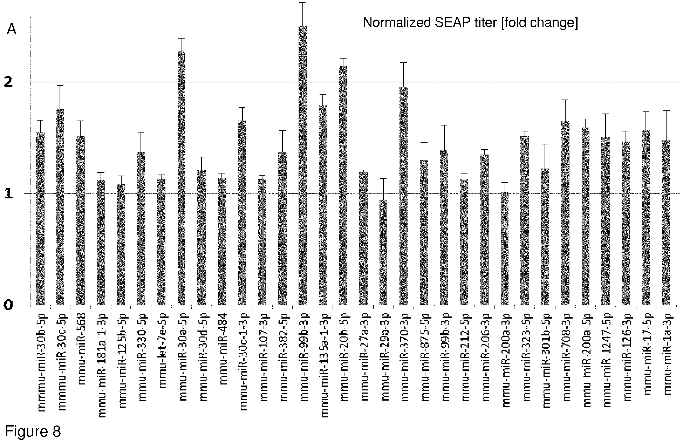

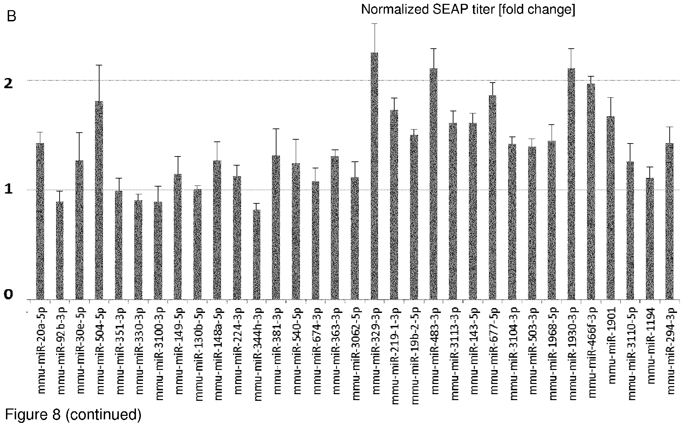

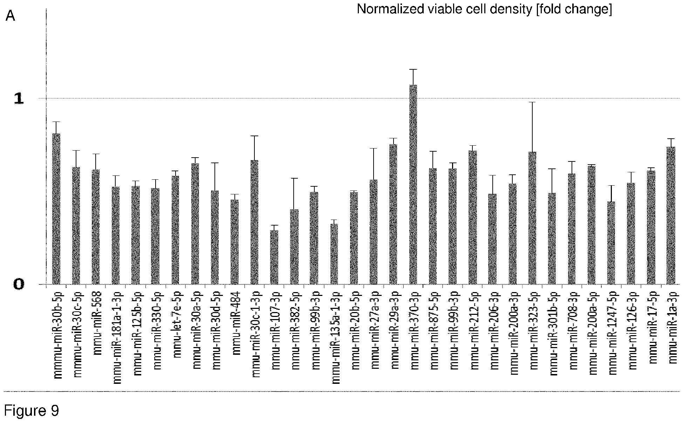

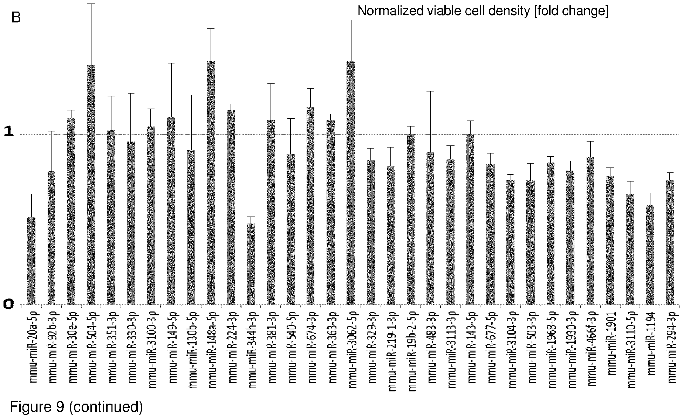

Figures 7 to 9 show the results of the secondary (validation) miRNA screen for regarding specific SEAP productivity (7), volumetric SEAP productivity (8) and proliferation (9). Data was normalized to values of the miR-NT transfected control cells and error bars represent the SD of three independent transfections. For statistical analysis an unpaired two-tailed t-test was applied (** p<0.01; *** p<0.001).

### Detailed Description

The present invention relates to a nucleic acid construct for increasing the yield of a biomolecule produced by a Chinese hamster ovary cell cultured *in vitro,* the nucleic acid construct comprising at least two different regions, wherein the regions are
- a first region encoding for at least one miRNA stimulating cellular production of a biomolecule in a cell, the miRNA is selected from group 1, or
- a second region encoding for at least one miRNA and/or miRNA inhibitor suppressing cell death, the miRNA is selected from group 2 and the miRNA-inhibitor inhibits a miRNA selected from group 3, and
- a third region encoding for at least one miRNA regulating cell proliferation,
wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

The present invention further relates to a cell comprising a construct according to the invention, wherein the cell is a Chinese hamster ovary cell.

The present invention further relates to a method for increasing the yield of a biomolecule produced by a cell cultured *in vitro* comprising at least two steps selected of
- stimulating cellular production of the biomolecule by increasing the level of at least one miRNA selected from group 1 in the cell, or
- reducing cell death by increasing the level of at least one miRNA selected from group 2 in the cell and/or decreasing the level of at least one miRNA selected from group 3, and
- regulating proliferation of the cell by increasing the level of at least one miRNA in the cell, wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451,452, 453, 454, 455, 456, 457, 458, 459, 460, 461, 462, 463, 464, 465, 466, 467, 468, 469, 470, 471, 472, 473, 474, 475, 476, 477, 478, 479, 480, 497, 498, 499, 500, 501, 502, 503, 504, 505, 506, 507, 508, 509, 510, 511, 512, 513, 514, 515, 605, 607, 608, and 609,
and wherein the cell is a Chinese hamster ovary cell.

The present invention further relates to a use of a combination of a miRNA of SEQ ID NO.: 160 and at least one miRNA selected from group 1, at least one miRNA selected from group 2 and/or at least one miRNA-inhibitor inhibiting a miRNA selected from group 3 for increasing the yield of a biomolecule produced by a Chinese hamster ovary cell cultured *in vitro,* wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

In a first aspect, disclosed is a nucleic acid construct comprising at least two different regions, wherein the regions are selected of a first region encoding for at least one miRNA stimulating cellular production of a biomolecule in a cell, the miRNA is selected from group 1, a second region encoding for at least one miRNA and/or miRNA-inhibitor suppressing cell death, the miRNA is selected from group 2 and the miRNA-inhibitor inhibits a miRNA selected from group 3, and a third region encoding for at least one miRNA and/or miRNA-inhibitor regulating cell proliferation, the miRNA is selected from group 4 or 5 and the miRNA-inhibitor inhibits a miRNA selected from group 4 or 5.

Micro RNAs (miRNAs) are endogenous small non-coding RNA molecules of about 22 nucleotides that post-transcriptionally regulate global gene expression in eukaryotic cells and are highly conserved across species. A single miRNA usually regulates up to hundreds of different messenger RNAs (mRNAs) and most mRNAs are expected to be targeted by multiple miRNAs. miRNA genes are transcribed by RNA polymerase-II and subsequently processed, giving rise to single-stranded mature miRNAs, which are incorporated into the RNA-induced silencing complex (RISC). As a central part of the RISC complex, the miRNA guides RISC to its mRNA targets, where the miRNA binds the 3'-untranslated region (3'UTR) of the mRNA transcript by partial complementary base pairing. Gene silencing occurs either through argonaute-2 (AGO2)-mediated mRNA cleavage or translational repression facilitated by AGO1 to 4, with both ways finally reducing the levels of corresponding proteins (van Rooij, 2011). In contrast to small interference RNAs (siRNAs), miRNAs are only partially complementary to binding sites within the 3'UTR of the target transcript, leading to less specificity and, thus, increasing the pool of potential target genes. Complete Watson-Crick base pairing is only compository at the miRNA "seed" region which covers the sequence between nucleotide 2 to 7/8 at the 5-end of the mature miRNA. miRNAs with identical "seed" sequences such as the miR-30 members are grouped into families. According to bioinformatic target prediction tools, members of the same family are supposed to share a large number of target mRNAs.

The inventors found that despite the large number of targets of a single miRNA, several miRNAs show a specific effect on certain cellular processes. By performing a functional high-content miRNA screen, using an entire murine miRNA mimics library comprising 1139 miRNAs, in a recombinant CHO-SEAP suspension cell line, the inventors revealed distinct miRNAs, which are suitable to improve specific cell functions. In particular, the miRNAs of group 1 (table 1) were found to stimulate cellular production of the biomolecule. The term "cellular production of a biomolecule" as used herein refers to the amount of a biomolecule produced per cell. Cellular production is also referred to as "specific productivity", in contrast to the "volumetric productivity" of an entire culture, which refers to the yield of biomolecule that can be harvested from the culture. The level of cellular production mainly depends on the amount of biomolecule synthesized per time by one cell e.g. on protein translation speed, and where applicable on the efficiency, with which the biomolecule is secreted from the cell. Thus, miRNAs of group 1 are expected to influence one or even both processes. Within group 1, the miRNAs of group 9, consisting of SEQ ID NO.: 1, 3, 6, 8, 10, 29, 39, 40, 47, 49, 51, 55, 91, 103, 115, 132, 137, 171, 211 and 294 were found to show the most prominent effect on cellular production. Accordingly, the first region preferably encodes for at least one miRNA selected from group 9.

**Table 1: miRNAs stimulating cellular production (group 1)**

| SEQ ID NO.: | miRNA |
|---|---|
| 1 | mmu-miR-99b-3p |
| 2 | mmu-miR-767 |
| 3 | mmu-miR-30a-5p |
| 4 | mmu-miR-3062-5p |
| 6 | mmu-miR-200a-5p |
| 8 | mmu-miR-135a-1-3p |
| 9 | mmu-miR-743a-5p |
| 10 | mmu-miR-694 |
| 11 | mmu-miR-674-3p |
| 12 | mmu-miR-669d-3p |
| 13 | mmu-miR-301b-5p |
| 14 | mmu-miR-212-5p |
| 15 | mmu-miR-203-5p |
| 16 | mmu-miR-200b-5p |
| 17 | mmu-miR-200a-3p |
| 18 | mmu-miR-1968-5p |
| 19 | mmu-miR-150-3p |
| 20 | mmu-miR-30d-5p |
| 21 | mmu-miR-92b-5p |
| 25 | mmu-miR-871-3p |
| 26 | mmu-miR-760-5p |
| 27 | mmu-miR-741-3p |
| 28 | mmu-miR-713 |
| 29 | mmu-miR-700-5p |
| 32 | mmu-miR-669d-2-3p |
| 34 | mmu-miR-666-5p |
| 37 | mmu-miR-5623-5p |
| 39 | mmu-miR-5134 |
| 40 | mmu-miR-5132 |
| 41 | mmu-miR-5127 |
| 42 | mmu-miR-5124 |
| 44 | mmu-miR-5117-3p |
| 45 | mmu-miR-5111-3p |
| 46 | mmu-miR-5099 |
| 47 | mmu-miR-504-5p |
| 48 | mmu-miR-497-3p |
| 49 | mmu-miR-484 |
| 51 | mmu-miR-466f-3p |
| 52 | mmu-miR-463-5p |
| 53 | mmu-miR-3971 |
| 55 | mmu-miR-370-3p |
| 56 | mmu-miR-344g-5p |
| 57 | mmu-miR-344d-3p |
| 58 | mmu-miR-341-5p |
| 62 | mmu-miR-3113-3p |
| 63 | mmu-miR-3107-3p |
| 66 | mmu-miR-3094-5p |
| 67 | mmu-miR-3083-5p |
| 69 | mmu-miR-3074-5p |
| 70 | mmu-miR-3065-3p |
| 75 | mmu-miR-218-1-3p |
| 76 | mmu-miR-215-3p |
| 77 | mmu-miR-20b-5p |
| 80 | mmu-miR-1b-3p |
| 81 | mmu-miR-1956 |
| 82 | mmu-miR-1953 |
| 83 | mmu-miR-193b-3p |
| 86 | mmu-miR-1898 |
| 88 | mmu-miR-155-5p |
| 90 | mmu-miR-149-5p |
| 91 | mmu-miR-143-5p |
| 93 | mmu-miR-136-5p |
| 98 | mmu-let-7a-1-3p |
| 99 | mmu-miR-875-5p |
| 100 | mmu-miR-802-5p |
| 101 | mmu-miR-708-3p |
| 102 | mmu-miR-681 |
| 103 | mmu-miR-677-5p |
| 104 | mmu-miR-675-3p |
| 106 | mmu-miR-669e-5p |
| 109 | mmu-miR-5115 |
| 110 | mmu-miR-5105 |
| 111 | mmu-miR-5104 |
| 112 | mmu-miR-503-3p |
| 113 | mmu-miR-489-5p |
| 114 | mmu-miR-485-3p |
| 115 | mmu-miR-483-3p |
| 116 | mmu-miR-467c-3p |
| 117 | mmu-miR-3970 |
| 118 | mmu-miR-3969 |
| 120 | mmu-miR-376c-5p |
| 121 | mmu-miR-375-5p |
| 128 | mmu-miR-30b-5p |
| 130 | mmu-miR-3057-3p |
| 132 | mmu-miR-20a-5p |
| 134 | mmu-miR-1942 |
| 136 | mmu-miR-1903 |
| 137 | mmu-miR-1901 |
| 138 | mmu-miR-1843b-3p |
| 141 | mmu-miR-1264-3p |
| 142 | mmu-miR-1194 |
| 143 | mmu-miR-1188-3p |
| 147 | mmu-miR-30c-1-3p |
| 151 | mmu-miR-92b-3p |
| 152 | mmu-miR-879-3p |
| 155 | mmu-miR-764-5p |
| 158 | mmu-miR-720 |
| 163 | mmu-miR-702 |
| 165 | mmu-miR-669d-5p |
| 171 | mmu-miR-568 |
| 174 | mmu-miR-5621-5p |
| 182 | mmu-miR-5114 |
| 183 | mmu-miR-5106 |
| 185 | mmu-miR-5097 |
| 186 | mmu-miR-5046 |
| 188 | mmu-miR-488-5p |
| 190 | mmu-miR-467d-5p |
| 201 | mmu-miR-351-3p |
| 203 | mmu-miR-344d-1-5p |
| 207 | mmu-miR-330-5p |
| 211 | mmu-miR-3104-3p |
| 212 | mmu-miR-3102-3p.2-3p |
| 214 | mmu-miR-3100-3p |
| 217 | mmu-miR-3093-3p |
| 222 | mmu-miR-3075-3p |
| 223 | mmu-miR-3073b-3p |
| 228 | mmu-miR-3065-5p |
| 230 | mmu-miR-3061-5p |
| 233 | mmu-miR-302a-5p |
| 238 | mmu-miR-29a-3p |
| 239 | mmu-miR-299-3p |
| 240 | mmu-miR-294-5p |
| 254 | mmu-miR-1966 |
| 255 | mmu-miR-1963 |
| 269 | mmu-miR-132-5p |
| 276 | mmu-miR-1231-5p |
| 278 | mmu-miR-1196-5p |
| 279 | mmu-miR-1193-5p |
| 285 | mmu-let-7e-5p |
| 294 | mmu-miR-30c-5p |

The miRNAs of groups 2 (table 2) and 3 (table 3) were found to influence cell survival either by suppressing cell death (group 2) or by promoting apoptosis or necrosis (group 3). Cell death within the culture does not only reduce the number of producing cells but also provides a significant burden to the entire culture. Dead cells remain within the culture as debris, which increases cellular stress and can even become toxic at higher concentrations. Accordingly, cell debris needs to be removed from the cultures, which disturbs the culture conditions and provides physical stress to the cells, all of which finally results in a reduced productivity. Therefore, for suppressing cell death, the nucleic acid construct encodes for a miRNA of group 2, which are suitable to directly inhibit apoptosis. Of these miRNAs, the miRNAs of group 10 consisting of SEQ ID NO.: 3, 7, 20, 54, 59, 73, 94, 145, 159, 175, 176, 178, 179, 199, 206, 248, 251, 252, 266 and 272 were found to show the most prominent effect on cell survival. Accordingly, the second region preferably encodes for at least one miRNA selected from group 10. The miRNAs of group 3 (table 3) were found to promote cell death, in particular apoptosis (group 6; table 6) or necrosis (group 7; table 7). Thus, inhibition of these miRNAs is suitable for suppressing cell death. Within group 3, the miRNAs of group 11 consisting of SEQ ID NO.: 297, 305, 307, 311, 312, 313, 321, 330, 331, 335, 336, 340, 345, 351, 359, 405, 412, 458, 510 and 608 were found to have the most prominent cell death inducing effect, such that an inhibition of these miRNAs is most preferred. Accordingly, the second region preferably encodes for at least one miRNA-inhibitor inhibiting a miRNA selected from group 11.

**Table 2: miRNAs suppressing apoptosis**

| SEQ ID NO.: | miRNA |
|---|---|
| 1 | mmu-miR-99b-3p |
| 2 | mmu-miR-767 |
| 3 | mmu-miR-30a-5p |
| 4 | mmu-miR-3062-5p |
| 5 | mmu-miR-291b-3p |
| 6 | mmu-miR-200a-5p |
| 7 | mmu-miR-1a-3p |
| 8 | mmu-miR-135a-1-3p |
| 9 | mmu-miR-743a-5p |
| 20 | mmu-miR-30d-5p |
| 24 | mmu-miR-878-3p |
| 31 | mmu-miR-669f-3p |
| 33 | mmu-miR-669a-3-3p |
| 50 | mmu-miR-466f-5p |
| 52 | mmu-miR-463-5p |
| 54 | mmu-miR-382-5p |
| 59 | mmu-miR-329-3p |
| 60 | mmu-miR-323-5p |
| 61 | mmu-miR-322-3p |
| 64 | mmu-miR-30e-5p |
| 68 | mmu-miR-3076-3p |
| 71 | mmu-miR-3058-5p |
| 73 | mmu-miR-2861 |
| 74 | mmu-miR-219-1-3p |
| 79 | mmu-miR-205-5p |
| 85 | mmu-miR-1899 |
| 87 | mmu-miR-1896 |
| 89 | mmu-miR-155-3p |
| 92 | mmu-miR-141-5p |
| 94 | mmu-miR-136-3p |
| 95 | mmu-miR-1247-5p |
| 97 | mmu-miR-106a-3p |
| 98 | mmu-let-7a-1-3p |
| 145 | mmu-miR-126-3p |
| 153 | mmu-miR-872-5p |
| 154 | mmu-miR-871-5p |
| 156 | mmu-miR-760-3p |
| 159 | mmu-miR-717 |
| 161 | mmu-miR-711 |
| 166 | mmu-miR-669c-5p |
| 167 | mmu-miR-592-5p |
| 168 | mmu-miR-590-5p |
| 169 | mmu-miR-590-3p |
| 170 | mmu-miR-574-5p |
| 172 | mmu-miR-5626-5p |
| 175 | mmu-miR-551b-5p |
| 176 | mmu-miR-551b-3p |
| 178 | mmu-miR-543-3p |
| 179 | mmu-miR-542-5p |
| 180 | mmu-miR-5136 |
| 181 | mmu-miR-5117-5p |
| 184 | mmu-miR-5100 |
| 191 | mmu-miR-453 |
| 192 | mmu-miR-452-5p |
| 194 | mmu-miR-429-3p |
| 195 | mmu-miR-423-5p |
| 196 | mmu-miR-421-3p |
| 197 | mmu-miR-379-3p |
| 199 | mmu-miR-367-3p |
| 200 | mmu-miR-365-2-5p |
| 204 | mmu-miR-342-5p |
| 205 | mmu-miR-340-3p |
| 206 | mmu-miR-33-3p |
| 208 | mmu-miR-31-5p |
| 209 | mmu-miR-3110-3p |
| 210 | mmu-miR-3109-3p |
| 213 | mmu-miR-3100-5p |
| 216 | mmu-miR-3094-3p |
| 219 | mmu-miR-3088-3p |
| 220 | mmu-miR-3081-5p |
| 221 | mmu-miR-3076-5p |
| 224 | mmu-miR-3072-5p |
| 225 | mmu-miR-3071-3p |
| 226 | mmu-miR-3067-5p |
| 227 | mmu-miR-3066-3p |
| 229 | mmu-miR-3062-3p |
| 231 | mmu-miR-3059-5p |
| 236 | mmu-miR-300-5p |
| 237 | mmu-miR-29b-1-5p |
| 241 | mmu-miR-293-5p |
| 242 | mmu-miR-293-3p |
| 243 | mmu-miR-23a-5p |
| 245 | mmu-miR-223-3p |
| 247 | mmu-miR-217-3p |
| 248 | mmu-miR-211-3p |
| 251 | mmu-miR-200c-5p |
| 252 | mmu-miR-19a-3p |
| 253 | mmu-miR-196a-2-3p |
| 256 | mmu-miR-1952 |
| 257 | mmu-miR-194-1-3p |
| 258 | mmu-miR-193b-5p |
| 259 | mmu-miR-1935 |
| 260 | mmu-miR-1934-5p |
| 262 | mmu-miR-1902 |
| 265 | mmu-miR-188-3p |
| 266 | mmu-miR-182-3p |
| 268 | mmu-miR-134-3p |
| 271 | mmu-miR-128-2-5p |
| 272 | mmu-miR-128-1-5p |
| 273 | mmu-miR-127-5p |
| 274 | mmu-miR-127-3p |
| 277 | mmu-miR-1197-5p |
| 280 | mmu-miR-1191 |
| 282 | mmu-miR-101a-5p |
| 284 | mmu-let-7g-3p |
| 289 | mmu-miR-10b-5p |
| 293 | mmu-miR-221-3p |
| 295 | mmu-miR-346-3p |

**Table 3: miRNAs promoting cell death**

| SEQ ID NO.: | miRNA |
|---|---|
| 296 | mmu-miR-9-5p |
| 297 | mmu-miR-133a-3p |
| 298 | mmu-miR-134-5p |
| 299 | mmu-miR-135a-5p |
| 300 | mmu-miR-137-3p |
| 301 | mmu-miR-154-5p |
| 302 | mmu-miR-183-5p |
| 303 | mmu-miR-185-5p |
| 304 | mmu-let-7d-3p |
| 305 | mmu-miR-29c-3p |
| 306 | mmu-miR-337-3p |
| 307 | mmu-miR-28-5p |
| 308 | mmu-miR-218-5p |
| 309 | mmu-miR-33-5p |
| 310 | mmu-miR-378-5p |
| 311 | mmu-miR-410-3p |
| 312 | mmu-miR-540-3p |
| 313 | mmu-miR-690 |
| 314 | mmu-miR-133a-5p |
| 315 | mmu-miR-673-5p |
| 316 | mmu-miR-744-5p |
| 317 | mmu-miR-183-3p |
| 318 | mmu-miR-29a-5p |
| 319 | mmu-miR-338-5p |
| 320 | mmu-miR-466a-5p |
| 321 | mmu-miR-882 |
| 322 | mmu-miR-466e-5p |
| 323 | mmu-miR-466g |
| 324 | mmu-miR-466j |
| 325 | mmu-miR-467g |
| 326 | mmu-miR-1906 |
| 327 | mmu-miR-1904 |
| 328 | mmu-miR-1943-5p |
| 329 | mmu-miR-1962 |
| 330 | mmu-miR-1839-5p |
| 331 | mmu-miR-3064-5p |
| 332 | mmu-miR-3068-3p |
| 333 | mmu-miR-3073-3p |
| 334 | mmu-miR-3091-5p |
| 335 | mmu-miR-3098-5p |
| 336 | mmu-miR-344c-5p |
| 337 | mmu-miR-3102-3p |
| 338 | mmu-miR-3104-5p |
| 339 | mmu-miR-3112-3p |
| 340 | mmu-miR-192-3p |
| 341 | mmu-miR-103-1-5p |
| 342 | mmu-miR-135a-2-3p |
| 343 | mmu-miR-452-3p |
| 344 | mmu-miR-669f-5p |
| 345 | mmu-miR-1948-5p |
| 346 | mmu-miR-1964-5p |
| 347 | mmu-miR-3096b-3p |
| 348 | mmu-miR-3968 |
| 349 | mmu-miR-5101 |
| 350 | mmu-miR-5709 |
| 351 | mmu-miR-3070a-5p // mmu-miR-3070b-5p |
| 352 | mmu-miR-669m-5p // mmu-miR-466m-5p |
| 353 | mmu-miR-706 |
| 354 | mmu-let-7i-5p |
| 355 | mmu-miR-101a-3p |
| 356 | mmu-miR-125a-5p |
| 357 | mmu-miR-152-3p |
| 358 | mmu-miR-201-5p |
| 359 | mmu-miR-202-3p |
| 360 | mmu-miR-290-5p |
| 361 | mmu-miR-34c-5p |
| 362 | mmu-let-7b-5p |
| 363 | mmu-miR-351-5p |
| 364 | mmu-miR-135b-5p |
| 365 | mmu-miR-181c-5p |
| 366 | mmu-miR-217-5p |
| 367 | mmu-miR-380-3p |
| 368 | mmu-miR-215-5p |
| 369 | mmu-miR-448-3p |
| 370 | mmu-miR-449a-5p |
| 371 | mmu-miR-547-3p |
| 372 | mmu-miR-494-3p |
| 373 | mmu-miR-302c-5p |
| 374 | mmu-miR-302c-3p |
| 375 | mmu-miR-679-5p |
| 376 | mmu-miR-683 |
| 377 | mmu-miR-686 |
| 378 | mmu-miR-146b-5p |
| 379 | mmu-miR-467b-3p |
| 380 | mmu-miR-455-5p |
| 381 | mmu-miR-698 |
| 382 | mmu-miR-706 |
| 383 | mmu-miR-707 |
| 384 | mmu-miR-714 |
| 385 | mmu-miR-501-3p |
| 386 | mmu-miR-450b-3p |
| 387 | mmu-miR-505-3p |
| 388 | mmu-miR-718 |
| 389 | mmu-miR-675-5p |
| 390 | mmu-miR-374-3p |
| 391 | mmu-miR-665-3p |
| 392 | mmu-miR-758-3p |
| 393 | mmu-miR-763 |
| 394 | mmu-miR-202-5p |
| 395 | mmu-miR-15a-3p |
| 396 | mmu-miR-20a-3p |
| 397 | mmu-miR-31-3p |
| 398 | mmu-miR-93-3p |
| 399 | mmu-miR-337-5p |
| 400 | mmu-miR-339-3p |
| 401 | mmu-miR-345-3p |
| 402 | mmu-miR-20b-3p |
| 403 | mmu-miR-666-3p |
| 404 | mmu-miR-743b-5p |
| 405 | mmu-miR-883a-3p |
| 406 | mmu-miR-876-3p |
| 407 | mmu-miR-327 |
| 408 | mmu-miR-466b-3p // mmu-miR-466c-3p // mmu-miR-466p-3p |
| 409 | mmu-miR-467c-5p |
| 410 | mmu-miR-493-3p |
| 411 | mmu-miR-509-5p |
| 412 | mmu-miR-654-5p |
| 413 | mmu-miR-449b |
| 414 | mmu-miR-669k-3p |
| 415 | mmu-miR-1186 |
| 416 | mmu-miR-1187 |
| 417 | mmu-miR-669h-5p |
| 418 | mmu-miR-1195 |
| 419 | mmu-miR-1198-5p |
| 420 | mmu-miR-1897-5p |
| 421 | mmu-miR-1905 |
| 422 | mmu-miR-1907 |
| 423 | mmu-miR-1894-3p |
| 424 | mmu-miR-1933-5p |
| 425 | mmu-miR-1947-5p |
| 426 | mmu-miR-1948-3p |
| 427 | mmu-miR-1960 |
| 428 | mmu-miR-1946b |
| 429 | mmu-miR-1970 |
| 430 | mmu-miR-1971 |
| 431 | mmu-miR-1982-5p |
| 432 | mmu-miR-2139 |
| 433 | mmu-miR-1249-5p |
| 434 | mmu-miR-3099-3p |
| 435 | mmu-miR-3106-5p |
| 436 | mmu-miR-3106-3p |
| 437 | mmu-miR-3057-5p |
| 438 | mmu-miR-3061-3p |
| 439 | mmu-miR-3063-3p |
| 440 | mmu-miR-3069-5p |
| 441 | mmu-miR-3073-5p |
| 442 | mmu-miR-3079-5p |
| 443 | mmu-miR-3082-3p |
| 444 | mmu-miR-3084-5p |
| 445 | mmu-miR-466m-3p |
| 446 | mmu-miR-466n-5p |
| 447 | mmu-miR-466n-3p |
| 448 | mmu-miR-3092-5p |
| 449 | mmu-miR-3092-3p |
| 450 | mmu-miR-3096-5p |
| 451 | mmu-miR-3097-5p |
| 452 | mmu-miR-3097-3p |
| 453 | mmu-miR-3102-5p |
| 454 | mmu-miR-3102-5p.2-5p |
| 455 | mmu-miR-3108-5p |
| 456 | mmu-miR-3109-5p |
| 457 | mmu-miR-374c-5p |
| 458 | mmu-miR-1912-3p |
| 459 | mmu-miR-3471 |
| 460 | mmu-miR-1186b |
| 461 | mmu-miR-3474 |
| 462 | mmu-miR-137-5p |
| 463 | mmu-miR-146a-3p |
| 464 | mmu-miR-153-5p |
| 465 | mmu-miR-196a-1-3p |
| 466 | mmu-miR-1a-2-5p |
| 467 | mmu-miR-25-5p |
| 468 | mmu-miR-29b-2-5p |
| 469 | mmu-miR-92a-1-5p |
| 470 | mmu-miR-181b-1-3p |
| 471 | mmu-miR-133b-5p |
| 472 | mmu-miR-448-5p |
| 473 | mmu-miR-471-3p |
| 474 | mmu-miR-541-3p |
| 475 | mmu-miR-367-5p |
| 476 | mmu-miR-487b-5p |
| 477 | mmu-miR-669c-3p |
| 478 | mmu-miR-499-3p |
| 479 | mmu-miR-701-3p |
| 480 | mmu-miR-181d-3p |
| 481 | mmu-miR-466h-3p |
| 482 | mmu-miR-493-5p |
| 483 | mmu-miR-653-3p |
| 484 | mmu-miR-669e-3p |
| 485 | mmu-miR-1199-3p |
| 486 | mmu-miR-1947-3p |
| 487 | mmu-miR-1955-3p |
| 488 | mmu-miR-664-5p |
| 489 | mmu-miR-3964 |
| 490 | mmu-miR-3473b |
| 491 | mmu-miR-3473c |
| 492 | mmu-miR-5109 |
| 493 | mmu-miR-5118 |
| 494 | mmu-miR-5120 |
| 495 | mmu-miR-5121 |
| 496 | mmu-miR-3544-3p |
| 497 | mmu-miR-5615-3p |
| 498 | mmu-miR-1231-3p |
| 499 | mmu-miR-5616-3p |
| 500 | mmu-miR-5617-3p |
| 501 | mmu-miR-3073b-5p |
| 502 | mmu-miR-5710 |
| 503 | mmu-miR-1929-3p |
| 504 | mmu-miR-669a-5p // mmu-miR-669p-5p |
| 505 | mmu-miR-466b-5p // mmu-miR-466o-5p |
| 506 | mmu-miR-344e-5p // mmu-miR-344h-5p |
| 507 | mmu-miR-96-5p |
| 508 | mmu-miR-200c-3p |
| 509 | mmu-miR-216a-5p |
| 510 | mmu-miR-761 |
| 511 | mmu-miR-18a-3p |
| 512 | mmu-miR-466k |
| 513 | mmu-miR-467h |
| 514 | mmu-miR-1955-5p |
| 515 | mmu-miR-3096-3p |
| 605 | mmu-let-7f-5p |
| 607 | mmu-miR-24-3p |
| 608 | mmu-miR-298-3p |
| 609 | mmu-miR-7b-5p |

The miRNAs of group 4 (table 4) were found to promote proliferation, whereas those miRNAs of group 5 (table 5) reduced cell division. Thus, expression of miRNAs of group 4 and inhibition of miRNAs of group 5 is suitable for promoting proliferation, whereas expression of miRNAs of group 5 and inhibition of miRNAs of group 4 is suitable for inhibiting proliferation. Besides the cellular production of each single cell, the number of cells present in a culture determines the yield of biomolecule that can be harvested. Therefore, stimulating cell proliferation can be desired for increasing the size of the producing culture, in particular if slowly growing cells are used or when starting a cell culture. On the other hand, once a culture has reached an optimal cell density, inhibiting cell proliferation may be desired. For dividing, a cell needs to roughly duplicate almost all components including membrane, cell nucleus and further organelles. This consumes energy and protein translation capacity, which is then not provided for production of the biomolecule of interest. Therefore, inhibiting cell proliferation can be desired, in particular once an optimal culture size is reached. Of the miRNAs of group 4, miRNAs of group 12 consisting of SEQ ID NO.: 5, 7, 22, 30, 35, 43, 68, 72, 78, 84, 96, 146, 148, 160, 173, 177, 198, 202, 232, 234, 244, 267 and 283, had the most prominent effect on cell proliferation and of those miRNAs found to repress proliferation, the miRNAs of group 13 consisting of SEQ ID NO.: 517, 523, 526, 529, 531, 533, 537, 548, 550, 558, 560, 561, 563, 566, 567, 571, 575, 577, 583, 591, 600, 601 and 604 were most effective. Accordingly, for promoting cell proliferation, the third region preferably encodes for a miRNA selected from group 12 and/or for a miRNA-inhibitor inhibiting a miRNA selected from group 13. For inhibiting cell proliferation, the third region preferably encodes for a miRNA of group 13 and/or for a miRNA-inhibitor inhibiting a miRNA of group 12.

**Table 4: miRNAs promoting proliferation**

| SEQ ID NO.: | miRNA |
|---|---|
| 5 | mmu-miR-291b-3p |
| 7 | mmu-miR-1a-3p |
| 68 | mmu-miR-3076-3p |
| 79 | mmu-miR-205-5p |
| 94 | mmu-miR-136-3p |
| 10 | mmu-miR-694 |
| 11 | mmu-miR-674-3p |
| 12 | mmu-miR-669d-3p |
| 13 | mmu-miR-301b-5p |
| 14 | mmu-miR-212-5p |
| 15 | mmu-miR-203-5p |
| 16 | mmu-miR-200b-5p |
| 17 | mmu-miR-200a-3p |
| 18 | mmu-miR-1968-5p |
| 19 | mmu-miR-150-3p |
| 22 | mmu-miR-880-5p |
| 23 | mmu-miR-878-5p |
| 30 | mmu-miR-684 |
| 35 | mmu-miR-582-5p |
| 36 | mmu-miR-582-3p |
| 38 | mmu-miR-540-5p |
| 43 | mmu-miR-5122 |
| 65 | mmu-miR-3096b-5p |
| 72 | mmu-miR-294-3p |
| 78 | mmu-miR-206-3p |
| 84 | mmu-miR-1930-3p |
| 96 | mmu-miR-1190 |
| 105 | mmu-miR-669I-3p |
| 107 | mmu-miR-539-3p |
| 108 | mmu-miR-5123 |
| 119 | mmu-miR-381-3p |
| 122 | mmu-miR-370-5p |
| 123 | mmu-miR-363-3p |
| 124 | mmu-miR-350-3p |
| 125 | mmu-miR-344h-3p |
| 126 | mmu-miR-330-3p |
| 127 | mmu-miR-3110-5p |
| 129 | mmu-miR-3070a-3p |
| 131 | mmu-miR-224-3p |
| 133 | mmu-miR-1961 |
| 135 | mmu-miR-1931 |
| 139 | mmu-miR-148a-5p |
| 140 | mmu-miR-130b-5p |
| 144 | mmu-miR-125b-5p |
| 146 | mmu-miR-27a-3p |
| 148 | mmu-miR-99a-5p |
| 149 | mmu-miR-99a-3p |
| 150 | mmu-miR-93-5p |
| 160 | mmu-miR-712-5p |
| 162 | mmu-miR-709 |
| 164 | mmu-miR-676-3p |
| 173 | mmu-miR-5622-5p |
| 177 | mmu-miR-544-5p |
| 187 | mmu-miR-491-3p |
| 189 | mmu-miR-488-3p |
| 193 | mmu-miR-431-5p |
| 198 | mmu-miR-376b-5p |
| 202 | mmu-miR-3470a |
| 215 | mmu-miR-3095-5p |
| 218 | mmu-miR-3089-5p |
| 232 | mmu-miR-302b-3p |
| 234 | mmu-miR-302a-3p |
| 235 | mmu-miR-301a-5p |
| 244 | mmu-miR-224-5p |
| 246 | mmu-miR-219-5p |
| 249 | mmu-miR-208b-5p |
| 250 | mmu-miR-208a-3p |
| 261 | mmu-miR-1933-3p |
| 263 | mmu-miR-18a-5p |
| 264 | mmu-miR-1894-5p |
| 267 | mmu-miR-148a-3p |
| 270 | mmu-miR-132-3p |
| 275 | mmu-miR-1251-3p |
| 281 | mmu-miR-103-2-5p |
| 283 | mmu-miR-100-3p |
| 287 | mmu-miR-107-5p |
| 288 | mmu-miR-10a-3p |
| 291 | mmu-miR-191-5p |

**Table 5: miRNAs reducing cell division**

| SEQ ID NO.: | miRNA |
|---|---|
| 516 | mmu-miR-9-3p |
| 517 | mmu-miR-136-5p |
| 518 | mmu-miR-155-5p |
| 519 | mmu-miR-193-3p |
| 520 | mmu-miR-204-5p |
| 521 | mmu-miR-143-3p |
| 522 | mmu-let-7c-5p |
| 523 | mmu-let-7e-5p |
| 524 | mmu-miR-29a-3p |
| 525 | mmu-miR-34a-5p |
| 526 | mmu-miR-320-3p |
| 527 | mmu-miR-379-5p |
| 528 | mmu-miR-196b-5p |
| 529 | mmu-miR-484 |
| 530 | mmu-miR-546 |
| 531 | mmu-miR-488-5p |
| 532 | mmu-miR-696 |
| 533 | mmu-miR-720 |
| 534 | mmu-miR-697 |
| 535 | mmu-miR-713 |
| 536 | mmu-miR-501-5p |
| 537 | mmu-miR-666-5p |
| 538 | mmu-miR-764-5p |
| 539 | mmu-miR-804 |
| 540 | mmu-miR-145-3p |
| 541 | mmu-miR-294-5p |
| 542 | mmu-miR-299-3p |
| 543 | mmu-miR-302a-5p |
| 544 | mmu-miR-330-5p |
| 545 | mmu-miR-340-5p |
| 546 | mmu-miR-139-3p |
| 547 | mmu-miR-362-3p |
| 548 | mmu-miR-409-5p |
| 549 | mmu-miR-671-3p |
| 550 | mmu-miR-881-3p |
| 551 | mmu-miR-297c-5p |
| 552 | mmu-miR-466h-5p |
| 553 | mmu-miR-467d-5p |
| 554 | mmu-miR-568 |
| 555 | mmu-miR-872-3p |
| 556 | mmu-miR-669d-5p |
| 557 | mmu-miR-669e-5p |
| 558 | mmu-miR-1197-3p |
| 559 | mmu-miR-1941-5p |
| 560 | mmu-miR-1953 |
| 561 | mmu-miR-1963 |
| 562 | mmu-miR-1966 |
| 563 | mmu-miR-1249-3p |
| 564 | mmu-miR-3058-3p |
| 565 | mmu-miR-344d-1-5p |
| 566 | mmu-miR-3060-3p |
| 567 | mmu-miR-3061-5p |
| 568 | mmu-miR-3065-5p |
| 569 | mmu-miR-3074-5p |
| 570 | mmu-miR-669d-2-3p |
| 571 | mmu-miR-3093-3p |
| 572 | mmu-miR-3100-3p |
| 573 | mmu-miR-344g-5p |
| 574 | mmu-miR-3102-3p.2-3p |
| 575 | mmu-miR-3104-3p |
| 576 | mmu-miR-3107-3p |
| 577 | mmu-miR-3112-5p |
| 578 | mmu-miR-130a-5p |
| 579 | mmu-miR-132-5p |
| 580 | mmu-miR-187-5p |
| 581 | mmu-let-7a-2-3p |
| 582 | mmu-miR-351-3p |
| 583 | mmu-miR-215-3p |
| 584 | mmu-miR-412-5p |
| 585 | mmu-miR-592-3p |
| 586 | mmu-miR-760-5p |
| 587 | mmu-miR-497-3p |
| 588 | mmu-miR-700-5p |
| 589 | mmu-miR-871-3p |
| 590 | mmu-miR-874-5p |
| 591 | mmu-miR-504-3p |
| 592 | mmu-miR-669k-5p |
| 593 | mmu-miR-466i-5p |
| 594 | mmu-miR-1193-5p |
| 595 | mmu-miR-5098 |
| 596 | mmu-miR-5106 |
| 597 | mmu-miR-5114 |
| 598 | mmu-miR-5134 |
| 599 | mmu-miR-1231-5p |
| 600 | mmu-miR-5617-5p |
| 601 | mmu-miR-5621-5p |
| 602 | mmu-miR-5621-3p |
| 603 | mmu-miR-5623-5p |
| 604 | mmu-miR-3073b-3p |
| 606 | mmu-miR-24-2-5p |

**Table 6: miRNAs promoting apoptosis**

| SEQ ID NO.: | miRNA |
|---|---|
| 296 | mmu-miR-9-5p |
| 297 | mmu-miR-133a-3p |
| 298 | mmu-miR-134-5p |
| 299 | mmu-miR-135a-5p |
| 300 | mmu-miR-137-3p |
| 301 | mmu-miR-154-5p |
| 302 | mmu-miR-183-5p |
| 303 | mmu-miR-185-5p |
| 304 | mmu-let-7d-3p |
| 305 | mmu-miR-29c-3p |
| 306 | mmu-miR-337-3p |
| 307 | mmu-miR-28-5p |
| 308 | mmu-miR-218-5p |
| 309 | mmu-miR-33-5p |
| 310 | mmu-miR-378-5p |
| 311 | mmu-miR-410-3p |
| 312 | mmu-miR-540-3p |
| 313 | mmu-miR-690 |
| 314 | mmu-miR-133a-5p |
| 315 | mmu-miR-673-5p |
| 316 | mmu-miR-744-5p |
| 317 | mmu-miR-183-3p |
| 318 | mmu-miR-29a-5p |
| 319 | mmu-miR-338-5p |
| 320 | mmu-miR-466a-5p |
| 321 | mmu-miR-882 |
| 322 | mmu-miR-466e-5p |
| 323 | mmu-miR-466g |
| 324 | mmu-miR-466j |
| 325 | mmu-miR-467g |
| 326 | mmu-miR-1906 |
| 327 | mmu-miR-1904 |
| 328 | mmu-miR-1943-5p |
| 329 | mmu-miR-1962 |
| 330 | mmu-miR-1839-5p |
| 331 | mmu-miR-3064-5p |
| 332 | mmu-miR-3068-3p |
| 333 | mmu-miR-3073-3p |
| 334 | mmu-miR-3091-5p |
| 335 | mmu-miR-3098-5p |
| 336 | mmu-miR-344c-5p |
| 337 | mmu-miR-3102-3p |
| 338 | mmu-miR-3104-5p |
| 339 | mmu-miR-3112-3p |
| 340 | mmu-miR-192-3p |
| 341 | mmu-miR-103-1-5p |
| 342 | mmu-miR-135a-2-3p |
| 343 | mmu-miR-452-3p |
| 344 | mmu-miR-669f-5p |
| 345 | mmu-miR-1948-5p |
| 346 | mmu-miR-1964-5p |
| 347 | mmu-miR-3096b-3p |
| 348 | mmu-miR-3968 |
| 349 | mmu-miR-5101 |
| 350 | mmu-miR-5709 |
| 351 | mmu-miR-3070a-5p // mmu-miR-3070b-5p |
| 352 | mmu-miR-669m-5p // mmu-miR-466m-5p |
| 507 | mmu-miR-96-5p |
| 508 | mmu-miR-200c-3p |
| 509 | mmu-miR-216a-5p |
| 510 | mmu-miR-761 |
| 511 | mmu-miR-18a-3p |
| 512 | mmu-miR-466k |
| 513 | mmu-miR-467h |
| 514 | mmu-miR-1955-5p |
| 515 | mmu-miR-3096-3p |
| 605 | mmu-let-7f-5p |
| 608 | mmu-miR-298-3p |

**Table 7: miRNAs promoting necrosis**

| SEQ ID NO.: | miRNA |
|---|---|
| 296 | mmu-miR-9-5p |
| 297 | mmu-miR-133a-3p |
| 298 | mmu-miR-134-5p |
| 299 | mmu-miR-135a-5p |
| 300 | mmu-miR-137-3p |
| 301 | mmu-miR-154-5p |
| 302 | mmu-miR-183-5p |
| 303 | mmu-miR-185-5p |
| 304 | mmu-let-7d-3p |
| 305 | mmu-miR-29c-3p |
| 306 | mmu-miR-337-3p |
| 307 | mmu-miR-28-5p |
| 308 | mmu-miR-218-5p |
| 309 | mmu-miR-33-5p |
| 310 | mmu-miR-378-5p |
| 311 | mmu-miR-410-3p |
| 312 | mmu-miR-540-3p |
| 313 | mmu-miR-690 |
| 314 | mmu-miR-133a-5p |
| 315 | mmu-miR-673-5p |
| 316 | mmu-miR-744-5p |
| 317 | mmu-miR-183-3p |
| 318 | mmu-miR-29a-5p |
| 319 | mmu-miR-338-5p |
| 320 | mmu-miR-466a-5p |
| 321 | mmu-miR-882 |
| 322 | mmu-miR-466e-5p |
| 323 | mmu-miR-466g |
| 324 | mmu-miR-466j |
| 325 | mmu-miR-467g |
| 326 | mmu-miR-1906 |
| 327 | mmu-miR-1904 |
| 328 | mmu-miR-1943-5p |
| 329 | mmu-miR-1962 |
| 330 | mmu-miR-1839-5p |
| 331 | mmu-miR-3064-5p |
| 332 | mmu-miR-3068-3p |
| 333 | mmu-miR-3073-3p |
| 334 | mmu-miR-3091-5p |
| 335 | mmu-miR-3098-5p |
| 336 | mmu-miR-344c-5p |
| 337 | mmu-miR-3102-3p |
| 338 | mmu-miR-3104-5p |
| 339 | mmu-miR-3112-3p |
| 340 | mmu-miR-192-3p |
| 341 | mmu-miR-103-1-5p |
| 342 | mmu-miR-135a-2-3p |
| 343 | mmu-miR-452-3p |
| 344 | mmu-miR-669f-5p |
| 345 | mmu-miR-1948-5p |
| 346 | mmu-miR-1964-5p |
| 347 | mmu-miR-3096b-3p |
| 348 | mmu-miR-3968 |
| 349 | mmu-miR-5101 |
| 350 | mmu-miR-5709 |
| 351 | mmu-miR-3070a-5p // mmu-miR-3070b-5p |
| 352 | mmu-miR-669m-5p // mmu-miR-466m-5p |
| 353 | mmu-miR-706 |
| 354 | mmu-let-7i-5p |
| 355 | mmu-miR-101a-3p |
| 356 | mmu-miR-125a-5p |
| 357 | mmu-miR-152-3p |
| 358 | mmu-miR-201-5p |
| 359 | mmu-miR-202-3p |
| 360 | mmu-miR-290-5p |
| 361 | mmu-miR-34c-5p |
| 362 | mmu-let-7b-5p |
| 363 | mmu-miR-351-5p |
| 364 | mmu-miR-135b-5p |
| 365 | mmu-miR-181c-5p |
| 366 | mmu-miR-217-5p |
| 367 | mmu-miR-380-3p |
| 368 | mmu-miR-215-5p |
| 369 | mmu-miR-448-3p |
| 370 | mmu-miR-449a-5p |
| 371 | mmu-miR-547-3p |
| 372 | mmu-miR-494-3p |
| 373 | mmu-miR-302c-5p |
| 374 | mmu-miR-302c-3p |
| 375 | mmu-miR-679-5p |
| 376 | mmu-miR-683 |
| 377 | mmu-miR-686 |
| 378 | mmu-miR-146b-5p |
| 379 | mmu-miR-467b-3p |
| 380 | mmu-miR-455-5p |
| 381 | mmu-miR-698 |
| 382 | mmu-miR-706 |
| 383 | mmu-miR-707 |
| 384 | mmu-miR-714 |
| 385 | mmu-miR-501-3p |
| 386 | mmu-miR-450b-3p |
| 387 | mmu-miR-505-3p |
| 388 | mmu-miR-718 |
| 389 | mmu-miR-675-5p |
| 390 | mmu-miR-374-3p |
| 391 | mmu-miR-665-3p |
| 392 | mmu-miR-758-3p |
| 393 | mmu-miR-763 |
| 394 | mmu-miR-202-5p |
| 395 | mmu-miR-15a-3p |
| 396 | mmu-miR-20a-3p |
| 397 | mmu-miR-31-3p |
| 398 | mmu-miR-93-3p |
| 399 | mmu-miR-337-5p |
| 400 | mmu-miR-339-3p |
| 401 | mmu-miR-345-3p |
| 402 | mmu-miR-20b-3p |
| 403 | mmu-miR-666-3p |
| 404 | mmu-miR-743b-5p |
| 405 | mmu-miR-883a-3p |
| 406 | mmu-miR-876-3p |
| 407 | mmu-miR-327 |
| 408 | mmu-miR-466b-3p // mmu-miR-466c-3p // mmu-miR-466p-3p |
| 409 | mmu-miR-467c-5p |
| 410 | mmu-miR-493-3p |
| 411 | mmu-miR-509-5p |
| 412 | mmu-miR-654-5p |
| 413 | mmu-miR-449b |
| 414 | mmu-miR-669k-3p |
| 415 | mmu-miR-1186 |
| 416 | mmu-miR-1187 |
| 417 | mmu-miR-669h-5p |
| 418 | mmu-miR-1195 |
| 419 | mmu-miR-1198-5p |
| 420 | mmu-miR-1897-5p |
| 421 | mmu-miR-1905 |
| 422 | mmu-miR-1907 |
| 423 | mmu-miR-1894-3p |
| 424 | mmu-miR-1933-5p |
| 425 | mmu-miR-1947-5p |
| 426 | mmu-miR-1948-3p |
| 427 | mmu-miR-1960 |
| 428 | mmu-miR-1946b |
| 429 | mmu-miR-1970 |
| 430 | mmu-miR-1971 |
| 431 | mmu-miR-1982-5p |
| 432 | mmu-miR-2139 |
| 433 | mmu-miR-1249-5p |
| 434 | mmu-miR-3099-3p |
| 435 | mmu-miR-3106-5p |
| 436 | mmu-miR-3106-3p |
| 437 | mmu-miR-3057-5p |
| 438 | mmu-miR-3061-3p |
| 439 | mmu-miR-3063-3p |
| 440 | mmu-miR-3069-5p |
| 441 | mmu-miR-3073-5p |
| 442 | mmu-miR-3079-5p |
| 443 | mmu-miR-3082-3p |
| 444 | mmu-miR-3084-5p |
| 445 | mmu-miR-466m-3p |
| 446 | mmu-miR-466n-5p |
| 447 | mmu-miR-466n-3p |
| 448 | mmu-miR-3092-5p |
| 449 | mmu-miR-3092-3p |
| 450 | mmu-miR-3096-5p |
| 451 | mmu-miR-3097-5p |
| 452 | mmu-miR-3097-3p |
| 453 | mmu-miR-3102-5p |
| 454 | mmu-miR-3102-5p.2-5p |
| 455 | mmu-miR-3108-5p |
| 456 | mmu-miR-3109-5p |
| 457 | mmu-miR-374c-5p |
| 458 | mmu-miR-1912-3p |
| 459 | mmu-miR-3471 |
| 460 | mmu-miR-1186b |
| 461 | mmu-miR-3474 |
| 462 | mmu-miR-137-5p |
| 463 | mmu-miR-146a-3p |
| 464 | mmu-miR-153-5p |
| 465 | mmu-miR-196a-1-3p |
| 466 | mmu-miR-1a-2-5p |
| 467 | mmu-miR-25-5p |
| 468 | mmu-miR-29b-2-5p |
| 469 | mmu-miR-92a-1-5p |
| 470 | mmu-miR-181b-1-3p |
| 471 | mmu-miR-133b-5p |
| 472 | mmu-miR-448-5p |
| 473 | mmu-miR-471-3p |
| 474 | mmu-miR-541-3p |
| 475 | mmu-miR-367-5p |
| 476 | mmu-miR-487b-5p |
| 477 | mmu-miR-669c-3p |
| 478 | mmu-miR-499-3p |
| 479 | mmu-miR-701-3p |
| 480 | mmu-miR-181d-3p |
| 481 | mmu-miR-466h-3p |
| 482 | mmu-miR-493-5p |
| 483 | mmu-miR-653-3p |
| 484 | mmu-miR-669e-3p |
| 485 | mmu-miR-1199-3p |
| 486 | mmu-miR-1947-3p |
| 487 | mmu-miR-1955-3p |
| 488 | mmu-miR-664-5p |
| 489 | mmu-miR-3964 |
| 490 | mmu-miR-3473b |
| 491 | mmu-miR-3473c |
| 492 | mmu-miR-5109 |
| 493 | mmu-miR-5118 |
| 494 | mmu-miR-5120 |
| 495 | mmu-miR-5121 |
| 496 | mmu-miR-3544-3p |
| 497 | mmu-miR-5615-3p |
| 498 | mmu-miR-1231-3p |
| 499 | mmu-miR-5616-3p |
| 500 | mmu-miR-5617-3p |
| 501 | mmu-miR-3073b-5p |
| 502 | mmu-miR-5710 |
| 503 | mmu-miR-1929-3p |
| 504 | mmu-miR-669a-5p // mmu-miR-669p-5p |
| 505 | mmu-miR-466b-5p // mmu-miR-466o-5p |
| 506 | mmu-miR-344e-5p // mmu-miR-344h-5p |
| 607 | mmu-miR-24-3p |
| 609 | mmu-miR-7b-5p |

The production efficiency and the total output of biomolecules that can be harvested from a cell culture depends on several cellular processes, of which the most important are protein cellular production of the biomolecule (translation/secretion), cell survival and cell proliferation, regulation of which is even interrelated. By introducing at least two miRNAs and/or miRNA-inhibitors that influence different cellular processes, a multitude of cellular pathways can be optimized resulting in an increased yield of the biomolecule of interest. Each of the miRNA and miRNA-inhibitors influences a variety of target genes of several interrelated cellular pathways, thereby influencing the composition of proteins within the cell. In contrast to the overexpression of one or several enzymes for enhancing protein synthesis, shifting the balance within the cells' endogenous protein pool does not withdraw energy from the production of the desired biomolecule. Expressing a miRNA actually reduces translation, thus, releasing energy and protein translation capacity for production of the biomolecule. Moreover, the limiting factor of productivity may differ depending on the biomolecule produced, or may even change during cultivation depending on the culture conditions. By influencing the composition of a large variety of proteins by controlling miRNAs, it is possible to regulate entire pathways. Thereby, it is possible to overcome various limitations, which could not be addressed by overexpressing a single synthesis enzyme or inhibiting or knocking out a single protein degrading enzyme.

The term "biomolecule" as used herein refers to any compound suitable to be produced by a cell and harvested therefrom. Preferably, the biomolecule is a biopharmaceutical, i.e. a pharmaceutical including therapeutics, prophylactics and diagnostics, which is inherently biological in nature and manufactured using biotechnology. Biopharmaceuticals include *inter alia* antibodies, enzymes, hormones, vaccines but also viruses, e.g. oncolytic viruses and viruses used for gene therapy. Thus, the biopharmaceutical is preferably a recombinant molecule, more preferred a recombinant protein or a recombinant virus.

The term "miRNA-inhibitor" as used herein refers to any compound suitable to specifically reduce the amount of a given miRNA within a cell. miRNA-inhibitors include for example nucleic acid molecules that specifically bind to the miRNA of interest thereby preventing its binding to the target mRNA. Such inhibitors include antagomirs, miRNA sponge and miRNA decoy. Antagomirs are small oligonucleotides that are perfectly complementary to the targeted miRNA, whereas miRNA sponge and RNA decoy are nucleic acid molecules comprising multiple tandem binding sites to the miRNA of interest. Due to the multiple binding sites, the molecules act as strong competitive inhibitors of the miRNA (Ebert and Sharp, 2010). Accordingly, the miRNA-inhibitor is preferably selected from the group consisting of antagomir, miRNA sponge and miRNA decoy. Alternatively, the miRNAs inhibitors may target a regulatory element of the miRNA of interest, e.g. its promoter or enhancer.

In a preferred embodiment, the nucleic acid construct comprises three different regions. By comprising at least one miRNA and/or miRNA-inhibitor influencing each of cellular production, cell death and cell proliferation, the cell's efficiency in producing the biomolecule can be optimized.

In a preferred embodiment, at least one region, preferably each region, encodes for at least two, three, four or five different miRNAs and/or miRNA-inhibitors. Any region may encode for more than one miRNA or miRNA-inhibitor. For example, several miRNAs belonging to the same family and thus targeting related mRNAs, may be comprised. Thereby, it is possible to strengthen the regulation of one particular pathway as e.g. observed by a combined introduction of several members of the miR-30 family. Alternatively, a variety of miRNAs targeting different pathways may be used to produce a more wide spread effect. Cell processes as proliferation, protein synthesis and cell death are usually regulated by more than one signalling pathway, of which many are interrelated. Thus, targeting several pathways is particularly advantageous in cases in which it is not known which cellular pathways present the limiting factor for biomolecule production. Due to the rather small size of miRNAs, the nucleic acid constructs of the invention may encode for 20 miRNAs and/or miRNA-inhibitors, or even more. The term "region" as used herein refers to sections along the nucleic acid construct comprising a part that is transcribed into a miRNA or a miRNA-inhibitor as e.g. an antagomir or a miRNA decoy. The region may further comprise regulatory elements to control the transcription of the miRNA or miRNA-inhibitor, such as promoters, operators (e.g. enhancers, repressors and insulators), 3'UTR regulatory elements (e.g. siRNA binding sites, miRNA binding sites) or splicing signals.

In a preferred embodiment, the at least two different regions are controlled by different promoters. This is to say that each region comprises its own regulatory elements, such that the transcription of the miRNA and/or miRNA-inhibitor comprised in said region can be regulated independently of the miRNAs and/or miRNA-inhibitors contained in other regions of the construct. This is advantageous if cell proliferation and cellular production should be regulated at different time points during cell culture. When inducing the culture, cell proliferation can be promoted whereas once an optimal cell density is reached, cellular productivity may be enhanced. Regulation of cell death could be specifically induced depending on the state of the culture. Moreover, using independent regulatory elements allows providing the miRNAs and miRNA-inhibitors for different cellular processes at various amounts. For example, miRNAs stimulating cellular production may be set under a strong promoter, whereas those miRNAs or miRNA-inhibitors influencing cell death may be regulated by a weaker promoter.

In a preferred embodiment, the at least two different regions are controlled by one common promoter. This allows a fast and easy preparation of the nucleic acid construct and is preferably applied in cases in which a rather simple regulation already results in satisfying yields of the biomolecule.

In a preferred embodiment, at least one promoter is inducible or inhibitable. Inducible/Inhibitable promoters are characterized in that their activity depends on external circumstances, such as temperature, light, oxygen or the presence of chemical compounds. Using inducible or inhibitable promoters, it is possible to exactly determine the time point during the cell culture when transcription of one or all regions of the construct is initiated and/or terminated. Inducible regulatory elements include for example the tetracycline/doxycycline "Tet-On"-system, inhibitable regulatory elements include for example the "Tet-Off'-system or regulated optogenetic gene expression systems, temperature controlled promotors and TrsR-based systems (quorum sensing based).

In a preferred embodiment, the nucleic acid construct is an expression vector, an episomal vector or a viral vector. For expressing a miRNA and/or a miRNA-inhibitor within a cell, the nucleic acid construct needs to be introduced into the cell. This is possible by different means, for example by transfection, i.e. non-viral methods for transferring a nucleic acid molecule into eukaryotic cells. For such applications, the nucleic acid construct is preferably provided as an expression vector or an episomal vector. Alternatively, the nucleic acid construct can be introduced into a cell by transduction, i.e. by a virus-mediated transfer of the nucleic acid into the cell. For such applications, the nucleic acid construct is preferably provided as a viral vector.

In a further aspect, disclosed is a cell comprising a nucleic acid construct of the present disclosure. Such cells are suitable for producing a biomolecule, wherein the efficiency of production and the overall yield is optimized by regulating at least two miRNAs involved in different cellular processes. Such cells are preferably used in biopharmaceutical manufacturing.

In a preferred embodiment, the construct is integrated into the cell's genome. By introducing a construct according to the invention into the cell's endogenous genome, a stable cell line for biopharmaceutical manufacturing can be provided. Such cell lines produce biomolecules at constant and reliable amounts and are, thus, particularly preferred for large scale productions, which are usually operated to provide established and highly demanded biopharmaceuticals. Moreover, by integrating the nucleic acid construct into the genome, the probability of loosing the construct during continuous cell proliferation is reduced and the nucleic acid construct is present throughout the entire culture's lifetime. Accordingly, the cell is preferably a stable cell line cell.

In a preferred embodiment, the construct is introduced into the cell by transfection. Transient transfection is an easy and fast way to provide a given cell with new properties. It is neither labour nor cost intensive and does not need extensive selection processes. Introducing the nucleic acid construct by transfection is particularly preferred where biomolecules need to be produced on short term notice or only small amounts of the biomolecule are needed, such that the labour-intensive establishment of a stable cell line would be inefficient.

In a preferred instance, a region of the cell's genome encoding for at least one miRNA selected from group 1, 2, 4 and/or 5 is amplified, and/or a region of the cell's genome encoding for at least one miRNA selected from group 3, 4 and/or 5 is deleted or silenced. Besides introducing a nucleic acid construct of the invention into a cell, a miRNA may be provided or inhibited by altering the cells endogenous expression of the miRNA. For increasing the level of a specific miRNA, the region of the cells genome encoding for this miRNA may be amplified. Likewise, the region encoding for an endogenous miRNA may be deleted such that the miRNA is no longer present in the cell. Instead of deleting the gene encoding for the miRNA, the levels of miRNA within the cell may be reduced by silencing, e.g. using competitive inhibitors.

The cell of the present disclosure may be a mammalian cell. Mammalian cells are particularly preferred for producing biomolecules of complex structures, as for example proteins comprising sophisticated post-translational modifications. Mammalian cells endogenously comprise the synthesis pathways necessary for generating, folding and modifying complex proteins. A variety of cellular systems derived from different origins as e.g. from hamster, mouse, duck or human are available.

The mammalian cell may be a Chinese hamster ovary cell (CHO), preferably a CHO-K1 cell, a CHO DG44 cell, a CHO DUKX B11 cell, a CHO dhfr cell or a CHO-S cell.

The cell may be a human cell, preferably a kidney cell, a liver cell, an embryonic retina cell, an amniocytic cell or a mesenchymal stem cell. Cellular production systems derived from human cells are preferred for the production of biomolecules of human origin, in particular if these are intended to be used in human medicine. Marginal alternations of the biomolecules due to incorrect folding of modification may cause the protein to be less active or even to show adverse effects.

The cell may be an insect cell, preferably a Sf9, Sf21, TriEX™ or a Hi5 cell. Such cell systems are particularly preferred for the production of molecules, e.g. proteins, which originate from other systems, in which they exert essential functions. Expressing such proteins in their natural cellular environment would disturb the cellular processes of the producing cell or even result in cell death. This would significantly impair the production efficiency of the biomolecule, strongly limiting the yield that can be achieved. For example, certain human receptor molecules with significant influence on cellular pathways can be produced in high yields from insect cells, as they do not exert any biological effect in these cells.

In a further aspect, disclosed is a method for increasing the yield of a biomolecule produced by a cell cultured *in vitro* comprising at least two steps selected of stimulating cellular production of the biomolecule by increasing the level of at least one miRNA selected from group 1 in the cell, reducing cell death by increasing the level of at least one miRNA selected from group 2 in the cell and/or decreasing the level of at least one miRNA selected from group 3, and regulating proliferation of the cell by increasing the level of at least one miRNA selected from group 4 or 5 in the cell and/or decreasing the level of at least one miRNA selected from group 4 or 5. The term "yield of a biomolecule" as used herein refers to the volumetric productivity of an entire culture, i.e. the total amount of a biomolecule of interest that can be harvested from a culture. For producing the biomolecule, a cell culture is established, preferably from a stable cell line that is adapted to produce the biomolecule of interest. In case the biomolecule is a protein, this may be achieved by introducing one or multiple copies of a gene encoding for the desired protein. By using the described method, at least two cellular processes of cellular production, proliferation and cell survival are influenced via regulating the level of specific miRNAs. By balancing cellular production capacity, cell proliferation and cell survival, it is possible to increase the output of biomolecule without loading the cell with additional translational burden that would increase the cell cultures consumption of energy and nutrients.

In a preferred embodiment, the level of at least one miRNA is increased by overexpressing the miRNA in the cell, by electroporating the cell in the presence of the miRNA or by adding the miRNA and a transfectant to a medium, in which the cell is cultured. Alternatively, the miRNA and the transfectant may be added to a buffer into which the cells are transferred for transfection. For increasing the level of a miRNA within a cell two distinct approaches are available. A nucleic acid molecule encoding for the miRNA may be introduced into a cell such that the cellular transcription machinery expresses the miRNA from the construct. This may be achieved by use of an expression vector or a viral vector that is maintained in the cell as an individual episomal molecule or integrates into the cell's genome, or by use of a stable cell line. Alternatively, the level of a miRNA within a cell may be increased by providing the miRNA as a RNA molecule, e.g. as a pri- or pre-miRNA, a mature miRNA or a miRNA mimic. For example, the RNA molecules are added to the culture together with a transfectant, i.e. lipofectamine (Invitrogen), which contains lipid subunits that form liposomes encapsulating the nucleic acid or miRNA. The liposomes then fuse with the membrane of the cell, such that the nucleic acid becomes introduced into the cytoplasm.

In a preferred embodiment, the level of at least one miRNA is decreased by deleting the region of the cell's genome encoding for the miRNA or regulating its transcription by expressing a miRNA-inhibitor in a cell directed against the miRNA, by electroporating the cell in the presence of the miRNA-inhibitor or by adding a miRNA-inhibitor and a transfectant to a medium, in which the cell is cultured. Alternatively, the miRNA-inhibitor and the transfectant may be added to a buffer into which the cells are transferred for transfection. Reduction of the level of a miRNA may be achieved by various approaches. For example, the endogenous gene encoding for the miRNA may be deleted from the cell's genome. This is preferred when the biomolecule should be produced by a stable cell line. However, this approach may be irreversible in some cases. Alternatively, the endogenous gene encoding for the miRNA may be put under an inducible regulatory element, such that transcription of the miRNA may be determinably activated or inactivated. Besides that, an endogenous miRNA may be also inhibited by providing a competitive inhibitor, e.g. an antagomir or RNA sponge. These may be expressed within the cell upon transfection or may be added as a RNA molecule to the culture together with a transfectant. Instead of a single approach, a combination of different approaches may also be applied.

In a preferred embodiment, cell death is reduced by reducing apoptosis by increasing the level of at least one miRNA selected from group 2 in the cell and/or decreasing the level of at least one miRNA selected from group 6. Two major types of cell death are known, which differ distinctly from each other. Apoptosis, also called programmed cell death, involves a distinct sequence of cellular transformations which is usually initiated as a result from failing cellular processes. Necrosis, in contrast, describes a rather traumatic dissolving of the cell usually initiated by external impacts, e.g. cellular damage. According to cell type and culture conditions one type of cell death may be more prominent than the other. Interestingly, the inventors found several miRNAs involved in regulating both apoptosis and necrosis. Moreover, with respect to apoptosis, inhibiting as well as promoting miRNAs were identified (groups 2 and 6, respectively). In contrast, regarding necrosis, exclusively promoting miRNAs were found (group 7). Depending on the specific cell culture and on the culture conditions, apoptosis or necrosis may be more prevalent during biomolecule production. Accordingly, in a preferred embodiment, cell death is reduced by attenuating necrosis by decreasing the level of at least one miRNA selected from group 7.

In a further aspect, disclosed is a method for producing a biomolecule in a cell comprising the steps of propagating the cell in a cell culture, increasing the level of at least one miRNA selected from the group consisting of SEQ ID NO.: 1, 2, 3, 4, 5, 6, 7, 8, 9, 10, 11, 12, 13, 14, 15, 16, 17, 18, 19, and 20, and isolating the biomolecule from the cell culture. When revealing miRNAs specifically regulating distinct cellular processes such as cellular production, proliferation and cell survival, the inventors further found certain miRNAs, which influence more than one of these processes. miR-99b-3p (SEQ ID NO.: 1) not only increases the cellular production of a biomolecule, but also shows an anti-apoptotic effect. Similar combined effects were observed for miR-767 (SEQ ID NO.: 2), miR-30a-5p (SEQ ID NO.: 3), miR-3062-5p (SEQ ID NO.: 4), miR-200a-5p (SEQ ID NO.: 6), miR-135a-1-3p (SEQ ID NO.: 8), miR-743a-5p (SEQ ID NO.: 9) and miR-30d-5p (SEQ ID NO.: 20). miR-291b-3p (SEQ ID NO.: 5) and miR-1a-3p (SEQ ID NO.: 7) were found to promote both cell survival and cell proliferation resulting in an overall increased yield of the produced biomolecule. Additionally, miR-694 (SEQ ID NO.: 10), miR-674-3p (SEQ ID NO.: 11), miR-669d-3p (SEQ ID NO.: 12); miR-301b-5p (SEQ ID NO.: 13), miR-212-5p (SEQ ID NO.: 14), miR-203-5p (SEQ ID NO.: 15), miR-200b-5p (SEQ ID NO.: 16), miR-200a-3p (SEQ ID NO.: 17), miR-1968-5p (SEQ ID NO.: 18) and miR-150-3p (SEQ ID NO.: 19) were found to influence both, proliferation and cellular productivity. Increasing one or more of these miRNAs provides an easy and efficient approach for optimizing the production of a biomolecule. For introducing the miRNAs into the cell any of the methods mentioned herein or combinations thereof may be used.

In a further aspect, disclosed is the use of a combination of at least one miRNA selected from group 1, at least one miRNA selected from group 2 and/or at least one miRNA-inhibitor inhibiting a miRNA selected from group 3, and at least one miRNA selected from group 4 or 5 and/or at least one miRNA-inhibitor inhibiting a miRNA selected from group 4 or 5. A combination of a miRNA promoting cellular production, a miRNA or miRNA-inhibitor suppressing cell death and/or a miRNA or miRNA-inhibitor regulating cell proliferation may be provided in various forms. For example, the miRNAs/inhibitors may be provided as a single nucleic acid construct. Alternatively, a multitude of nucleic acid molecules each encoding for a subset of miRNAs may be provided e.g. one expression vector encoding for at least one miRNA of group 1, a second expression vector encoding for a miRNA of group 2 and a third expression vector encoding for a miRNA-inhibitor directed against a miRNA of group 5. Likewise, the miRNAs and miRNA-inhibitors may be provided as a compilation of several pri- or pre-miRNA molecules or miRNA mimics. Thus, the miRNAs may be provided as a kit comprising the diverse miRNAs and/or inhibitors in a single composition or separately.

In a further aspect, disclosed is a nucleic acid construct comprising a region encoding for at least one miRNA selected from the group consisting of SEQ ID NO.: 1 - 295 and/or a region encoding for at least one inhibitor directed against at least one miRNA selected from the group consisting of SEQ ID NO.: 296 - 609. All of the miRNAs of SEQ ID NO.: 1 - 295 were found to promote total biomolecule production, such that an increased amount of biomolecule could be harvested form cultures overexpressing any of these miRNAs. For most miRNAs, specific effects on distinct pathways (namely cell proliferation, cell death and cellular productivity) were found, which are supposed to account, at least partially, for the observed increase in overall biomolecule production. Accordingly, each of the miRNAs alone or in combination is suitable to enhance biomolecule production from a production cell. Additionally, some miRNAs appear to influence the cell's performance more generally, leading to an overall increase in volumetric production without significant alterations of cell survival, proliferation or cellular production. These miRNA were miR-721 (SEQ ID NO.: 157), miR-107-3p (SEQ ID NO.: 286), miR-181a-1-3p (SEQ ID NO.: 290) and miR-19b-2-5p (SEQ ID NO.: 292). It is suggested that these miRNAs instead of significantly altering one or two of said processes, rather influence all of them and possibly further cell signalling pathways. Similar to most of the miRNAs of SEQ ID NO.: 1 - 295 each of SEQ ID NO.: 296 to 609 were found to exert effects on cell proliferation and/or cell death. Inhibition of a single or a plurality of these miRNAs is suitable to promote cell survival and/or proliferation, leading to an increase in overall biomolecule production.

Accordingly, in a further aspect, disclosed is a method for increasing the yield of a biomolecule produced by a cell cultured *in vitro* comprising the steps increasing the level of at least one miRNA selected from the group consisting of SEQ ID NO.: 1 - 295 in the cell, and/or decreasing the level of at least one miRNA selected from the group consisting of SEQ ID NO.: 296 - 609 in the cell.

Furthermore, disclosed is a method for producing a biomolecule in a cell comprising the steps propagating the cell in a cell culture, increasing the level of at least one miRNA selected from the group consisting of SEQ ID NO.: 1 - 295 in the cell, and/or decreasing the level of at least one miRNA selected from the group consisting of SEQ ID NO.: 296 - 609 in the cell, and isolating the biomolecule from the cell culture.

The miRNA and/or miRNA-inhibitor may be added to the cell culture by electroporation or together with a transfectant, or is introduced to the cell culture by a viral vector.

In a further aspect, disclosed is the use of at least one miRNA selected from group 1 for stimulating cellular production of a biomolecule produced by a cell cultured *in vitro.* The miRNAs of group 1 all showed a significant increase in cellular production, leading to an increase in the amount of biomolecule that was produced by the entire culture.

In a further aspect, disclosed is the use of at least one miRNA selected from group 2 and/or a miRNA-inhibitor directed against a miRNA of group 3 for suppressing cell death of a cell cultured *in vitro.* By overexpressing any of the miRNAs of group 2 and/or inhibiting any of the miRNAs of group 3, cell survival is promoted, increasing the total number of production cells. This in turn results in an increased amount of total biomolecule produced.

In a further aspect, disclosed is the use of at least one miRNA selected from group 4 or 5 and/or a miRNA-inhibitor directed against a miRNA of group 4 or 5 for regulating proliferation of a cell cultured *in vitro.* Cell proliferation may be specifically regulated depending on the state of the culture. At the beginning of the culture, proliferation may be enhanced to reach an optimal cell density as fast as possible. This may be achieved by overexpressing any of the miRNAs of group 4 and/or inhibiting any of the miRNAs of group 5. In contrast, once the culture is fully established, a reduction of proliferation may be advantageous to provide more capacity to the production of the biomolecule. This may be achieved by overexpressing any of the miRNAs of group 5 and/or by inhibiting any of the miRNAs of group 4.

### Examples

### Materials and methods

### Cell culture

Suspension-adapted CHO-SEAP cells, established from CHO DG44 cells (Life Technologies, Carlsbad, CA, USA), were grown in TubeSpin® bioreactor 50 tubes (TPP, Trasadingen, Switzerland) in ProCHO5 culture medium (Lonza, Vervier, Belgium), supplemented with 4 mM L-Glutamine (Lonza) and 0.1 % anti-clumping agent (Life Technologies). Culture medium for stable miRNA overexpressing CHO-SEAP cells was additionally supplemented with 10 µg/mL puromycin-dihydrochloride (InvivoGen, San Diego, CA, USA). Generally, cell concentration of the pre-cultures was adjusted to 0.5 x 10⁶ viable cells per ml one day prior to transfection to ensure exponential growth and the cells were maintained at 37 °C, 5 % CO₂ and 85 % humidity with agitation at 140 rpm (25 mm orbit) in an orbital shaker incubator (Sartorius Stedim, Goettingen, Germany or Kuehner, Birsfelden, Switzerland).

### Transfection

Non-viral delivery of miRNA mimics or small interfering RNAs (siRNAs) was performed using ScreenFect®A (InCella, Eggenstein-Leopoldshafen, Germany). Small scale transfections for the primary and secondary screening were conducted in U-bottom shaped 96-well suspension culture plates (Greiner, Frickenhausen, Germany). For secondary screening, selected miRNA mimics were transfected again and plates were placed on a Mini-Orbital digital shaker (Bellco, Vineland, USA) located inside a Heraeus® BBD 6220 cell culture incubator (Thermo Scientific) at 37 °C, 5% CO₂, 90% humidity and agitation at 800 rpm. Scaled up transfections for target validation were carried out in 12-well suspension culture plates (Greiner) and plates were incubated in an orbital shaker incubator with agitation at 140 rpm. An entire murine miRNA mimics library (based on Sanger miRBase release 18.0) comprising 1139 different miRNA mimics (Qiagen, Hilden, Germany) was used for transfection and all transfections were done in biological triplicates. An Alexa Fluor®647 labeled non-targeting siRNA (AF647-siRNA) (Qiagen) was co-transfected with each effector and control miRNA as indicator of transfection efficiency. As functional transfection controls, an anti-SEAP siRNA (Qiagen), a CHO-specific anti-proliferative (used for the primary screen) as well as a cell death control siRNA (secondary screen) were used. A non-targeting, scrambled miRNA (Qiagen) was used as negative control (miR-NT). For plasmid DNA (pDNA) transfections, CHO-SEAP cells were nucleofected employing the NEON® transfection system (Life Technologies). 1.0 x 10⁷ viable cells were pelleted and resuspended in 110 µL of Buffer R (Life Technologies) followed by the addition of 25 µg endotoxin-free pDNA. Cells were nucleofected with one pulse at 1650 volts for 20 milliseconds and seeded in 10 mL of fresh culture medium. Transfected cells were subjected to antibiotic selection pressure 48 h post transfection by adding 10 µg/mL of puromycin-dihydrochloride to the cultures.

### Cloning of miRNA expression vectors

Native miRNA precursor (pre-miR) sequences of *Cricetulus griseus (cgr)* cgr-MIR30a, cgr-MIR30c-1, and cgr-MIR30e were obtained by polymerase chain reaction (PCR) from hamster genomic DNA (gDNA). Therefore, gDNA was isolated from CHO-SEAP cultures. PCR from gDNA was performed using a 1:1 mixture of two different DNA polymerases from *Thermus aquaticus (Taq)* and *Pyrococcus furiosus (Pfu)* (Fisher Scientific, St.Leon Rot, Germany). The following PCR primers were used to amplify pre-miR sequences including approximately 100 bp of upstream and downstream genomic flanking regions: *cgr*-MIR30a (332 bp PCR fragment length), forward 5'-TTGGATCCAGGGCCTGTATGTGTGAATGA-3' (SEQ ID NO.: 610), reverse 5'-TTTTGCTAGCACACTTGTGCTTTAGAAGTTGC-3' (SEQ ID NO.: 611), *cgr*-MIR30c-1 (344 bp PCR fragment length), forward 5'-TTGGATCCAAAATTACTCAGCCC-ATGTAGTTG-3' (SEQ ID NO.: 612), reverse 5'- TTTTGCTAGCTTAGCCAGAGAAGTG-CAACC-3' (SEQ ID NO.: 613); cgr-MIR30e (337 bp PCR fragment length), forward 5'-TTGGATCCATGTGTCGGAGAAGTGGTCATC-3' (SEQ ID NO.: 614), reverse 5'-TTTTGCTAGCCTCCAAAGGAAGAGAGGCAGTT-3' (SEQ ID NO.: 615). Amplified PCR products contained *Bam*HI*lNhe*I restriction sites at their respective ends which were introduced by the PCR primers. Digested PCR fragments were ligated into a miRNASelect™ pEGP-miR expression vector (Cell Biolabs, San Diego, CA, USA) between *Bam*HI and *Nhe*I restriction sites employing the Rapid DNA Dephos & Ligation Kit (Roche Diagnostics). The correct integration of the pre-miR sequences was confirmed for all miRNAs by DNA sequencing (SRD, Bad Homburg, Germany). The miRNASelect™ pEGP-miR-Null vector (Cell Biolabs) which lacks any pre-miR sequence served as negative control.

### Quantitative flow cytometry

For cellular analysis, transfected CHO-SEAP cells were analyzed for cell concentration, viability, necrosis and transfection efficiency 72 h post transfection. Cells were analyzed by high-throughput quantitative flow cytometry employing a MACSQuant® Analyzer (Miltenyi Biotech, Bergisch-Gladbach, Germany) equipped with a violet (405 nm), blue (488 nm) and red (635 nm) excitation laser. 40 µL of a 3X staining solution [ProCHO5 medium (Lonza) supplemented with 15 µg/mL propidium iodide (PI) (Roth, Karlsruhe, Germany), 6 µg/mL Calcein-Violet450-AM (eBioscience, Frankfurt, Germany), 0.5 mM EDTA] were added to 80 µL of cell suspension and incubated for 20 min at 37 °C, 5 % CO₂ and 85 % humidity. Subsequently, cells were counted and viability was measured by means of Calcein-Violet450-AM staining. Necrotic/late apoptotic cells were detected by PI exclusion and transfection efficiency was determined by analyzing viable cells for Alexa Fluor®647 fluorescence.

### Analysis of apoptosis

Transfected cells were analyzed for apoptosis using a Nicoletti staining procedure. Cells with a DNA content less than 2n (= Sub-G₀/G₁ cells) were classified as apoptotic. Hence, 50 µL of transfected cell suspension was transferred to a new 96-well microplate containing 100 µL of culture medium per well and centrifuged for 5 min at 150 x g. 100 µL of supernatant was transferred to another 96-well microplate used for SEAP protein quantification. The cell pellet was resuspended in 100 µL of Nicoletti staining solution (1X Phosphate buffered saline (PBS) supplemented with 0.1 % sodium citrate, 0.05 % Triton X-100, 10 µg/mL PI and 1 U/µL RNase A) and incubated in the dark for 30 min at 4 °C. Treated cells were analyzed by quantitative flow cytometry on a MACSQuant® Analyzer (Miltenyi Biotech).

### SEAP quantification

SEAP protein levels in the culture supernatant of transfected cells were quantified in white 96-well non-binding microplates using a SEAP reporter gene assay (Roche Diagnostics). In principle, the chemiluminescent substrate CSPD (3-(4-methoxyspiro[1,2-dioxetane-3,2'(5'-chloro)-tricyclo(3.3.1.1^{3,7})decane]-4-yl)phenylphosphate) is dephosphorylated by SEAP, resulting in an unstable dioxetane anion that decomposes and emits light at a maximum wavelength of 477 nm. Endogenous alkaline phosphatases were inhibited by incubating the samples for 30 min at 65 °C following a chemical inactivation using a provided Inactivation Buffer (Roche Diagnostics). Due to high SEAP expression levels of the CHO-SEAP cell line, culture supernatants were pre-diluted 1:60 in fresh culture medium. Chemiluminescence was detected after addition of CSPD substrate using a SpectraMax® M5e microplate reader (Molecular Devices, Sunnyvale, CA, USA).

### qRT-PCR analysis

Total RNA (including small RNAs <200 bp) was isolated using the miRNeasy mini Kit (Qiagen) according to the protocol provided by the manufacturer. RNA concentration and purity was determined by UV-spectrometry using a NanoDrop® spectrophotometer (Thermo Scientific). Complementary DNA (cDNA) was synthesized from 1 µg total RNA using the miScript II RT Kit (Qiagen). RT-PCR was performed with 20⁻¹ diluted cDNA using the miScript SYBR green PCR kit (Qiagen) for detection of mature miRNAs on a LightCycler® 480 (Roche Diagnostics). The following miRNA-specific primers were used: mature miR-30a-5p forward, 5'-TGTAAACATCCTCGACTGGAAGC-3' (SEQ ID NO.: 616); miR-30b-5p forward, 5'-TGTAAACATCCTACACTCAGCT-3' (SEQ ID NO.: 617); miR-30c-5p forward, 5'-TGTAAACATCCTACACTCTCAGC-3' (SEQ ID NO.: 618); miR-30d-5p forward, 5'-CTTTCAGTCAGATGTTTGCTGC-3' (SEQ ID NO.: 619); miR-30e-5p forward, 5'-TGTAAACATCCTTGACTGGAAGC-3' (SEQ ID NO.: 620); the miScript Universal Primer (Qiagen) served as reverse primer for each mature miRNA; U6 forward, 5'-CTCGCTTCGGCAGCACA-3' (SEQ ID NO.: 621); U6 reverse, 5'-AACGCTTCACGAATTTGCGT-3' (SEQ ID NO.: 622). Relative mature miRNA expression differences were calculated by applying the comparative C(T) method.

### Results

### Transient high-content miRNA screen in recombinant CHO-SEAP suspension cells

The inventors performed a high-content microRNA screen using 1139 different miRNA mimics in a recombinant CHO-SEAP suspension cell line to identify miRNAs improving cellular function. In this conjunction, all transfected cells were analyzed for various cellular parameters employing a multiparametric flow cytometry-based cell analysis. Transfection conditions for small double-stranded RNAs in 96-well format were carefully optimized and several functional controls were used which included a non-targeting control miRNA (miR-NT), a siRNA against the SEAP mRNA (anti-SEAP siRNA) as well as a CHO-specific anti-proliferative siRNA. Using a novel non-viral transfection reagent (ScreenFect®A) which has previously been demonstrated to efficiently and functionally deliver miRNA mimics into cells grown in a complex production medium (Fischer et al., 2013), high transfection rates of >95 % could be reproducibly achieved at low cytotoxicity rates. As delivery control, a fluorescently-labelled non-targeting siRNA (AlexaFluor®647-siRNA) was co-transfected with all effector and control miRNAs/siRNAs, respectively. Cell concentration, viability, necrosis and transfection efficiency was measured 72 h post transfection by high-throughput quantitative flow cytometry. Analysis of apoptotic cell death by means of Nicoletti staining was also performed on a quantitative flow cytometer. SEAP protein concentrations were determined using a commercially available SEAP reporter assay. A cultivation period of three days was chosen to account for both the time-limited transient effects of miRNA mimics and the manifestation of changes in cell phenotype. A significant decrease in SEAP productivity of cells transfected with an anti-SEAP siRNA as well as significant decrease in the viable cell density (VCD) of cells transfected with an anti-proliferative siRNA was indicative for functional transfections in all screen plates (Figure 1). In addition, spiked-in AlexaFluor®(AF) 647-siRNA confirmed uptake of miRNA mimics in each well.

Data normalization was performed to allow for inter-plate comparisons by normalizing each sample value to the mean value of the respective on-plate control (miR-NT). Significant changes on each readout parameter were determined by applying a one-way analysis of variances (ANOVA) combined with a Dunnett's multiple comparison test (against the on-plate miR-NT control; p<0.05). The normalized mean values for all 1139 effector miRNAs considering important cell characteristics such as VCD, apoptosis, necrosis/late apoptosis, specific and volumetric SEAP productivity were determined. Cake charts indicating the number of statistically significant hits as percentage of all mimics tested are shown in Figure 2. Regarding SEAP productivity, a large proportion of 16 % of the transfected miRNAs significantly increased SEAP yields in the supernatant after 72 h with the best candidates showing an improvement of up to two-fold (Figure 2A). Significantly elevated cell-specific productivity was even detected for 21 % of the miRNAs (Figure 2B). However, this was partly in conjunction with a decreased cell concentration without inducing cell death. In particular, of the 314 miRNAs which increased mean specific productivity by at least 20 % were also found to decrease mean VCD by up to 69 % three days post-transfection without lowering cell viability. Significantly higher viable cell densities were determined for 5 % of all miRNAs, whereas 13 % of all miRNAs decreased apoptosis rates (Figures 2C and D). The percentage of miRNAs boosting cell proliferation was in line with the fact that 4 % of the miRNA library decreased the number of necrotic cells indicating higher viabilities following miRNA introduction (Figure 2E).

### Screen analysis identified functionality of miR-30 family

In order to validate the results obtained through the primary screen the inventors performed a secondary screen by transiently transfecting a subset of selected miRNA hits in agitated cultures. An agitated culture mode in multi-well plates is much more comparable to standard shaking flask cultivations, in which putative oxygen limitations of static cultures are substantially overcome. Shaking speed for 96-well plates together with the miRNA mimics concentration was carefully optimized to allow for robust cultivation and transient transfection in suspension. In a first step, 297 miRNA hits derived from the primary cellular screen were selected for a reassessment of their positive influence on at least one of the bioprocess relevant cellular parameters mentioned above. This approach confirmed phenotypic effects for most miRNAs as compared to the primary screen (Figures 7, 8 and 9), pointing towards a high reproducibility of the high-content screening method.

By analyzing the results of the both screens, the entire miR-30 family (comprising miR-30a, miR-30b, miR-30c, miR-30d, miR-30e) clearly contributed towards an enhanced SEAP production in CHO cells. In all miR-30 members the mature 5p-strands considered to be the guide strand induced the observed cellular phenotypes. However, miR-30c-1-3p, as the only 3p strand among the miR-30 family, was also found to substantially elevate SEAP productivity. Figure 3A shows the respective fold changes in volumetric SEAP yield for all six productivity-improving miR-30 family members in the primary screen. Although the increase mediated by miR-30b-5p was not statistically significant due to high standard deviation of the biological triplicates, the inventors included it into the graph as for its obvious tendency contributing towards a higher volumetric productivity.

In addition, the miR-30 family could be reliably confirmed as potent driver of recombinant protein expression in CHO cells in the validation screen (Figure 3B). By transfection of larger proportions of miRNA mimics (50 nM), compared to transfections in static cultures (15 nM), the increase in SEAP productivity was even more pronounced without an induction of concentration dependent off-target effects. The marked increase in SEAP production was accompanied by decreased cell densities in miR-30 transfected cultures (Figure 3C). However, viability was not negatively affected (Figure 3D) which promotes the assumption that the cells used most of their energetic resources for the substantially enhanced protein production rather than for cell growth and proliferation.

A characteristic feature of a miRNA family is that the mature miRNA strands share a common miRNA 'seed' sequence that are perfectly base paired with their mRNA targets. Besides a common 'seed' composing 7 nucleotides at the 5' end of all miR-30-5ps, they also share the nucleotides at positions 9 to 11 (UCC), and 15 to 17 (ACU), respectively. Considering an overall length of 22-23 nucleotides for miR-30, this finding suggests that this miRNA family share a minimum of 60 % sequence similarity, while miR-30a-5p, miR-30d-5p and miR-30e-5p even share >90 % sequence homology.

To gain insights into multiple effects of respective miRNAs, one cellular parameter can be plotted against another, enabling the identification of highly interesting functional candidate miRNAs for cell engineering. Towards this end, phenotypic changes beneficial for bioprocess performance, such as an increase in protein production and viable cell density, or a decrease in apoptosis were investigated. A detailed analysis of the miR-30 family revealed that the three miRNAs miR-30a-5p, miR-30c-1-3p and miR-30d-5p exhibited combined effects in both increasing volumetric and specific productivity (Figure 3E), and miR-30a-5p and miR-30d-5p both additionally decreased the number of apoptotic cells highlighting their potential as attractive targets for cell engineering (Figure 3F).

To further examine the potential of the miR-30 family to enhance protein production in CHO cells, the inventors selected two miR-30 family members exhibiting various extent of recombinant SEAP production increase (miR-30a-5p and miR-30c-5p) and performed a scale-up experiment by transfecting these miRNAs separately as well as combinations of both miRNAs in elevated culture scale. Similarly, miR-30a-5p and miR-30c-5p substantially increased volumetric and specific SEAP productivity after transient transfection in 2 mL batch cultures (Figure 4A). CHO-SEAP cultures transfected with miR-30a-5p alone showed higher cell densities after 72 h, while introduction of miR-30c-5p resulted in decreased cell density (Figure 4B). However, viability was not negatively affected suggesting that reduced cell densities might be due to a substantial increase in cell-specific SEAP productivity. Co-transfection of both miRNA species in equal concentrations (25 nM each) could reverse the growth-inhibiting effect of miR-30c-5p and resulted in higher SEAP titers as compared to cells transfected with 50 nM of miR-30c-5p mimics. Moreover, by increasing miR-30a/miR-30c concentrations up to 50 nM (100 nM total miRNA concentration), viable cell density was further increased compared to miR-NT transfected control cells, and exhibited values similar to cells transfected with 50 nM miR-30a-5p mimics. This might argue for additive or even synergistic effects of miR-30a and miR-30c, which would have important implications for a combined stable expression of various miRNAs.

### Stable overexpression of miR-30 family members

To confirm that results of transient introduction of miRNA mimics can be interpolated to stable miRNA overexpression, the inventors selected three miR-30 family members and established stable overexpressing cell pools based on the CHO-SEAP parental cell line. For stable long-term expression of target miRNAs the respective precursor sequences have to be integrated into the host cell genome. A correct intranuclear Drosha/DGCR8 processing requires the native genomic sequence context of endogenous pre-miRs including appropriate upstream and downstream flanking regions. The inventors have therefore PCR-amplified the endogenous precursor miRNA sequences of MIR30a, MIR30c and MIR30e, including approximately 100 bp of both up- and downstream flanking regions from genomic DNA, and subcloned them into a mammalian expression vector. The pre-miR sequences were inserted upstream of a green fluorescent protein-puromycin (GFP-Puro) fusion protein under the control of the human elongation factor 1 alpha (EF1α) promoter (Figure 5A). This feature offers two advantages: Firstly, it enables the detection of positively transfected cells via GFP-fluorescence (as well as facilitates fluorescent-activated cell sorting), and secondly, it allows for selection of stably transfected cells by adding antibiotic pressure to the cultures. Moreover, the EF1α promoter induces strong transgene as well as miRNA expression and has been reported to be less prone to epigenetic gene silencing in CHO cells compared to viral promoters such as the human cytomegalovirus (hCMV) immediate early promoter. As a result, long-term miRNA overexpression in recombinant CHO cells is expected to be more stable and efficient as compared to previously described miRNA expression approaches in which only the mature miRNA -5p and -3p strands were integrated into an artificially created chimeric stem-loop.

Stable cell pools overexpressing each member of the miR-30 family were successfully established by puromycin selection and overexpression of mature miRNAs was assessed via qRT-PCR (Figure 5B). Notably, the fold-change value of miRNA overexpression is highly dependent on the endogenous level of the respective mature miRNA. qRT-PCR analysis revealed that miR-30e-5p is highly abundant in CHO cells as compared to miR-30a-5p or miR-30c-5p, which are only moderately expressed, possibly explaining the observed differences in miR-30 overexpression in the stable pools.

Stable MIR30a, MIR30c and MIR30e overexpressing pools were batch-cultivated for 7 days and compared to mock control cells (pEGP-MIR-Null) as well as the parental CHO-SEAP cell line. Analysis of SEAP protein concentration in the supernatant confirmed that CHO-pEGP-MIR30a, CHO-pEGP-MIR30c and CHO-pEGP-MIR30e produced significantly more SEAP as compared to control cells (Figure 5C). To investigate if the observed increase in volumetric productivity was due to an increase in either cell number or specific productivity, the inventors have analyzed the cell density and viability and discovered that MIR30a overexpressing cells reached far higher cell density and viability from day 3 post-seeding as compared to parental CHO-SEAP cells (Figure 5D). The accumulation of metabolic side products as well as a decrease in nutrient supply by depleted culture media is usually in conjunction with decreased proliferation rates as seen by the initiation of the stationary growth phase of negative control (pEGP-MIR-Null) and parental CHO-SEAP cells. The fact that MIR30a overexpressing cells kept growing at higher viabilities until day 6 post seeding, together with the observation that transient introduction of miR-30a-5p decreased apoptosis rate (Figure 3F), points toward an anti-apoptotic function of MIR30a.

In contrast, MIR30c overexpressing pools showed slightly decreased cell concentrations whereas MIR30e overexpression had no significant effect on cell density and viability during batch cultivation. However, cell-specific SEAP productivity was substantially increased by almost two-fold in MIR30c and MIR30e overexpressing cells, respectively (Figure 5E). In this conjunction, the extraordinarily enhanced recombinant protein productivity might be one possible reason for the diminished cell growth of the CHO-pEGP-MIR30c pool as well as for the earlier drop in viability, which might be due to a faster consumption of nutrients in the media. Moreover, the inventors observed that both miR-30c strands (5p and 3p), which are derived from the same pre-miR-30c precursor, enhanced recombinant protein expression in transient screenings (Figures 3A and B). Hence, another possible reason could be that since both strands are more abundant as a result of MIR30c overexpression, mature miR-30c-5p and miR-30c-1-3p act simultaneously leading to stronger phenotypic effects.

Strikingly, although these three miRNAs share the same seed sequence and the residual sequence only varies in a few nucleotides the effects on the cell phenotype is remarkably diverse. This illuminates that the specificity of a given miRNA to its target mRNAs and therefore its biological function is determined by the entire miRNA sequence rather than solely by the seed sequence. Another reason for the diverse function of the miR-30 family could be that since mature sequences are almost identical, it would be conceivable that miRNA precursor sequences play a critical role for miRNA fate and might be involved in determining function of the miRNA. Taken together the results of stable miR-30 overexpression finally proved that effects of transient miRNA mimics transfection experiments can be reproduced in a stable fashion, and more importantly, it highlights again that miRNAs are attractive tools for improving culture performance of biopharmaceutical production cells.

### Mature miR-30 expression levels are upregulated during stationary growth phase

A batch suspension cell culture production process is generally divided into different phases with the stationary phase to be considered as the main production period where cells switch their metabolism from growth to increased protein expression, a feature which is exploited in fed-batch as well as in biphasic production processes. The miR-30 family has previously been demonstrated to be expressed by different CHO strains as well as under various culture conditions. The inventors hypothesized that if the miR-30 family actually contributes to increased protein production in CHO cells, the concentration of mature miR-30 molecules might be more abundant in the stationary phase than during exponential growth. To test this postulate the inventors performed three independent batch cultivations of CHO-SEAP cells, and analyzed expression levels of miR-30a-5p and miR-30c-5p, respectively, during the cultivation process. qRT-PCR analysis revealed that mature miR-30a and miR-30c were strongly upregulated during the stationary phase of a CHO batch culture (Figure 6A). Although expression levels of both miRNAs still remained upregulated in the decline phase which is the last stage mainly driven by apoptotic cell death the miR-30 family may not be involved in apoptosis since at no times after transient (Figure 6B) or stable miR-30 overexpression (Figure 5D), increased apoptosis was observed. It is therefore rather likely that the cells might use miR-30 as endogenous vehicle to control the metabolic shift towards a more effective protein expression. Compared to proteins which have to be tediously translated, correctly folded as well as posttranslationally modified, microRNAs as small RNAs only have to be transcribed and processed to be readily available for gene regulation. This would promote the assumption of recent studies which classified miRNAs as smart endogenous tool to confer rapid transformation in cell phenotype.

To determine whether the observed effects after ectopic miR-30 overexpression could be reversed by knockdown of miR-30 family members, the inventors transiently repressed endogenous miR-30c-5p and subsequently examined the consequences on protein productivity and viable cell density of CHO-SEAP cells. Using antisense inhibitors specific for mature miR-30c-5p, so-called antagomiRs or miRNA-inhibitors, the inventors found that endogenous miR-30c expression was strongly attenuated (Figure 6C). However, neither specific SEAP productivity nor viable cell density was significantly affected by anti-miR-30c-5p antagomiRs, suggesting that lowering endogenous miR-30 levels might not lead to opposing effects regarding protein production (Figure 6D and E).

### References

Ebert MS, Sharp PA; MicroRNA sponges: progress and possibilities; RNA. 2010 Nov;16(11):2043-50
Fischer S, Wagner A, Kos A, Aschrafi A, Handrick R, Hannemann J, Otte K. Breaking limitations of complex culture media: functional non-viral miRNA delivery into pharmaceutical production cell lines. J Biotechnol. 2013 Dec;168(4):589-600.
Krämer O, Klausing S, Noll T; Methods in mammalian cell line engineering: from random mutagenesis to sequence-specific approaches; Appl Microbiol Biotechnol. 2010 Sep;88(2):425-36.
van Rooij E; The art of microRNA research; Circ Res. 2011 Jan 21;108(2):219-34.

## Claims

1. A nucleic acid construct for increasing the yield of a biomolecule produced by a Chinese hamster ovary cell cultured *in vitro,* the nucleic acid construct comprising at least two different regions, wherein the regions are
- a first region encoding for at least one miRNA stimulating cellular production of a biomolecule in a cell, the miRNA is selected from group 1, or
- a second region encoding for at least one miRNA and/or miRNA inhibitor suppressing cell death, the miRNA is selected from group 2 and the miRNA-inhibitor inhibits a miRNA selected from group 3, and
- a third region encoding for at least one miRNA regulating cell proliferation,
wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

2. The nucleic acid construct according to claim 1, wherein the first region encodes for at least one miRNA selected from group 9, and/or the second region encodes for at least one miRNA selected from group 10 and/or an miRNA-inhibitor inhibiting a miRNA selected from group 11,
wherein group 9, consists of SEQ ID NO.: 1, 3, 6, 8, 10, 29, 39, 40, 47, 49, 51, 55, 91, 103, 115, 132, 137, 171, 211 and 294; group 10 consists of SEQ ID NO.: 3, 7, 20, 54, 59, 73, 94, 145, 159, 175, 176, 178, 179, 199, 206, 248, 251, 252, 266 and 272; and group 11 consists of SEQ ID NO.: 297, 305, 307, 311, 312, 313, 321, 330, 331, 335, 336, 340, 345, 351, 359, 405, 412, 458, 510 and 608.

3. The nucleic acid construct according to claim 1 or 2, wherein the third region further encodes for at least one miRNA and/or miRNA-inhibitor regulating cell proliferation, the miRNA is selected from group 4 or 5 and the miRNA-inhibitor inhibits a miRNA selected from group 4 or 5,
wherein group 4 consists of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288, and 291;
and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606,
preferably the third region further encodes for at least one miRNA selected from group 12 or 13 and/or an miRNA-inhibitor inhibiting a miRNA selected from group 12 or 13,
wherein group 12 consists of SEQ ID NO.: 5, 7, 22, 30, 35, 43, 68, 72, 78, 84, 96, 146, 148, 173, 177, 198, 202, 232, 234, 244, 267 and 283; and group 13 consists of SEQ ID NO.: 517, 523, 526, 529, 531, 533, 537, 548, 550, 558, 560, 561, 563, 566, 567, 571, 575, 577, 583, 591, 600, 601 and 604.

4. The nucleic acid construct according to any of the preceding claims, wherein the at least two different regions are controlled by different promoters, preferably at least one promoter is inducible or inhibitable.

5. The nucleic acid construct according to any of the preceding claims, wherein the biomolecule is a biopharmaceutical, preferably a recombinant molecule, more preferred a recombinant protein or a recombinant virus.

6. The nucleic acid construct according to any of the preceding claims, wherein the nucleic acid construct is an expression vector, an episomal vector or a viral vector.

7. A cell comprising a construct according to any of claims 1 to 6, wherein the cell is a Chinese hamster ovary cell.

8. The cell according to claim 7, wherein the construct is integrated into the cell's genome.

9. The cell according to claim 7 or 8, wherein a region of the cell's genome encoding for at least one miRNA selected from group 1, 2, or 4 is amplified and/or a region of the cell's genome encoding for at least one miRNA selected from group 3 or 5 is deleted or silenced.

10. The cell according to any of claims 7 to 9, wherein the cell is stable cell line cell.

11. Method for increasing the yield of a biomolecule produced by a cell cultured *in vitro* comprising at least two steps selected of
- stimulating cellular production of the biomolecule by increasing the level of at least one miRNA selected from group 1 in the cell, or
- reducing cell death by increasing the level of at least one miRNA selected from group 2 in the cell and/or decreasing the level of at least one miRNA selected from group 3, and
- regulating proliferation of the cell by increasing the level of at least one miRNA in the cell, wherein the miRNA is a miRNA of SEQ ID NO.: 160,
wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the cell is a Chinese hamster ovary cell.

12. The method of claim 11, wherein the step of regulating proliferation of the cell further comprises increasing the level of at least one miRNA selected from group 4 or 5 in the cell and/or decreasing the level of at least one miRNA selected from group 4 or 5,
wherein group 4 consists of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288, and 291;
and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606.

13. The method of claim 11 or 12, wherein the level of a miRNA is increased by overexpressing the miRNA in the cell, by electroporating the cell in the presence of the miRNA or by adding the miRNA and a transfectant to a medium, in which the cell is cultured.

14. The method of any of claims 11 to 13, wherein the level of a miRNA is decreased by deleting the region of the cell's genome encoding for the miRNA or regulating its transcription, by expressing a miRNA-inhibitor in the cell directed against the miRNA, by electroporating the cell in the presence of the miRNA-inhibitor or by adding a miRNA-inhibitor and a transfectant to a medium, in which the cell is cultured, wherein the miRNA inhibitor is an antagomir, a miRNA sponge or a miRNA decoy.

15. Use of a combination of a miRNA of SEQ ID NO.: 160 and at least one miRNA selected from group 1, at least one miRNA selected from group 2 and/or at least one miRNA-inhibitor inhibiting a miRNA selected from group 3 for increasing the yield of a biomolecule produced by a Chinese hamster ovary cell cultured *in vitro,* wherein group 1 consists of SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, and 294;
group 2 consists of SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, and 295;
group 3 consists of SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, and 609,
and wherein the miRNA inhibitor is an antagomir, a miRNA sponge or a miRNA decoy.

16. The use of claim 15, wherein the combination further comprises at least one miRNA selected from group 4 or 5 and/or at least one miRNA-inhibitor inhibiting a miRNA selected from group 4 or 5,
wherein group 4 consists of SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288, and 291;
and group 5 consists of SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, and 606.

## Patentansprüche

1. Nukleinsäurekonstrukt zum Erhöhen der Ausbeute eines Biomoleküls, das von einer *in vitro* kultivierten Ovarialzelle des chinesischen Hamsters produziert wird, wobei das Nukleinsäurekonstrukt mindestens zwei verschiedene Bereiche umfasst, wobei die Bereiche
- ein erster Bereich, der für mindestens eine miRNA kodiert, die die zelluläre Produktion eines Biomoleküls in einer Zelle stimuliert, wobei die miRNA aus Gruppe 1 ausgewählt ist, oder
- ein zweiter Bereich, der für mindestens eine miRNA und/oder einen miRNA-Inhibitor kodiert, die/der den Zelltod unterdrückt, wobei die miRNA aus Gruppe 2 ausgewählt ist und der miRNA-Inhibitor eine miRNA, die aus Gruppe 3 ausgewählt ist, inhibiert, und
- ein dritter Bereich, der für mindestens eine miRNA kodiert, die die Zellproliferation reguliert, wobei die miRNA eine miRNA der SEQ ID NO.: 160 ist,
sind,
wobei Gruppe 1 aus SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, und 294 besteht; Gruppe 2 aus SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95, 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, und 295 besteht;
Gruppe 3 aus SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, und 609 besteht,
und wobei der miRNA-Inhibitor ein Antagomir, ein miRNA-Schwamm oder ein miRNA-Köder ist.

2. Nukleinsäurekonstrukt gemäß Anspruch 1, wobei der erste Bereich für mindestens eine miRNA kodiert, die aus Gruppe 9 ausgewählt ist, und/oder der zweite Bereich für mindestens eine miRNA, die aus Gruppe 10 ausgewählt ist, und/oder einen miRNA-Inhibitor, der eine miRNA, die aus Gruppe 11 ausgewählt ist, inhibiert, kodiert,
wobei Gruppe 9 aus SEQ ID NO.: 1, 3, 6, 8, 10, 29, 39, 40, 47, 49, 51, 55, 91, 103, 115, 132, 137, 171, 211 und 294 besteht; Gruppe 10 aus SEQ ID NO.: 3, 7, 20, 54, 59, 73, 94, 145, 159, 175, 176, 178, 179, 199, 206, 248, 251, 252, 266 und 272 besteht; und Gruppe 11 aus SEQ ID NO.: 297, 305, 307, 311, 312, 313, 321, 330, 331, 335, 336, 340, 345, 351, 359, 405, 412, 458, 510 und 608 besteht.

3. Nukleinsäurekonstrukt gemäß Anspruch 1 oder 2, wobei der dritte Bereich weiter für mindestens eine miRNA und/oder einen miRNA-Inhibitor kodiert, die/der die Zellproliferation reguliert, wobei die miRNA aus Gruppe 4 oder 5 ausgewählt ist und der miRNA-Inhibitor eine miRNA, die aus Gruppe 4 oder 5 ausgewählt ist, inhibiert,
wobei Gruppe 4 aus SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288, und 291 besteht;
und Gruppe 5 aus SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, und 606 besteht,
wobei der dritte Bereich vorzugsweise weiter für mindestens eine miRNA, die aus Gruppe 12 oder 13 ausgewählt ist, und/oder einen miRNA-Inhibitor, der eine miRNA, die aus Gruppe 12 oder 13 ausgewählt ist, inhibiert, kodiert, wobei Gruppe 12 aus SEQ ID NO.: 5, 7, 22, 30, 35, 43, 68, 72, 78, 84, 96, 146, 148, 173, 177, 198, 202, 232, 234, 244, 267 und 283 besteht; und Gruppe 13 aus SEQ ID NO.: 517, 523, 526, 529, 531, 533, 537, 548, 550, 558, 560, 561, 563, 566, 567, 571, 575, 577, 583, 591, 600, 601 und 604 besteht.

4. Nukleinsäurekonstrukt gemäß einem der vorhergehenden Ansprüche, wobei die mindestens zwei verschiedenen Bereiche von verschiedenen Promotoren gesteuert werden, wobei vorzugsweise mindestens ein Promotor induzierbar oder inhibierbar ist.

5. Nukleinsäurekonstrukt gemäß einem der vorhergehenden Ansprüche, wobei das Biomolekül ein Biopharmazeutikum, vorzugsweise ein rekombinantes Molekül, weiter bevorzugt ein rekombinantes Protein oder ein rekombinantes Virus ist.

6. Nukleinsäurekonstrukt gemäß einem der vorhergehenden Ansprüche, wobei das Nukleinsäurekonstrukt ein Expressionsvektor, ein episomaler Vektor oder ein viraler Vektor ist.

7. Zelle, die ein Konstrukt gemäß einem der Ansprüche 1 bis 6 umfasst, wobei die Zelle eine Ovarialzelle des chinesischen Hamsters ist.

8. Zelle gemäß Anspruch 7, wobei das Konstrukt in das Genom der Zelle integriert ist.

9. Zelle gemäß Anspruch 7 oder 8, wobei ein Bereich des Genoms der Zelle, der für mindestens eine miRNA kodiert, die aus Gruppe 1, 2 oder 4 ausgewählt ist, amplifiziert ist, und/oder wobei ein Bereich des Genoms der Zelle, der für mindestens eine miRNA kodiert, die aus Gruppe 3 oder 5 ausgewählt ist, entfernt oder stillgelegt ist.

10. Zelle gemäß einem der Ansprüche 7 bis 9, wobei die Zelle eine Zelle einer stabilen Zelllinie ist.

11. Verfahren zum Erhöhen der Ausbeute eines Biomoleküls, das von einer *in vitro* kultivierten Zelle produziert wird, umfassend mindestens zwei Schritte, die ausgewählt sind aus
- Stimulieren der zellulären Produktion des Biomoleküls durch Erhöhen des Niveaus von mindestens einer miRNA, die aus Gruppe 1 ausgewählt ist, in der Zelle, oder
- Reduzieren des Zelltods durch Erhöhen des Niveaus von mindestens einer miRNA, die aus Gruppe 2 ausgewählt ist, in der Zelle und/oder Verringern des Niveaus von mindestens einer miRNA, die aus Gruppe 3 ausgewählt ist, und
- Regulieren der Proliferation der Zelle durch Erhöhen des Niveaus von mindestens einer miRNA in der Zelle, wobei die miRNA eine miRNA der SEQ ID NO.: 160 ist,
wobei Gruppe 1 aus SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285, und 294 besteht;
Gruppe 2 aus SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170,172, 175,176, 178, 179,180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, und 295 besteht;
Gruppe 3 aus SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, und 609 besteht,
und wobei die Zelle eine Ovarialzelle des chinesischen Hamsters ist.

12. Verfahren nach Anspruch 11, wobei der Schritt des Regulierens der Proliferation der Zelle weiter das Erhöhen des Niveaus von mindestens einer miRNA, die aus Gruppe 4 oder 5 ausgewählt ist, in der Zelle und/oder das Verringern des Niveaus von mindestens einer miRNA, die aus Gruppe 4 oder 5 ausgewählt ist, umfasst,
wobei Gruppe 4 aus SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150,162,164, 173,177, 187, 189, 193, 198, 202, 215, 218, 232, 234, 235, 244, 246, 249, 250, 261, 263, 264, 267, 270, 275, 281, 283, 287, 288, und 291 besteht;
und Gruppe 5 aus SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, und 606 besteht.

13. Verfahren nach Anspruch 11 oder 12, wobei das Niveau einer miRNA durch Überexprimieren der miRNA in der Zelle, durch Elektroporieren der Zelle in Gegenwart der miRNA oder durch Zugeben der miRNA und eines Transfektionsmittels zu einem Medium, in dem die Zelle kultiviert wird, erhöht wird.

14. Verfahren nach einem der Ansprüche 11 bis 13, wobei das Niveau einer miRNA durch Entfernen des Bereichs des Genoms der Zelle, der für die miRNA kodiert, oder Regulieren von dessen Transkription, durch Exprimieren eines miRNA-Inhibitors in der Zelle, der gegen die miRNA gerichtet ist, durch Elektroporieren der Zelle in Gegenwart des miRNA-Inhibitors oder durch Zugeben eines miRNA-Inhibitors und eines Transfektionsmittels zu einem Medium, in dem die Zelle kultiviert wird, verringert wird, wobei der miRNA-Inhibitor ein Antagomir, ein miRNA-Schwamm oder ein miRNA-Köder ist.

15. Verwendung einer Kombination aus einer miRNA der SEQ ID NO.: 160 und mindestens einer miRNA, die aus Gruppe 1 ausgewählt ist, mindestens einer miRNA, die aus Gruppe 2 ausgewählt ist, und/oder mindestens eines miRNA-Inhibitors, der eine miRNA, die aus Gruppe 3 ausgewählt ist, inhibiert, zum Erhöhen der Ausbeute eines Biomoleküls, das von einer *in vitro* kultivierten Ovarialzelle des chinesischen Hamsters produziert wird,
wobei Gruppe 1 aus SEQ ID NO.: 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174,182, 183, 185, 186, 188, 190, 201, 203, 207, 211, 212, 214, 217, 222, 223, 228, 230, 233, 238, 239, 240, 254, 255, 269, 276, 278, 279, 285, und 294 besteht;
Gruppe 2 aus SEQ ID NO.: 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293, und 295 besteht;
Gruppe 3 aus SEQ ID NO.: 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608, und 609 besteht,
und wobei der miRNA-Inhibitor ein Antagomir, ein miRNA-Schwamm oder ein miRNA-Köder ist.

16. Verwendung nach Anspruch 15, wobei die Kombination weiter mindestens eine miRNA, die aus Gruppe 4 oder 5 ausgewählt ist, und/oder mindestens einen miRNA-Inhibitor, der eine miRNA, die aus Gruppe 4 oder 5 ausgewählt ist, inhibiert, umfasst,
wobei Gruppe 4 aus SEQ ID NO.: 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135,139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288, und 291 besteht;
und Gruppe 5 aus SEQ ID NO.: 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604, und 606 besteht.

## Revendications

1. Construction d'acide nucléique pour augmenter le rendement d'une biomolécule produite par une cellule d'ovaire de hamster chinois cultivée *in vitro,* la construction d'acide nucléique comprenant au moins deux régions différentes, dans laquelle les régions sont
- une première région codant pour au moins un miARN stimulant la production cellulaire d'une biomolécule dans une cellule, le miARN est sélectionné dans le groupe 1, ou
- une deuxième région codant pour au moins un miARN et/ou un inhibiteur de miARN supprimant la mort cellulaire, le miARN est sélectionné dans le groupe 2 et l'inhibiteur de miARN inhibe un miARN sélectionné dans le groupe 3, et
- une troisième région codant pour au moins un miARN régulant la prolifération cellulaire, dans laquelle le miARN est un miARN de SEQ ID n° : 160,
dans lequel le groupe 1 consiste en
SEQ ID n° : 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285 et 294 ;
le groupe 2 consiste en SEQ ID n° : 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95, 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293 et 295 ;
le groupe 3 consiste en SEQ ID n° : 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608 et 609,
et dans lequel l'inhibiteur de miARN est un antagomir, une éponge de miARN ou un leurre de miARN.

2. Construction d'acide nucléique selon la revendication 1, dans laquelle la première région code pour au moins un miARN sélectionné dans le groupe 9, et/ou la deuxième région code pour au moins un miARN sélectionné dans le groupe 10 et/ou un inhibiteur de miARN inhibant un miARN sélectionné dans le groupe 11,
dans laquelle le groupe 9 consiste en SEQ ID n° : 1, 3, 6, 8, 10, 29, 39, 40, 47, 49, 51, 55, 91, 103, 115, 132, 137, 171, 211 et 294 ; le groupe 10 consiste en SEQ ID n° : 3, 7, 20, 54, 59, 73, 94, 145, 159, 175, 176, 178, 179, 199, 206, 248, 251, 252, 266 et 272 ; et le groupe 11 consiste en SEQ ID n° : 297, 305, 307, 311, 312, 313, 321, 330, 331, 335, 336, 340, 345, 351, 359, 405, 412, 458, 510 et 608.

3. Construction d'acide nucléique selon la revendication 1 ou 2, dans laquelle la troisième région code en outre pour au moins un miARN et/ou un inhibiteur de miARN régulant la prolifération cellulaire, le miARN est sélectionné dans le groupe 4 ou 5 et l'inhibiteur de miARN inhibe un miARN sélectionné dans le groupe 4 ou 5,
dans lequel le groupe 4 consiste en SEQ ID n° : 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288 et 291 ;
et le groupe 5 consiste en SEQ ID n° : 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604 et 606,
de préférence la troisième région code en outre pour au moins un miARN sélectionné dans le groupe 12 ou 13 et/ou un inhibiteur de miARN inhibant un miARN sélectionné dans le groupe 12 ou 13,
dans lequel le groupe 12 consiste en SEQ ID n° : 5, 7, 22, 30, 35, 43, 68, 72, 78, 84, 96, 146, 148, 173, 177, 198, 202, 232, 234, 244, 267 et 283 ; et le groupe 13 consiste en SEQ ID n° : 517, 523, 526, 529, 531, 533, 537, 548, 550, 558, 560, 561, 563, 566, 567, 571, 575, 577, 583, 591, 600, 601 et 604.

4. Construction d'acide nucléique selon l'une des revendications précédentes, dans laquelle les au moins deux régions différentes sont commandées par des promoteurs différents, de préférence au moins un promoteur est inductible ou peut être inhibé.

5. Construction d'acide nucléique selon l'une des revendications précédentes, dans laquelle la biomolécule est un produit biopharmaceutique, de préférence une molécule recombinante, plus préféré une protéine recombinante ou un virus recombinant.

6. Construction d'acide nucléique selon l'une des revendications précédentes, dans laquelle la construction d'acide nucléique est un vecteur d'expression, un vecteur épisomique ou un vecteur viral.

7. Cellule comprenant une construction selon l'une quelconque des revendications 1 à 6, dans laquelle la cellule est une cellule d'ovaire de hamster chinois.

8. Cellule selon la revendication 7, dans laquelle la construction est intégrée dans le génome de la cellule.

9. Cellule selon la revendication 7 ou 8, dans lequel une région du génome de la cellule codant pour au moins un miARN sélectionné dans le groupe 1, 2, ou 4 est amplifiée et/ou une région du génome de la cellule codant pour au moins un miARN sélectionné dans le groupe 3 ou 5 est supprimée ou rendue silencieuse.

10. Cellule selon l'une quelconque des revendications 7 à 9, dans lequel la cellule est une cellule de lignée cellulaire stable.

11. Procédé pour augmenter le rendement d'une biomolécule produite par une cellule cultivée *in vitro* comprenant au moins deux étapes sélectionnées parmi
- la stimulation de la production cellulaire de la biomolécule en augmentant le niveau d'au moins un miARN sélectionné dans le groupe 1 dans la cellule, ou
- la réduction de la mort cellulaire en augmentant le niveau d'au moins un miARN sélectionné dans le groupe 2 dans la cellule et/ou en diminuant le niveau d'au moins un miARN sélectionné dans le groupe 3, et
- la régulation de la prolifération cellulaire en augmentant le niveau d'au moins un miARN dans la cellule, dans lequel le miARN est un miARN de SEQ ID n° : 160,
dans lequel
le groupe 1 consiste en SEQ ID n° : 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285 et 294 ;
le groupe 2 consiste en SEQ ID n° : 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293 et 295 ;
le groupe 3 consiste en SEQ ID n° : 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608 et 609,
et dans lequel la cellule est une cellule d'ovaire de hamster chinois.

12. Procédé selon la revendication 11, dans lequel l'étape de régulation de la prolifération cellulaire comprend en outre l'augmentation du niveau d'au moins un miARN sélectionné dans le groupe 4 ou 5 dans la cellule et/ou la diminution du niveau d'au moins un miARN sélectionné dans le groupe 4 ou 5,
dans lequel le groupe 4 consiste en SEQ ID n° : 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288 et 291 ;
et le groupe 5 consiste en SEQ ID n° : 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604 et 606.

13. Procédé selon la revendication 11 ou 12, dans lequel le niveau d'un miARN est augmenté en surexprimant le miARN dans la cellule, par électroporation de la cellule en présence du miARN ou en ajoutant le miARN et un transfectant à un milieu, dans lequel la cellule est cultivée.

14. Procédé selon l'une quelconque des revendications 11 à 13, dans lequel le niveau d'un miARN est diminué en supprimant la région du génome de la cellule codant pour le miARN ou en régulant sa transcription, en exprimant un inhibiteur de miARN dans la cellule dirigé contre le miARN, par électroporation de la cellule en présence de l'inhibiteur de miARN ou en ajoutant un inhibiteur de miARN et un transfectant à un milieu, dans lequel la cellule est cultivée, dans lequel l'inhibiteur de miARN est un antagomir, une éponge de miARN ou un leurre de miARN.

15. Utilisation d'une combinaison d'un miARN de SEQ ID n°: 160 et d'au moins un miARN sélectionné dans le groupe 1, d'au moins un miARN sélectionné dans le groupe 2 et/ou d'au moins un inhibiteur de miARN inhibant un miARN sélectionné dans le groupe 3 pour augmenter le rendement d'une biomolécule produite par une cellule d'ovaire de hamster chinois cultivée *in vitro,* dans lequel le groupe 1 consiste en SEQ ID n° : 1, 2, 3, 4, 6, 8, 9, 10, 11, 12, 13, 14, 15, 16, 17, 18, 19, 20, 21, 25, 26, 27, 28, 29, 32, 34, 37, 39, 40, 41, 42, 44, 45, 46, 47, 48, 49, 51, 52, 53, 55, 56, 57, 58, 62, 63, 66, 67, 69, 70, 75, 76, 77, 80, 81, 82, 83, 86, 88, 90, 91, 93, 98, 99, 100, 101, 102, 103, 104, 106, 109, 110, 111, 112, 113, 114, 115, 116, 117, 118, 120, 121, 128, 130, 132, 134, 136, 137, 138, 141, 142, 143, 147, 151, 152, 155, 158, 163, 165, 171, 174, 182, 183, 185, 186, 188, 190, 201, 203, 207, 211, 212, 214, 217, 222, 223, 228, 230, 233, 238, 239, 240, 254, 255, 269, 276, 278, 279, 285 et 294 ;
le groupe 2 consiste en SEQ ID n° : 1, 2, 3, 4, 6, 8, 9, 20, 52, 98, 5, 7, 24, 31, 33, 50, 54, 59, 60, 61, 64, 68, 71, 73, 74, 79, 85, 87, 89, 92, 94, 95 97, 145, 153, 154, 156, 159, 161, 166, 167, 168, 169, 170, 172, 175, 176, 178, 179, 180, 181, 184, 191, 192, 194, 195, 196, 197, 199, 200, 204, 205, 206, 208, 209, 210, 213, 216, 219, 220, 221, 224, 225, 226, 227, 229, 231, 236, 237, 241, 242, 243, 245, 247, 248, 251, 252, 253, 256, 257, 258, 259, 260, 262, 265, 266, 268, 271, 272, 273, 274, 277, 280, 282, 284, 289, 293 et 295 ;
le groupe 3 consiste en SEQ ID n° : 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449,450, 451, 452, 453, 454, 455, 456,457, 458,459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 605, 607, 608 et 609,
et dans lequel l'inhibiteur de miARN est un antagomir, une éponge de miARN ou un leurre de miARN.

16. Utilisation selon la revendication 15, dans laquelle la combinaison comprend en outre au moins un miARN sélectionné dans le groupe 4 ou 5 et/ou au moins un inhibiteur de miARN inhibant un miARN sélectionné dans le groupe 4 ou 5,
dans lequel le groupe 4 consiste en SEQ ID n° : 5, 7, 68, 79, 94, 10, 11, 12, 13, 14, 15, 16, 17, 18, 19, 22, 23, 30, 35, 36, 38, 43, 65, 72, 78, 84, 96, 105, 107, 108, 119, 122, 123, 124, 125, 126, 127, 129, 131, 133, 135, 139, 140, 144, 146, 148, 149, 150, 162, 164, 173, 177, 187, 189, 193, 198, 202, 215, 218, 232, 234, 235, 244, 246, 249, 250, 261, 263, 264, 267, 270, 275, 281, 283, 287, 288 et 291 ;
et le groupe 5 consiste en SEQ ID n° : 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 585, 586, 587, 588, 589, 590, 591, 592, 593, 594, 595, 596, 597, 598, 599, 600, 601, 602, 603, 604 et 606.


### SEQUENCE LISTING

<110> Hochschule Biberach
<120> miRNAs enhancing cell productivity
<130> T30058
<160> 622
<170> PatentIn version 3.3
<210> 1
   <211> 22
   <212> RNA
   <213> mouse
<400> 1
   caagcucgug ucuguggguc cg 22
<210> 2
   <211> 21
   <212> RNA
   <213> mouse
<400> 2
   ugcaccaugg uugucugagc a 21
<210> 3
   <211> 22
   <212> RNA
   <213> mouse
<400> 3
   uguaaacauc cucgacugga ag 22
<210> 4
   <211> 22
   <212> RNA
   <213> mouse
<400> 4
   ggagaaugua guguuaccgu ga 22
<210> 5
   <211> 22
   <212> RNA
   <213> mouse
<400> 5
   aaagugcauc cauuuuguuu gu 22
<210> 6
   <211> 22
   <212> RNA
   <213> mouse
<400> 6
   caucuuaccg gacagugcug ga 22
<210> 7
   <211> 22
   <212> RNA
   <213> mouse
<400> 7
   uggaauguaa agaaguaugu au 22
<210> 8
   <211> 22
   <212> RNA
   <213> mouse
<400> 8
   uauagggauu ggagccgugg cg 22
<210> 9
   <211> 22
   <212> RNA
   <213> mouse
<400> 9
   uauucagauu ggugccuguc au 22
<210> 10
   <211> 19
   <212> RNA
   <213> mouse
<400> 10
   cugaaaaugu ugccugaag 19
<210> 11
   <211> 22
   <212> RNA
   <213> mouse
<400> 11
   cacagcuccc aucucagaac aa 22
<210> 12
   <211> 21
   <212> RNA
   <213> mouse
<400> 12
   uauacauaca cacccauaua c 21
<210> 13
   <211> 22
   <212> RNA
   <213> mouse
<400> 13
   gcucugacua gguugcacua cu 22
<210> 14
   <211> 23
   <212> RNA
   <213> mouse
<400> 14
   accuuggcuc uagacugcuu acu 23
<210> 15
   <211> 22
   <212> RNA
   <213> mouse
<400> 15
   agugguucuu gacaguucaa ca 22
<210> 16
   <211> 22
   <212> RNA
   <213> mouse
<400> 16
   caucuuacug ggcagcauug ga 22
<210> 17
   <211> 22
   <212> RNA
   <213> mouse
<400> 17
   uaacacuguc ugguaacgau gu 22
<210> 18
   <211> 24
   <212> RNA
   <213> mouse
<400> 18
   ugcagcuguu aaggauggug gacu 24
<210> 19
   <211> 22
   <212> RNA
   <213> mouse
<400> 19
   cugguacagg ccugggggau ag 22
<210> 20
   <211> 22
   <212> RNA
   <213> mouse
<400> 20
   uguaaacauc cccgacugga ag 22
<210> 21
   <211> 24
   <212> RNA
   <213> mouse
<400> 21
   agggacggga cguggugcag uguu 24
<210> 22
   <211> 21
   <212> RNA
   <213> mouse
<400> 22
   uacucagauu gauaugaguc a 21
<210> 23
   <211> 22
   <212> RNA
   <213> mouse
<400> 23
   uaucuaguug gaugucaaga ca 22
<210> 24
   <211> 22
   <212> RNA
   <213> mouse
<400> 24
   gcaugacacc acacugggua ga 22
<210> 25
   <211> 23
   <212> RNA
   <213> mouse
<400> 25
   ugacuggcac cauucuggau aau 23
<210> 26
   <211> 23
   <212> RNA
   <213> mouse
<400> 26
   ccccucaggc caccagagcc cgg 23
<210> 27
   <211> 23
   <212> RNA
   <213> mouse
<400> 27
   ugagagaugc cauucuaugu aga 23
<210> 28
   <211> 19
   <212> RNA
   <213> mouse
<400> 28
   ugcacugaag gcacacagc 19
<210> 29
   <211> 22
   <212> RNA
   <213> mouse
<400> 29
   uaaggcuccu uccugugcuu gc 22
<210> 30
   <211> 19
   <212> RNA
   <213> mouse
<400> 30
   aguuuucccu ucaagucaa 19
<210> 31
   <211> 23
   <212> RNA
   <213> mouse
<400> 31
   cauauacaua cacacacacg uau 23
<210> 32
   <211> 22
   <212> RNA
   <213> mouse
<400> 32
   auauacauac acacccauau ac 22
<210> 33
   <211> 23
   <212> RNA
   <213> mouse
<400> 33
   acauaacaua cacacacaug uau 23
<210> 34
   <211> 22
   <212> RNA
   <213> mouse
<400> 34
   agcgggcaca gcugugagag cc 22
<210> 35
   <211> 22
   <212> RNA
   <213> mouse
<400> 35
   auacaguugu ucaaccaguu ac 22
<210> 36
   <211> 21
   <212> RNA
   <213> mouse
<400> 36
   uaaccuguug aacaacugaa c 21
<210> 37
   <211> 24
   <212> RNA
   <213> mouse
<400> 37
   uggcagcugg caagucagaa ugca 24
<210> 38
   <211> 23
   <212> RNA
   <213> mouse
<400> 38
   caagggucac ccucugacuc ugu 23
<210> 39
   <211> 21
   <212> RNA
   <213> mouse
<400> 39
   uuggcagaaa gggcagcugu g 21
<210> 40
   <211> 21
   <212> RNA
   <213> mouse
<400> 40
   gcguggggug guggacucag g 21
<210> 41
   <211> 19
   <212> RNA
   <213> mouse
<400> 41
   ucucccaacc cuuuuccca 19
<210> 42
   <211> 20
   <212> RNA
   <213> mouse
<400> 42
   gguccaguga cuaagagcau 20
<210> 43
   <211> 20
   <212> RNA
   <213> mouse
<400> 43
   ccgcgggacc cggggcugug 20
<210> 44
   <211> 19
   <212> RNA
   <213> mouse
<400> 44
   auugauuguu aagcugaaa 19
<210> 45
   <211> 19
   <212> RNA
   <213> mouse
<400> 45
   aagcgcaagg cccugggug 19
<210> 46
   <211> 20
   <212> RNA
   <213> mouse
<400> 46
   uuagaucgau guggugcucc 20
<210> 47
   <211> 22
   <212> RNA
   <213> mouse
<400> 47
   agacccuggu cugcacucua uc 22
<210> 48
   <211> 21
   <212> RNA
   <213> mouse
<400> 48
   caaaccacac ugugguguua g 21
<210> 49
   <211> 22
   <212> RNA
   <213> mouse
<400> 49
   ucaggcucag uccccucccg au 22
<210> 50
   <211> 22
   <212> RNA
   <213> mouse
<400> 50
   uacgugugug ugcaugugca ug 22
<210> 51
   <211> 21
   <212> RNA
   <213> mouse
<400> 51
   cauacacaca cacauacaca c 21
<210> 52
   <211> 22
   <212> RNA
   <213> mouse
<400> 52
   uaccuaauuu guuguccauc au 22
<210> 53
   <211> 22
   <212> RNA
   <213> mouse
<400> 53
   cuccccaccc cuguaccagu ga 22
<210> 54
   <211> 22
   <212> RNA
   <213> mouse
<400> 54
   gaaguuguuc gugguggauu cg 22
<210> 55
   <211> 22
   <212> RNA
   <213> mouse
<400> 55
   gccugcuggg guggaaccug gu 22
<210> 56
   <211> 21
   <212> RNA
   <213> mouse
<400> 56
   agucaggcuc cuggcaggag u 21
<210> 57
   <211> 22
   <212> RNA
   <213> mouse
<400> 57
   gauauaacca cugccagacu ga 22
<210> 58
   <211> 21
   <212> RNA
   <213> mouse
<400> 58
   cggucggccg aucgcucggu c 21
<210> 59
   <211> 22
   <212> RNA
   <213> mouse
<400> 59
   aacacaccca gcuaaccuuu uu 22
<210> 60
   <211> 22
   <212> RNA
   <213> mouse
<400> 60
   aggugguccg uggcgcguuc gc 22
<210> 61
   <211> 21
   <212> RNA
   <213> mouse
<400> 61
   aaacaugaag cgcugcaaca c 21
<210> 62
   <211> 20
   <212> RNA
   <213> mouse
<400> 62
   uccuggcccu gguccugguc 20
<210> 63
   <211> 20
   <212> RNA
   <213> mouse
<400> 63
   cggggcagcu caguacagga 20
<210> 64
   <211> 22
   <212> RNA
   <213> mouse
<400> 64
   uguaaacauc cuugacugga ag 22
<210> 65
   <211> 21
   <212> RNA
   <213> mouse
<400> 65
   ggccaaggau gagaacucua a 21
<210> 66
   <211> 21
   <212> RNA
   <213> mouse
<400> 66
   uguuggggac auuuuuaaag c 21
<210> 67
   <211> 22
   <212> RNA
   <213> mouse
<400> 67
   aggcugggaa uauuucagag au 22
<210> 68
   <211> 23
   <212> RNA
   <213> mouse
<400> 68
   cgcacucugg ucuucccuug cag 23
<210> 69
   <211> 22
   <212> RNA
   <213> mouse
<400> 69
   guuccugcug aacugagcca gu 22
<210> 70
   <211> 23
   <212> RNA
   <213> mouse
<400> 70
   ucagcaccag gauauuguug ggg 23
<210> 71
   <211> 23
   <212> RNA
   <213> mouse
<400> 71
   ucagccacgg cuuaccugga aga 23
<210> 72
   <211> 22
   <212> RNA
   <213> mouse
<400> 72
   aaagugcuuc ccuuuugugu gu 22
<210> 73
   <211> 19
   <212> RNA
   <213> mouse
<400> 73
   ggggccuggc ggcgggcgg 19
<210> 74
   <211> 22
   <212> RNA
   <213> mouse
<400> 74
   agaguugcgu cuggacgucc cg 22
<210> 75
   <211> 22
   <212> RNA
   <213> mouse
<400> 75
   aaacaugguu ccgucaagca cc 22
<210> 76
   <211> 21
   <212> RNA
   <213> mouse
<400> 76
   ucugucauuc uguaggccaa u 21
<210> 77
   <211> 23
   <212> RNA
   <213> mouse
<400> 77
   caaagugcuc auagugcagg uag 23
<210> 78
   <211> 22
   <212> RNA
   <213> mouse
<400> 78
   uggaauguaa ggaagugugu gg 22
<210> 79
   <211> 22
   <212> RNA
   <213> mouse
<400> 79
   uccuucauuc caccggaguc ug 22
<210> 80
   <211> 23
   <212> RNA
   <213> mouse
<400> 80
   uggguacaua aagaaguaug ugc 23
<210> 81
   <211> 22
   <212> RNA
   <213> mouse
<400> 81
   aguccagggc ugagucagcg ga 22
<210> 82
   <211> 22
   <212> RNA
   <213> mouse
<400> 82
   ugggaaaguu cucaggcuuc ug 22
<210> 83
   <211> 22
   <212> RNA
   <213> mouse
<400> 83
   aacuggccca caaagucccg cu 22
<210> 84
   <211> 22
   <212> RNA
   <213> mouse
<400> 84
   ggugcaguua cuguggcugu gg 22
<210> 85
   <211> 22
   <212> RNA
   <213> mouse
<400> 85
   agcgauggcc gaaucugcuu cc 22
<210> 86
   <211> 22
   <212> RNA
   <213> mouse
<400> 86
   aggucaaggu ucacagggga uc 22
<210> 87
   <211> 22
   <212> RNA
   <213> mouse
<400> 87
   cucucugaug gugggugagg ag 22
<210> 88
   <211> 23
   <212> RNA
   <213> mouse
<400> 88
   uuaaugcuaa uugugauagg ggu 23
<210> 89
   <211> 21
   <212> RNA
   <213> mouse
<400> 89
   cuccuaccug uuagcauuaa c 21
<210> 90
   <211> 23
   <212> RNA
   <213> mouse
<400> 90
   ucuggcuccg ugucuucacu ccc 23
<210> 91
   <211> 21
   <212> RNA
   <213> mouse
<400> 91
   ggugcagugc ugcaucucug g 21
<210> 92
   <211> 22
   <212> RNA
   <213> mouse
<400> 92
   caucuuccag ugcaguguug ga 22
<210> 93
   <211> 22
   <212> RNA
   <213> mouse
<400> 93
   acuccauuug uuuugaugau gg 22
<210> 94
   <211> 22
   <212> RNA
   <213> mouse
<400> 94
   aucaucgucu caaaugaguc uu 22
<210> 95
   <211> 22
   <212> RNA
   <213> mouse
<400> 95
   acccgucccg uucguccccg ga 22
<210> 96
   <211> 22
   <212> RNA
   <213> mouse
<400> 96
   ucagcugagg uuccccucug uc 22
<210> 97
   <211> 23
   <212> RNA
   <213> mouse
<400> 97
   acugcagugc cagcacuucu uac 23
<210> 98
   <211> 22
   <212> RNA
   <213> mouse
<400> 98
   cuauacaauc uacugucuuu cc 22
<210> 99
   <211> 22
   <212> RNA
   <213> mouse
<400> 99
   uauaccucag uuuuaucagg ug 22
<210> 100
   <211> 22
   <212> RNA
   <213> mouse
<400> 100
   ucaguaacaa agauucaucc uu 22
<210> 101
   <211> 22
   <212> RNA
   <213> mouse
<400> 101
   caacuagacu gugagcuucu ag 22
<210> 102
   <211> 21
   <212> RNA
   <213> mouse
<400> 102
   cagccucgcu ggcaggcagc u 21
<210> 103
   <211> 22
   <212> RNA
   <213> mouse
<400> 103
   uucagugaug auuagcuucu ga 22
<210> 104
   <211> 22
   <212> RNA
   <213> mouse
<400> 104
   cuguaugccc uaaccgcuca gu 22
<210> 105
   <211> 22
   <212> RNA
   <213> mouse
<400> 105
   auauacauac acacccauau au 22
<210> 106
   <211> 22
   <212> RNA
   <213> mouse
<400> 106
   ugucuugugu gugcauguuc au 22
<210> 107
   <211> 22
   <212> RNA
   <213> mouse
<400> 107
   cauacaagga uaauuucuuu uu 22
<210> 108
   <211> 24
   <212> RNA
   <213> mouse
<400> 108
   uguagaucca uaugccaugg ugug 24
<210> 109
   <211> 19
   <212> RNA
   <213> mouse
<400> 109
   cuggacgcga gcugggccc 19
<210> 110
   <211> 20
   <212> RNA
   <213> mouse
<400> 110
   ggcgccgcuc guggggggcc 20
<210> 111
   <211> 24
   <212> RNA
   <213> mouse
<400> 111
   cugugcuagu gagguggcuc agca 24
<210> 112
   <211> 22
   <212> RNA
   <213> mouse
<400> 112
   gaguauuguu uccacugccu gg 22
<210> 113
   <211> 25
   <212> RNA
   <213> mouse
<400> 113
   ugucauaugu gugaugacac uuucu 25
<210> 114
   <211> 22
   <212> RNA
   <213> mouse
<400> 114
   agucauacac ggcucuccuc uc 22
<210> 115
   <211> 21
   <212> RNA
   <213> mouse
<400> 115
   ucacuccucc ccucccgucu u 21
<210> 116
   <211> 22
   <212> RNA
   <213> mouse
<400> 116
   auauacauac acacaccuau ac 22
<210> 117
   <211> 20
   <212> RNA
   <213> mouse
<400> 117
   gagguuguag uuugugcuuu 20
<210> 118
   <211> 20
   <212> RNA
   <213> mouse
<400> 118
   cccuaaagua gaaaucacua 20
<210> 119
   <211> 22
   <212> RNA
   <213> mouse
<400> 119
   uauacaaggg caagcucucu gu 22
<210> 120
   <211> 22
   <212> RNA
   <213> mouse
<400> 120
   guggauauuc cuucuauguu ua 22
<210> 121
   <211> 22
   <212> RNA
   <213> mouse
<400> 121
   gcgacgagcc ccucgcacaa ac 22
<210> 122
   <211> 20
   <212> RNA
   <213> mouse
<400> 122
   caggucacgu cucugcaguu 20
<210> 123
   <211> 22
   <212> RNA
   <213> mouse
<400> 123
   aauugcacgg uauccaucug ua 22
<210> 124
   <211> 22
   <212> RNA
   <213> mouse
<400> 124
   uucacaaagc ccauacacuu uc 22
<210> 125
   <211> 22
   <212> RNA
   <213> mouse
<400> 125
   gguauaacca aagcccgacu gu 22
<210> 126
   <211> 23
   <212> RNA
   <213> mouse
<400> 126
   gcaaagcaca gggccugcag aga 23
<210> 127
   <211> 21
   <212> RNA
   <213> mouse
<400> 127
   uucugccucc ccugaaggcu c 21
<210> 128
   <211> 22
   <212> RNA
   <213> mouse
<400> 128
   uguaaacauc cuacacucag cu 22
<210> 129
   <211> 22
   <212> RNA
   <213> mouse
<400> 129
   uggugcuacc gucaggggua ga 22
<210> 130
   <211> 22
   <212> RNA
   <213> mouse
<400> 130
   ucccacaggc ccagcucaua gc 22
<210> 131
   <211> 22
   <212> RNA
   <213> mouse
<400> 131
   aaauggugcc cuagugacua ca 22
<210> 132
   <211> 23
   <212> RNA
   <213> mouse
<400> 132
   uaaagugcuu auagugcagg uag 23
<210> 133
   <211> 17
   <212> RNA
   <213> mouse
<400> 133
   ugagguagua guuagaa 17
<210> 134
   <211> 21
   <212> RNA
   <213> mouse
<400> 134
   ucagaugucu ucaucugguu g 21
<210> 135
   <211> 22
   <212> RNA
   <213> mouse
<400> 135
   augcaagggc uggugcgaug gc 22
<210> 136
   <211> 22
   <212> RNA
   <213> mouse
<400> 136
   ccuucuucuu cuuccugaga ca 22
<210> 137
   <211> 22
   <212> RNA
   <213> mouse
<400> 137
   ccgcucguac ucccgggggu cc 22
<210> 138
   <211> 20
   <212> RNA
   <213> mouse
<400> 138
   ccgaucguuc cccuccauac 20
<210> 139
   <211> 22
   <212> RNA
   <213> mouse
<400> 139
   aaaguucuga gacacuccga cu 22
<210> 140
   <211> 22
   <212> RNA
   <213> mouse
<400> 140
   acucuuuccc uguugcacua cu 22
<210> 141
   <211> 22
   <212> RNA
   <213> mouse
<400> 141
   caaaucuuau uugagcaccu gu 22
<210> 142
   <211> 22
   <212> RNA
   <213> mouse
<400> 142
   gaaugaguaa cugcuagauc cu 22
<210> 143
   <211> 25
   <212> RNA
   <213> mouse
<400> 143
   uccgaggcuc cccaccacac ccugc 25
<210> 144
   <211> 22
   <212> RNA
   <213> mouse
<400> 144
   ucccugagac ccuaacuugu ga 22
<210> 145
   <211> 22
   <212> RNA
   <213> mouse
<400> 145
   ucguaccgug aguaauaaug cg 22
<210> 146
   <211> 21
   <212> RNA
   <213> mouse
<400> 146
   uucacagugg cuaaguuccg c 21
<210> 147
   <211> 22
   <212> RNA
   <213> mouse
<400> 147
   cugggagagg guuguuuacu cc 22
<210> 148
   <211> 22
   <212> RNA
   <213> mouse
<400> 148
   aacccguaga uccgaucuug ug 22
<210> 149
   <211> 21
   <212> RNA
   <213> mouse
<400> 149
   caagcucguu ucuauggguc u 21
<210> 150
   <211> 23
   <212> RNA
   <213> mouse
<400> 150
   caaagugcug uucgugcagg uag 23
<210> 151
   <211> 22
   <212> RNA
   <213> mouse
<400> 151
   uauugcacuc gucccggccu cc 22
<210> 152
   <211> 22
   <212> RNA
   <213> mouse
<400> 152
   gcuuauggcu ucaagcuuuc gg 22
<210> 153
   <211> 21
   <212> RNA
   <213> mouse
<400> 153
   aagguuacuu guuaguucag g 21
<210> 154
   <211> 23
   <212> RNA
   <213> mouse
<400> 154
   uauucagauu agugccaguc aug 23
<210> 155
   <211> 19
   <212> RNA
   <213> mouse
<400> 155
   ggugcucaca uguccuccu 19
<210> 156
   <211> 20
   <212> RNA
   <213> mouse
<400> 156
   cggcucuggg ucugugggga 20
<210> 157
   <211> 21
   <212> RNA
   <213> mouse
<400> 157
   cagugcaauu aaaaggggga a 21
<210> 158
   <211> 18
   <212> RNA
   <213> mouse
<400> 158
   aucucgcugg ggccucca 18
<210> 159
   <211> 22
   <212> RNA
   <213> mouse
<400> 159
   cucagacaga gauaccuucu cu 22
<210> 160
   <211> 21
   <212> RNA
   <213> mouse
<400> 160
   cuccuucacc cgggcgguac c 21
<210> 161
   <211> 22
   <212> RNA
   <213> mouse
<400> 161
   gggacccggg gagagaugua ag 22
<210> 162
   <211> 19
   <212> RNA
   <213> mouse
<400> 162
   ggaggcagag gcaggagga 19
<210> 163
   <211> 21
   <212> RNA
   <213> mouse
<400> 163
   ugcccacccu uuaccccgcu c 21
<210> 164
   <211> 21
   <212> RNA
   <213> mouse
<400> 164
   ccguccugag guuguugagc u 21
<210> 165
   <211> 22
   <212> RNA
   <213> mouse
<400> 165
   acuugugugu gcauguauau gu 22
<210> 166
   <211> 22
   <212> RNA
   <213> mouse
<400> 166
   auaguugugu guggaugugu gu 22
<210> 167
   <211> 23
   <212> RNA
   <213> mouse
<400> 167
   auugugucaa uaugcgauga ugu 23
<210> 168
   <211> 22
   <212> RNA
   <213> mouse
<400> 168
   gagcuuauuc auaaaagugc ag 22
<210> 169
   <211> 21
   <212> RNA
   <213> mouse
<400> 169
   uaauuuuaug uauaagcuag u 21
<210> 170
   <211> 23
   <212> RNA
   <213> mouse
<400> 170
   ugagugugug ugugugagug ugu 23
<210> 171
   <211> 20
   <212> RNA
   <213> mouse
<400> 171
   auguauaaau guauacacac 20
<210> 172
   <211> 22
   <212> RNA
   <213> mouse
<400> 172
   gccccaucga uuaacugcuu cc 22
<210> 173
   <211> 22
   <212> RNA
   <213> mouse
<400> 173
   uucaccacac ccagcuuaaa ga 22
<210> 174
   <211> 23
   <212> RNA
   <213> mouse
<400> 174
   aggagguccu ggggccgccc uga 23
<210> 175
   <211> 23
   <212> RNA
   <213> mouse
<400> 175
   gaaaucaagc uugggugaga ccu 23
<210> 176
   <211> 21
   <212> RNA
   <213> mouse
<400> 176
   gcgacccaua cuugguuuca g 21
<210> 177
   <211> 21
   <212> RNA
   <213> mouse
<400> 177
   ucuuguuaaa aagcagaguc u 21
<210> 178
   <211> 22
   <212> RNA
   <213> mouse
<400> 178
   aaacauucgc ggugcacuuc uu 22
<210> 179
   <211> 22
   <212> RNA
   <213> mouse
<400> 179
   cucggggauc aucaugucac ga 22
<210> 180
   <211> 20
   <212> RNA
   <213> mouse
<400> 180
   auaugcgagg gaacuacugg 20
<210> 181
   <211> 23
   <212> RNA
   <213> mouse
<400> 181
   guuagugaug aucaauaaag uua 23
<210> 182
   <211> 22
   <212> RNA
   <213> mouse
<400> 182
   acuggagacg gaagcugcaa ga 22
<210> 183
   <211> 23
   <212> RNA
   <213> mouse
<400> 183
   aggucuguag cucaguuggc aga 23
<210> 184
   <211> 21
   <212> RNA
   <213> mouse
<400> 184
   ucgaauccca gcggugccuc u 21
<210> 185
   <211> 24
   <212> RNA
   <213> mouse
<400> 185
   guucaugucc cuguucaggc gcca 24
<210> 186
   <211> 24
   <212> RNA
   <213> mouse
<400> 186
   agcucccgcc acugugaccc ccuu 24
<210> 187
   <211> 22
   <212> RNA
   <213> mouse
<400> 187
   cuuaugcaag auucccuucu ac 22
<210> 188
   <211> 21
   <212> RNA
   <213> mouse
<400> 188
   cccagauaau agcacucuca a 21
<210> 189
   <211> 21
   <212> RNA
   <213> mouse
<400> 189
   uugaaaggcu guuucuuggu c 21
<210> 190
   <211> 22
   <212> RNA
   <213> mouse
<400> 190
   uaagugcgcg cauguauaug cg 22
<210> 191
   <211> 24
   <212> RNA
   <213> mouse
<400> 191
   agguugccuc auagugagcu ugca 24
<210> 192
   <211> 22
   <212> RNA
   <213> mouse
<400> 192
   uguuugcaga ggaaacugag ac 22
<210> 193
   <211> 21
   <212> RNA
   <213> mouse
<400> 193
   ugucuugcag gccgucaugc a 21
<210> 194
   <211> 22
   <212> RNA
   <213> mouse
<400> 194
   uaauacuguc ugguaaugcc gu 22
<210> 195
   <211> 23
   <212> RNA
   <213> mouse
<400> 195
   ugaggggcag agagcgagac uuu 23
<210> 196
   <211> 23
   <212> RNA
   <213> mouse
<400> 196
   aucaacagac auuaauuggg cgc 23
<210> 197
   <211> 22
   <212> RNA
   <213> mouse
<400> 197
   uauguaacau gguccacuaa cu 22
<210> 198
   <211> 22
   <212> RNA
   <213> mouse
<400> 198
   guggauauuc cuucuauggu ua 22
<210> 199
   <211> 22
   <212> RNA
   <213> mouse
<400> 199
   aauugcacuu uagcaauggu ga 22
<210> 200
   <211> 23
   <212> RNA
   <213> mouse
<400> 200
   agggacuuuc aggggcagcu gug 23
<210> 201
   <211> 21
   <212> RNA
   <213> mouse
<400> 201
   ggucaagagg cgccugggaa c 21
<210> 202
   <211> 21
   <212> RNA
   <213> mouse
<400> 202
   ucacuuugua gaccaggcug g 21
<210> 203
   <211> 24
   <212> RNA
   <213> mouse
<400> 203
   agucaggcug cuggcuauac acca 24
<210> 204
   <211> 22
   <212> RNA
   <213> mouse
<400> 204
   aggggugcua ucugugauug ag 22
<210> 205
   <211> 22
   <212> RNA
   <213> mouse
<400> 205
   uccgucucag uuacuuuaua gc 22
<210> 206
   <211> 22
   <212> RNA
   <213> mouse
<400> 206
   caauguuucc acagugcauc ac 22
<210> 207
   <211> 22
   <212> RNA
   <213> mouse
<400> 207
   ucucugggcc ugugucuuag gc 22
<210> 208
   <211> 22
   <212> RNA
   <213> mouse
<400> 208
   aggcaagaug cuggcauagc ug 22
<210> 209
   <211> 22
   <212> RNA
   <213> mouse
<400> 209
   gcacuccauc ggaggcagac ac 22
<210> 210
   <211> 21
   <212> RNA
   <213> mouse
<400> 210
   uagggccauc ucauccagau a 21
<210> 211
   <211> 23
   <212> RNA
   <213> mouse
<400> 211
   acgcucugcu uugcuccccc aga 23
<210> 212
   <211> 21
   <212> RNA
   <213> mouse
<400> 212
   cucuacuccc ugccccagcc a 21
<210> 213
   <211> 23
   <212> RNA
   <213> mouse
<400> 213
   uugggaacgg ggugucuuug gga 23
<210> 214
   <211> 21
   <212> RNA
   <213> mouse
<400> 214
   cugugacaca cccgcuccca g 21
<210> 215
   <211> 22
   <212> RNA
   <213> mouse
<400> 215
   aagcuuucuc aucugugaca cu 22
<210> 216
   <211> 21
   <212> RNA
   <213> mouse
<400> 216
   ccuuuaaauu guguccucaa g 21
<210> 217
   <211> 22
   <212> RNA
   <213> mouse
<400> 217
   uguggacacc gugggagguu gg 22
<210> 218
   <211> 22
   <212> RNA
   <213> mouse
<400> 218
   ugaguucagg gacagcgugu cu 22
<210> 219
   <211> 23
   <212> RNA
   <213> mouse
<400> 219
   uucaugagca gcugcaaagg ugu 23
<210> 220
   <211> 23
   <212> RNA
   <213> mouse
<400> 220
   gacuggagcu uggagcggug agc 23
<210> 221
   <211> 24
   <212> RNA
   <213> mouse
<400> 221
   cacaggggaa gcucagugcc agcc 24
<210> 222
   <211> 21
   <212> RNA
   <213> mouse
<400> 222
   auccuuggcc uuccuaggug u 21
<210> 223
   <211> 22
   <212> RNA
   <213> mouse
<400> 223
   cuggcgccaa cugugaccac ug 22
<210> 224
   <211> 22
   <212> RNA
   <213> mouse
<400> 224
   agggaccccg agggagggca gg 22
<210> 225
   <211> 22
   <212> RNA
   <213> mouse
<400> 225
   aucaucaaaa caaauggagu cc 22
<210> 226
   <211> 23
   <212> RNA
   <213> mouse
<400> 226
   aguucucagg cccgcugugg ugu 23
<210> 227
   <211> 22
   <212> RNA
   <213> mouse
<400> 227
   cacuuaauag accgcaaccu gc 22
<210> 228
   <211> 22
   <212> RNA
   <213> mouse
<400> 228
   ucaacaaaau cacugaugcu gg 22
<210> 229
   <211> 19
   <212> RNA
   <213> mouse
<400> 229
   cggggacaca cuuucuccu 19
<210> 230
   <211> 22
   <212> RNA
   <213> mouse
<400> 230
   cagugggccg ugaaagguag cc 22
<210> 231
   <211> 22
   <212> RNA
   <213> mouse
<400> 231
   uuuccucucu gccccauagg gu 22
<210> 232
   <211> 23
   <212> RNA
   <213> mouse
<400> 232
   uaagugcuuc cauguuuuag uag 23
<210> 233
   <211> 22
   <212> RNA
   <213> mouse
<400> 233
   acuuaaacgu gguuguacuu gc 22
<210> 234
   <211> 23
   <212> RNA
   <213> mouse
<400> 234
   uaagugcuuc cauguuuugg uga 23
<210> 235
   <211> 22
   <212> RNA
   <213> mouse
<400> 235
   gcucugacuu uauugcacua cu 22
<210> 236
   <211> 22
   <212> RNA
   <213> mouse
<400> 236
   uugaagagag guuauccuuu gu 22
<210> 237
   <211> 22
   <212> RNA
   <213> mouse
<400> 237
   gcugguuuca uauggugguu ua 22
<210> 238
   <211> 22
   <212> RNA
   <213> mouse
<400> 238
   uagcaccauc ugaaaucggu ua 22
<210> 239
   <211> 22
   <212> RNA
   <213> mouse
<400> 239
   uaugugggac gguaaaccgc uu 22
<210> 240
   <211> 22
   <212> RNA
   <213> mouse
<400> 240
   acucaaaaug gaggcccuau cu 22
<210> 241
   <211> 22
   <212> RNA
   <213> mouse
<400> 241
   acucaaacug ugugacauuu ug 22
<210> 242
   <211> 22
   <212> RNA
   <213> mouse
<400> 242
   agugccgcag aguuuguagu gu 22
<210> 243
   <211> 22
   <212> RNA
   <213> mouse
<400> 243
   gggguuccug gggaugggau uu 22
<210> 244
   <211> 21
   <212> RNA
   <213> mouse
<400> 244
   uaagucacua gugguuccgu u 21
<210> 245
   <211> 22
   <212> RNA
   <213> mouse
<400> 245
   ugucaguuug ucaaauaccc ca 22
<210> 246
   <211> 21
   <212> RNA
   <213> mouse
<400> 246
   ugauugucca aacgcaauuc u 21
<210> 247
   <211> 23
   <212> RNA
   <213> mouse
<400> 247
   caucaguucc uaaugcauug ccu 23
<210> 248
   <211> 21
   <212> RNA
   <213> mouse
<400> 248
   gcaaggacag caaagggggg c 21
<210> 249
   <211> 22
   <212> RNA
   <213> mouse
<400> 249
   aagcuuuuug cucgcguuau gu 22
<210> 250
   <211> 22
   <212> RNA
   <213> mouse
<400> 250
   auaagacgag caaaaagcuu gu 22
<210> 251
   <211> 22
   <212> RNA
   <213> mouse
<400> 251
   cgucuuaccc agcaguguuu gg 22
<210> 252
   <211> 23
   <212> RNA
   <213> mouse
<400> 252
   ugugcaaauc uaugcaaaac uga 23
<210> 253
   <211> 22
   <212> RNA
   <213> mouse
<400> 253
   ucggcaacaa gaaacugccu ga 22
<210> 254
   <211> 25
   <212> RNA
   <213> mouse
<400> 254
   aagggagcug gcucaggaga gaguc 25
<210> 255
   <211> 23
   <212> RNA
   <213> mouse
<400> 255
   ugggacgaga ucaugaggcc uuc 23
<210> 256
   <211> 17
   <212> RNA
   <213> mouse
<400> 256
   ucuccacccu ccuucug 17
<210> 257
   <211> 22
   <212> RNA
   <213> mouse
<400> 257
   ccaguggagc ugcuguuacu uc 22
<210> 258
   <211> 22
   <212> RNA
   <213> mouse
<400> 258
   cgggguuuug agggcgagau ga 22
<210> 259
   <211> 22
   <212> RNA
   <213> mouse
<400> 259
   aggcagaggc uggcggaucu cu 22
<210> 260
   <211> 23
   <212> RNA
   <213> mouse
<400> 260
   ucuggucccc ugcuucgucc ucu 23
<210> 261
   <211> 22
   <212> RNA
   <213> mouse
<400> 261
   ccaggaccau cagugugacu au 22
<210> 262
   <211> 22
   <212> RNA
   <213> mouse
<400> 262
   agaggugcag uaggcaugac uu 22
<210> 263
   <211> 23
   <212> RNA
   <213> mouse
<400> 263
   uaaggugcau cuagugcaga uag 23
<210> 264
   <211> 22
   <212> RNA
   <213> mouse
<400> 264
   cucuccccua ccaccugccu cu 22
<210> 265
   <211> 21
   <212> RNA
   <213> mouse
<400> 265
   cucccacaug caggguuugc a 21
<210> 266
   <211> 21
   <212> RNA
   <213> mouse
<400> 266
   gugguucuag acuugccaac u 21
<210> 267
   <211> 22
   <212> RNA
   <213> mouse
<400> 267
   ucagugcacu acagaacuuu gu 22
<210> 268
   <211> 20
   <212> RNA
   <213> mouse
<400> 268
   cugugggcca ccuagucacc 20
<210> 269
   <211> 22
   <212> RNA
   <213> mouse
<400> 269
   aaccguggcu uucgauuguu ac 22
<210> 270
   <211> 22
   <212> RNA
   <213> mouse
<400> 270
   uaacagucua cagccauggu cg 22
<210> 271
   <211> 23
   <212> RNA
   <213> mouse
<400> 271
   gggggccgau gcacuguaag aga 23
<210> 272
   <211> 21
   <212> RNA
   <213> mouse
<400> 272
   cggggccgua gcacugucug a 21
<210> 273
   <211> 22
   <212> RNA
   <213> mouse
<400> 273
   cugaagcuca gagggcucug au 22
<210> 274
   <211> 22
   <212> RNA
   <213> mouse
<400> 274
   ucggauccgu cugagcuugg cu 22
<210> 275
   <211> 21
   <212> RNA
   <213> mouse
<400> 275
   cgcuuugcuc agccagugua g 21
<210> 276
   <211> 23
   <212> RNA
   <213> mouse
<400> 276
   ucugggcaga gcugcaggag aga 23
<210> 277
   <211> 21
   <212> RNA
   <213> mouse
<400> 277
   cgguugacca ugguguguac g 21
<210> 278
   <211> 20
   <212> RNA
   <213> mouse
<400> 278
   aaaucuaccu gccucugccu 20
<210> 279
   <211> 20
   <212> RNA
   <213> mouse
<400> 279
   ugguagaccg gugacguaca 20
<210> 280
   <211> 21
   <212> RNA
   <213> mouse
<400> 280
   cagucuuacu auguagcccu a 21
<210> 281
   <211> 23
   <212> RNA
   <213> mouse
<400> 281
   agcuucuuua cagugcugcc uug 23
<210> 282
   <211> 22
   <212> RNA
   <213> mouse
<400> 282
   ucaguuauca cagugcugau gc 22
<210> 283
   <211> 22
   <212> RNA
   <213> mouse
<400> 283
   acaagcuugu gucuauaggu au 22
<210> 284
   <211> 22
   <212> RNA
   <213> mouse
<400> 284
   acuguacagg ccacugccuu gc 22
<210> 285
   <211> 22
   <212> RNA
   <213> mouse
<400> 285
   ugagguagga gguuguauag uu 22
<210> 286
   <211> 23
   <212> RNA
   <213> mouse
<400> 286
   agcagcauug uacagggcua uca 23
<210> 287
   <211> 23
   <212> RNA
   <213> mouse
<400> 287
   agcuucuuua caguguugcc uug 23
<210> 288
   <211> 22
   <212> RNA
   <213> mouse
<400> 288
   caaauucgua ucuaggggaa ua 22
<210> 289
   <211> 23
   <212> RNA
   <213> mouse
<400> 289
   uacccuguag aaccgaauuu gug 23
<210> 290
   <211> 22
   <212> RNA
   <213> mouse
<400> 290
   accaucgacc guugauugua cc 22
<210> 291
   <211> 23
   <212> RNA
   <213> mouse
<400> 291
   caacggaauc ccaaaagcag cug 23
<210> 292
   <211> 24
   <212> RNA
   <213> mouse
<400> 292
   aguuuugcag auuugcaguu cage 24
<210> 293
   <211> 23
   <212> RNA
   <213> mouse
<400> 293
   agcuacauug ucugcugggu uuc 23
<210> 294
   <211> 23
   <212> RNA
   <213> mouse
<400> 294
   uguaaacauc cuacacucuc agc 23
<210> 295
   <211> 22
   <212> RNA
   <213> mouse
<400> 295
   aggcaggggc ugggccugca gc 22
<210> 296
   <211> 23
   <212> RNA
   <213> mouse
<400> 296
   ucuuugguua ucuagcugua uga 23
<210> 297
   <211> 22
   <212> RNA
   <213> mouse
<400> 297
   uuuggucccc uucaaccagc ug 22
<210> 298
   <211> 22
   <212> RNA
   <213> mouse
<400> 298
   ugugacuggu ugaccagagg gg 22
<210> 299
   <211> 23
   <212> RNA
   <213> mouse
<400> 299
   uauggcuuuu uauuccuaug uga 23
<210> 300
   <211> 23
   <212> RNA
   <213> mouse
<400> 300
   uuauugcuua agaauacgcg uag 23
<210> 301
   <211> 22
   <212> RNA
   <213> mouse
<400> 301
   uagguuaucc guguugccuu cg 22
<210> 302
   <211> 22
   <212> RNA
   <213> mouse
<400> 302
   uauggcacug guagaauuca cu 22
<210> 303
   <211> 22
   <212> RNA
   <213> mouse
<400> 303
   uggagagaaa ggcaguuccu ga 22
<210> 304
   <211> 22
   <212> RNA
   <213> mouse
<400> 304
   cuauacgacc ugcugccuuu cu 22
<210> 305
   <211> 22
   <212> RNA
   <213> mouse
<400> 305
   uagcaccauu ugaaaucggu ua 22
<210> 306
   <211> 21
   <212> RNA
   <213> mouse
<400> 306
   uucagcuccu auaugaugcc u 21
<210> 307
   <211> 22
   <212> RNA
   <213> mouse
<400> 307
   aaggagcuca cagucuauug ag 22
<210> 308
   <211> 21
   <212> RNA
   <213> mouse
<400> 308
   uugugcuuga ucuaaccaug u 21
<210> 309
   <211> 21
   <212> RNA
   <213> mouse
<400> 309
   gugcauugua guugcauugc a 21
<210> 310
   <211> 22
   <212> RNA
   <213> mouse
<400> 310
   cuccugacuc cagguccugu gu 22
<210> 311
   <211> 21
   <212> RNA
   <213> mouse
<400> 311
   aauauaacac agauggccug u 21
<210> 312
   <211> 20
   <212> RNA
   <213> mouse
<400> 312
   aggucagagg ucgauccugg 20
<210> 313
   <211> 22
   <212> RNA
   <213> mouse
<400> 313
   aaaggcuagg cucacaacca aa 22
<210> 314
   <211> 21
   <212> RNA
   <213> mouse
<400> 314
   gcugguaaaa uggaaccaaa u 21
<210> 315
   <211> 22
   <212> RNA
   <213> mouse
<400> 315
   cucacagcuc ugguccuugg ag 22
<210> 316
   <211> 22
   <212> RNA
   <213> mouse
<400> 316
   ugcggggcua gggcuaacag ca 22
<210> 317
   <211> 22
   <212> RNA
   <213> mouse
<400> 317
   gugaauuacc gaagggccau aa 22
<210> 318
   <211> 22
   <212> RNA
   <213> mouse
<400> 318
   acugauuucu uuugguguuc ag 22
<210> 319
   <211> 22
   <212> RNA
   <213> mouse
<400> 319
   aacaauaucc uggugcugag ug 22
<210> 320
   <211> 22
   <212> RNA
   <213> mouse
<400> 320
   uaugugugug uacauguaca ua 22
<210> 321
   <211> 22
   <212> RNA
   <213> mouse
<400> 321
   aggagagagu uagcgcauua gu 22
<210> 322
   <211> 22
   <212> RNA
   <213> mouse
<400> 322
   gaugugugug uacauguaca ua 22
<210> 323
   <211> 21
   <212> RNA
   <213> mouse
<400> 323
   auacagacac augcacacac a 21
<210> 324
   <211> 23
   <212> RNA
   <213> mouse
<400> 324
   ugugugcaug ugcaugugug uaa 23
<210> 325
   <211> 21
   <212> RNA
   <213> mouse
<400> 325
   uauacauaca cacacauaua u 21
<210> 326
   <211> 22
   <212> RNA
   <213> mouse
<400> 326
   ugcagcagcc ugaggcaggg cu 22
<210> 327
   <211> 22
   <212> RNA
   <213> mouse
<400> 327
   guucugcucc ucuggaggga gg 22
<210> 328
   <211> 23
   <212> RNA
   <213> mouse
<400> 328
   aagggaggau cugggcaccu gga 23
<210> 329
   <211> 22
   <212> RNA
   <213> mouse
<400> 329
   agaggcuggc acugggacac au 22
<210> 330
   <211> 22
   <212> RNA
   <213> mouse
<400> 330
   aagguagaua gaacaggucu ug 22
<210> 331
   <211> 22
   <212> RNA
   <213> mouse
<400> 331
   ucuggcuguu guggugugca aa 22
<210> 332
   <211> 22
   <212> RNA
   <213> mouse
<400> 332
   ggugaauugc aguacuccaa ca 22
<210> 333
   <211> 22
   <212> RNA
   <213> mouse
<400> 333
   uugaugucca cugugaccau ag 22
<210> 334
   <211> 21
   <212> RNA
   <213> mouse
<400> 334
   caugggucug guugggcccg c 21
<210> 335
   <211> 22
   <212> RNA
   <213> mouse
<400> 335
   uccuaacagc aggaguagga gc 22
<210> 336
   <211> 23
   <212> RNA
   <213> mouse
<400> 336
   agucaggcug cuggcuagag uac 23
<210> 337
   <211> 23
   <212> RNA
   <213> mouse
<400> 337
   gagcacccca uuggcuaccc aca 23
<210> 338
   <211> 25
   <212> RNA
   <213> mouse
<400> 338
   uagggggcag gagccggagc ccucu 25
<210> 339
   <211> 21
   <212> RNA
   <213> mouse
<400> 339
   caguuugucu cuucuuuguc u 21
<210> 340
   <211> 22
   <212> RNA
   <213> mouse
<400> 340
   cugccaauuc cauaggucac ag 22
<210> 341
   <211> 23
   <212> RNA
   <213> mouse
<400> 341
   ggcuucuuua cagugcugcc uug 23
<210> 342
   <211> 21
   <212> RNA
   <213> mouse
<400> 342
   uguagggaug gaagccauga a 21
<210> 343
   <211> 22
   <212> RNA
   <213> mouse
<400> 343
   ucagucucau cugcaaagag gu 22
<210> 344
   <211> 24
   <212> RNA
   <213> mouse
<400> 344
   aguugugugu gcaugugcau gugu 24
<210> 345
   <211> 21
   <212> RNA
   <213> mouse
<400> 345
   auaugaguau ucugccuaaa u 21
<210> 346
   <211> 22
   <212> RNA
   <213> mouse
<400> 346
   agcuggagca caaaagccgg ug 22
<210> 347
   <211> 20
   <212> RNA
   <213> mouse
<400> 347
   aaaggauuua ccugaggcca 20
<210> 348
   <211> 21
   <212> RNA
   <213> mouse
<400> 348
   cgaaucccac uccagacacc a 21
<210> 349
   <211> 22
   <212> RNA
   <213> mouse
<400> 349
   uuuguuuguu uugcugaugc ag 22
<210> 350
   <211> 22
   <212> RNA
   <213> mouse
<400> 350
   auacgcacac uuaagacuuu ag 22
<210> 351
   <211> 23
   <212> RNA
   <213> mouse
<400> 351
   agccccugac cuugaaccug gga 23
<210> 352
   <211> 23
   <212> RNA
   <213> mouse
<400> 352
   ugugugcaug ugcaugugug uau 23
<210> 353
   <211> 22
   <212> RNA
   <213> mouse
<400> 353
   agagaaaccc ugucucaaaa aa 22
<210> 354
   <211> 22
   <212> RNA
   <213> mouse
<400> 354
   ugagguagua guuugugcug uu 22
<210> 355
   <211> 21
   <212> RNA
   <213> mouse
<400> 355
   uacaguacug ugauaacuga a 21
<210> 356
   <211> 24
   <212> RNA
   <213> mouse
<400> 356
   ucccugagac ccuuuaaccu guga 24
<210> 357
   <211> 21
   <212> RNA
   <213> mouse
<400> 357
   ucagugcaug acagaacuug g 21
<210> 358
   <211> 22
   <212> RNA
   <213> mouse
<400> 358
   uacucaguaa ggcauuguuc uu 22
<210> 359
   <211> 22
   <212> RNA
   <213> mouse
<400> 359
   agagguauag cgcaugggaa ga 22
<210> 360
   <211> 22
   <212> RNA
   <213> mouse
<400> 360
   acucaaacua ugggggcacu uu 22
<210> 361
   <211> 23
   <212> RNA
   <213> mouse
<400> 361
   aggcagugua guuagcugau ugc 23
<210> 362
   <211> 22
   <212> RNA
   <213> mouse
<400> 362
   ugagguagua gguugugugg uu 22
<210> 363
   <211> 24
   <212> RNA
   <213> mouse
<400> 363
   ucccugagga gcccuuugag ccug 24
<210> 364
   <211> 23
   <212> RNA
   <213> mouse
<400> 364
   uauggcuuuu cauuccuaug uga 23
<210> 365
   <211> 22
   <212> RNA
   <213> mouse
<400> 365
   aacauucaac cugucgguga gu 22
<210> 366
   <211> 23
   <212> RNA
   <213> mouse
<400> 366
   uacugcauca ggaacugacu gga 23
<210> 367
   <211> 22
   <212> RNA
   <213> mouse
<400> 367
   uauguaguau gguccacauc uu 22
<210> 368
   <211> 21
   <212> RNA
   <213> mouse
<400> 368
   augaccuaug auuugacaga c 21
<210> 369
   <211> 22
   <212> RNA
   <213> mouse
<400> 369
   uugcauaugu aggauguccc au 22
<210> 370
   <211> 22
   <212> RNA
   <213> mouse
<400> 370
   uggcagugua uuguuagcug gu 22
<210> 371
   <211> 21
   <212> RNA
   <213> mouse
<400> 371
   cuugguacau cuuugaguga g 21
<210> 372
   <211> 22
   <212> RNA
   <213> mouse
<400> 372
   ugaaacauac acgggaaacc uc 22
<210> 373
   <211> 22
   <212> RNA
   <213> mouse
<400> 373
   gcuuuaacau gggguuaccu gc 22
<210> 374
   <211> 22
   <212> RNA
   <213> mouse
<400> 374
   aagugcuucc auguuucagu gg 22
<210> 375
   <211> 22
   <212> RNA
   <213> mouse
<400> 375
   ggacugugag gugacucuug gu 22
<210> 376
   <211> 21
   <212> RNA
   <213> mouse
<400> 376
   ccugcuguaa gcuguguccu c 21
<210> 377
   <211> 22
   <212> RNA
   <213> mouse
<400> 377
   auugcuuccc agacggugaa ga 22
<210> 378
   <211> 22
   <212> RNA
   <213> mouse
<400> 378
   ugagaacuga auuccauagg cu 22
<210> 379
   <211> 22
   <212> RNA
   <213> mouse
<400> 379
   auauacauac acacaccaac ac 22
<210> 380
   <211> 22
   <212> RNA
   <213> mouse
<400> 380
   uaugugccuu uggacuacau cg 22
<210> 381
   <211> 19
   <212> RNA
   <213> mouse
<400> 381
   cauucucguu uccuucccu 19
<210> 382
   <211> 22
   <212> RNA
   <213> mouse
<400> 382
   agagaaaccc ugucucaaaa aa 22
<210> 383
   <211> 21
   <212> RNA
   <213> mouse
<400> 383
   cagucaugcc gcuugccuac g 21
<210> 384
   <211> 21
   <212> RNA
   <213> mouse
<400> 384
   cgacgagggc cggucggucg c 21
<210> 385
   <211> 22
   <212> RNA
   <213> mouse
<400> 385
   aaugcacccg ggcaaggauu ug 22
<210> 386
   <211> 22
   <212> RNA
   <213> mouse
<400> 386
   auugggaaca uuuugcaugc au 22
<210> 387
   <211> 22
   <212> RNA
   <213> mouse
<400> 387
   cgucaacacu ugcugguuuu cu 22
<210> 388
   <211> 21
   <212> RNA
   <213> mouse
<400> 388
   cuuccgcccg gccggguguc g 21
<210> 389
   <211> 22
   <212> RNA
   <213> mouse
<400> 389
   uggugcggaa agggcccaca gu 22
<210> 390
   <211> 22
   <212> RNA
   <213> mouse
<400> 390
   gguuguauua ucauuguccg ag 22
<210> 391
   <211> 20
   <212> RNA
   <213> mouse
<400> 391
   accaggaggc ugaggucccu 20
<210> 392
   <211> 19
   <212> RNA
   <213> mouse
<400> 392
   uuugugaccu gguccacua 19
<210> 393
   <211> 22
   <212> RNA
   <213> mouse
<400> 393
   ccagcuggga agaaccagug gc 22
<210> 394
   <211> 21
   <212> RNA
   <213> mouse
<400> 394
   uuccuaugca uauacuucuu u 21
<210> 395
   <211> 22
   <212> RNA
   <213> mouse
<400> 395
   caggccauac ugugcugccu ca 22
<210> 396
   <211> 22
   <212> RNA
   <213> mouse
<400> 396
   acugcauuac gagcacuuaa ag 22
<210> 397
   <211> 23
   <212> RNA
   <213> mouse
<400> 397
   ugcuaugcca acauauugcc auc 23
<210> 398
   <211> 22
   <212> RNA
   <213> mouse
<400> 398
   acugcugagc uagcacuucc cg 22
<210> 399
   <211> 21
   <212> RNA
   <213> mouse
<400> 399
   gaacggcguc augcaggagu u 21
<210> 400
   <211> 23
   <212> RNA
   <213> mouse
<400> 400
   ugagcgccuc ggcgacagag ccg 23
<210> 401
   <211> 22
   <212> RNA
   <213> mouse
<400> 401
   ccugaacuag gggucuggag ac 22
<210> 402
   <211> 22
   <212> RNA
   <213> mouse
<400> 402
   acugcagugu gagcacuucu ag 22
<210> 403
   <211> 22
   <212> RNA
   <213> mouse
<400> 403
   ggcugcagcg ugaucgccug cu 22
<210> 404
   <211> 21
   <212> RNA
   <213> mouse
<400> 404
   uguucagacu gguguccauc a 21
<210> 405
   <211> 22
   <212> RNA
   <213> mouse
<400> 405
   uaacugcaac agcucucagu au 22
<210> 406
   <211> 22
   <212> RNA
   <213> mouse
<400> 406
   uagugguuua caaaguaauu ca 22
<210> 407
   <211> 19
   <212> RNA
   <213> mouse
<400> 407
   acuugagggg caugaggau 19
<210> 408
   <211> 22
   <212> RNA
   <213> mouse
<400> 408
   auacauacac gcacacauaa ga 22
<210> 409
   <211> 22
   <212> RNA
   <213> mouse
<400> 409
   uaagugcgug cauguauaug ug 22
<210> 410
   <211> 23
   <212> RNA
   <213> mouse
<400> 410
   ugaagguccu acugugugcc agg 23
<210> 411
   <211> 22
   <212> RNA
   <213> mouse
<400> 411
   uacuccagaa uguggcaauc au 22
<210> 412
   <211> 22
   <212> RNA
   <213> mouse
<400> 412
   ugguaagcug cagaacaugu gu 22
<210> 413
   <211> 20
   <212> RNA
   <213> mouse
<400> 413
   aggcaguguu guuagcuggc 20
<210> 414
   <211> 21
   <212> RNA
   <213> mouse
<400> 414
   uaugcauaua cacgcaugca a 21
<210> 415
   <211> 22
   <212> RNA
   <213> mouse
<400> 415
   gagugcugga auuaaaggca ug 22
<210> 416
   <211> 23
   <212> RNA
   <213> mouse
<400> 416
   uaugugugug uguaugugug uaa 23
<210> 417
   <211> 24
   <212> RNA
   <213> mouse
<400> 417
   augcaugggu guauaguuga gugc 24
<210> 418
   <211> 23
   <212> RNA
   <213> mouse
<400> 418
   ugaguucgag gccagccugc uca 23
<210> 419
   <211> 22
   <212> RNA
   <213> mouse
<400> 419
   uauguguucc uggcuggcuu gg 22
<210> 420
   <211> 22
   <212> RNA
   <213> mouse
<400> 420
   cuuuggaugg agaaagaggg gg 22
<210> 421
   <211> 22
   <212> RNA
   <213> mouse
<400> 421
   caccaguccc accacgcggu ag 22
<210> 422
   <211> 22
   <212> RNA
   <213> mouse
<400> 422
   gagcagcaga ggaucuggag gu 22
<210> 423
   <211> 22
   <212> RNA
   <213> mouse
<400> 423
   gcaagggaga gggugaaggg ag 22
<210> 424
   <211> 24
   <212> RNA
   <213> mouse
<400> 424
   agucauggug uucggucuua guuu 24
<210> 425
   <211> 22
   <212> RNA
   <213> mouse
<400> 425
   aggacgagcu agcugagugc ug 22
<210> 426
   <211> 22
   <212> RNA
   <213> mouse
<400> 426
   uuuaggcaga gcacucguac ag 22
<210> 427
   <211> 22
   <212> RNA
   <213> mouse
<400> 427
   ccagugcugu uagaagaggg cu 22
<210> 428
   <211> 26
   <212> RNA
   <213> mouse
<400> 428
   gccgggcagu gguggcacau gcuuuu 26
<210> 429
   <211> 23
   <212> RNA
   <213> mouse
<400> 429
   ugugucacug gggauaggcu uug 23
<210> 430
   <211> 18
   <212> RNA
   <213> mouse
<400> 430
   guaaaggcug ggcugaga 18
<210> 431
   <211> 20
   <212> RNA
   <213> mouse
<400> 431
   uugggagggu ccuggggagg 20
<210> 432
   <211> 24
   <212> RNA
   <213> mouse
<400> 432
   agcugcgcug cuccugguaa cugc 24
<210> 433
   <211> 25
   <212> RNA
   <213> mouse
<400> 433
   aggagggagg ggaugggcca aguuc 25
<210> 434
   <211> 22
   <212> RNA
   <213> mouse
<400> 434
   uaggcuagag agagguuggg ga 22
<210> 435
   <211> 21
   <212> RNA
   <213> mouse
<400> 435
   uggcucauuu agaagcagcc a 21
<210> 436
   <211> 22
   <212> RNA
   <213> mouse
<400> 436
   gcugccuaua caugggcuuu cc 22
<210> 437
   <211> 24
   <212> RNA
   <213> mouse
<400> 437
   auuggagcug agauucugcg ggau 24
<210> 438
   <211> 22
   <212> RNA
   <213> mouse
<400> 438
   cuaccuuuga uaguccacug cc 22
<210> 439
   <211> 22
   <212> RNA
   <213> mouse
<400> 439
   ugaggaaucc ugaucucucg cc 22
<210> 440
   <211> 24
   <212> RNA
   <213> mouse
<400> 440
   uuggcaguca agauauuguu uagc 24
<210> 441
   <211> 22
   <212> RNA
   <213> mouse
<400> 441
   guggucacag uuggcgccag cc 22
<210> 442
   <211> 23
   <212> RNA
   <213> mouse
<400> 442
   uuugaucuga ugagcuaagc ugg 23
<210> 443
   <211> 22
   <212> RNA
   <213> mouse
<400> 443
   cacauggcac ucaacucugc ag 22
<210> 444
   <211> 22
   <212> RNA
   <213> mouse
<400> 444
   guugaagguu aauuagcaga gu 22
<210> 445
   <211> 22
   <212> RNA
   <213> mouse
<400> 445
   uacauacaca cauacacacg ca 22
<210> 446
   <211> 22
   <212> RNA
   <213> mouse
<400> 446
   gugugugcgu acauguacau gu 22
<210> 447
   <211> 22
   <212> RNA
   <213> mouse
<400> 447
   uauacaugag agcauacaua ga 22
<210> 448
   <211> 22
   <212> RNA
   <213> mouse
<400> 448
   aggggaaaau gccuuucucc ca 22
<210> 449
   <211> 21
   <212> RNA
   <213> mouse
<400> 449
   gaauggggcu guuuccccuc c 21
<210> 450
   <211> 18
   <212> RNA
   <213> mouse
<400> 450
   uggccaagga ugagaacu 18
<210> 451
   <211> 23
   <212> RNA
   <213> mouse
<400> 451
   cacagguggg aagugugugu cca 23
<210> 452
   <211> 22
   <212> RNA
   <213> mouse
<400> 452
   cucagaccuu ucuaccuguc ag 22
<210> 453
   <211> 22
   <212> RNA
   <213> mouse
<400> 453
   gugaguggcc aggguggggc ug 22
<210> 454
   <211> 21
   <212> RNA
   <213> mouse
<400> 454
   gguggugcag gcaggagagc c 21
<210> 455
   <211> 23
   <212> RNA
   <213> mouse
<400> 455
   gucucuaaag cuagacguuc cgg 23
<210> 456
   <211> 24
   <212> RNA
   <213> mouse
<400> 456
   aauggaugcg augguuccca ugcu 24
<210> 457
   <211> 20
   <212> RNA
   <213> mouse
<400> 457
   auaauacaac cugcuaagug 20
<210> 458
   <211> 22
   <212> RNA
   <213> mouse
<400> 458
   cacagaacau gcagugagaa cu 22
<210> 459
   <211> 22
   <212> RNA
   <213> mouse
<400> 459
   ugagauccaa cuguaaggca uu 22
<210> 460
   <211> 22
   <212> RNA
   <213> mouse
<400> 460
   ugggauuaaa ggcaugcacc ac 22
<210> 461
   <211> 22
   <212> RNA
   <213> mouse
<400> 461
   cccugggagg agacguggau uc 22
<210> 462
   <211> 23
   <212> RNA
   <213> mouse
<400> 462
   acggguauuc uuggguggau aau 23
<210> 463
   <211> 22
   <212> RNA
   <213> mouse
<400> 463
   ccugugaaau ucaguucuuc ag 22
<210> 464
   <211> 17
   <212> RNA
   <213> mouse
<400> 464
   uuugugacgu ugcagcu 17
<210> 465
   <211> 22
   <212> RNA
   <213> mouse
<400> 465
   caacgacauc aaaccaccug au 22
<210> 466
   <211> 23
   <212> RNA
   <213> mouse
<400> 466
   acauacuucu uuauguaccc aua 23
<210> 467
   <211> 22
   <212> RNA
   <213> mouse
<400> 467
   aggcggagac uugggcaauu gc 22
<210> 468
   <211> 25
   <212> RNA
   <213> mouse
<400> 468
   cugguuucac augguggcuu agauu 25
<210> 469
   <211> 23
   <212> RNA
   <213> mouse
<400> 469
   agguugggau uugucgcaau gcu 23
<210> 470
   <211> 19
   <212> RNA
   <213> mouse
<400> 470
   cucacugaac aaugaaugc 19
<210> 471
   <211> 22
   <212> RNA
   <213> mouse
<400> 471
   gcuggucaaa cggaaccaag uc 22
<210> 472
   <211> 22
   <212> RNA
   <213> mouse
<400> 472
   gaacauccug cauagugcug cc 22
<210> 473
   <211> 22
   <212> RNA
   <213> mouse
<400> 473
   ugaaaggugc cauacuaugu au 22
<210> 474
   <211> 22
   <212> RNA
   <213> mouse
<400> 474
   uggcgaacac agaauccaua cu 22
<210> 475
   <211> 21
   <212> RNA
   <213> mouse
<400> 475
   acuguugcua acaugcaacu c 21
<210> 476
   <211> 21
   <212> RNA
   <213> mouse
<400> 476
   ugguuauccc uguccucuuc g 21
<210> 477
   <211> 22
   <212> RNA
   <213> mouse
<400> 477
   uacacacaca cacacaagua aa 22
<210> 478
   <211> 23
   <212> RNA
   <213> mouse
<400> 478
   gaacaucaca gcaagucugu gcu 23
<210> 479
   <211> 20
   <212> RNA
   <213> mouse
<400> 479
   uaucuauuaa agaggcuagc 20
<210> 480
   <211> 21
   <212> RNA
   <213> mouse
<400> 480
   cccaccgggg gaugaauguc a 21
<210> 481
   <211> 19
   <212> RNA
   <213> mouse
<400> 481
   uacgcacgca cacacacac 19
<210> 482
   <211> 19
   <212> RNA
   <213> mouse
<400> 482
   uuguacaugg uaggcuuuc 19
<210> 483
   <211> 21
   <212> RNA
   <213> mouse
<400> 483
   uucacuggag uuuguuucag u 21
<210> 484
   <211> 21
   <212> RNA
   <213> mouse
<400> 484
   ugaauauaca cacacuuaca c 21
<210> 485
   <211> 21
   <212> RNA
   <213> mouse
<400> 485
   ugcggccggu gcucagucgg c 21
<210> 486
   <211> 22
   <212> RNA
   <213> mouse
<400> 486
   gcacugagcu agcucucccu cc 22
<210> 487
   <211> 21
   <212> RNA
   <213> mouse
<400> 487
   gagcauugca ugcugggaca u 21
<210> 488
   <211> 21
   <212> RNA
   <213> mouse
<400> 488
   cuggcugggg aaaaugacug g 21
<210> 489
   <211> 20
   <212> RNA
   <213> mouse
<400> 489
   auaagguaga aagcacuaaa 20
<210> 490
   <211> 20
   <212> RNA
   <213> mouse
<400> 490
   gggcuggaga gauggcucag 20
<210> 491
   <211> 22
   <212> RNA
   <213> mouse
<400> 491
   ucucuccagc ccccauaaua ag 22
<210> 492
   <211> 23
   <212> RNA
   <213> mouse
<400> 492
   uguugcggac caggggaauc cga 23
<210> 493
   <211> 19
   <212> RNA
   <213> mouse
<400> 493
   aagguuaggc cagccuggu 19
<210> 494
   <211> 23
   <212> RNA
   <213> mouse
<400> 494
   uuuggggcug uggugccacc age 23
<210> 495
   <211> 21
   <212> RNA
   <213> mouse
<400> 495
   agcuugugau gagacaucuc c 21
<210> 496
   <211> 22
   <212> RNA
   <213> mouse
<400> 496
   acuccugcau gacgccguuc cc 22
<210> 497
   <211> 22
   <212> RNA
   <213> mouse
<400> 497
   ugucucagaa aacaaccaag ga 22
<210> 498
   <211> 23
   <212> RNA
   <213> mouse
<400> 498
   ugcccugucu guucugccca cag 23
<210> 499
   <211> 22
   <212> RNA
   <213> mouse
<400> 499
   aacuugugau gaggugagac ag 22
<210> 500
   <211> 21
   <212> RNA
   <213> mouse
<400> 500
   caggcggccu cagcucucac u 21
<210> 501
   <211> 22
   <212> RNA
   <213> mouse
<400> 501
   auggucacag uggacaucaa cc 22
<210> 502
   <211> 22
   <212> RNA
   <213> mouse
<400> 502
   ucuugggaca uaguguaagg ca 22
<210> 503
   <211> 22
   <212> RNA
   <213> mouse
<400> 503
   cagcucaugg agaccuaggu gg 22
<210> 504
   <211> 24
   <212> RNA
   <213> mouse
<400> 504
   aguugugugu gcauguucau gucu 24
<210> 505
   <211> 22
   <212> RNA
   <213> mouse
<400> 505
   ugaugugugu guacauguac au 22
<210> 506
   <211> 20
   <212> RNA
   <213> mouse
<400> 506
   caggcuucug gcuauauucc 20
<210> 507
   <211> 23
   <212> RNA
   <213> mouse
<400> 507
   uuuggcacua gcacauuuuu gcu 23
<210> 508
   <211> 23
   <212> RNA
   <213> mouse
<400> 508
   uaauacugcc ggguaaugau gga 23
<210> 509
   <211> 22
   <212> RNA
   <213> mouse
<400> 509
   uaaucucagc uggcaacugu ga 22
<210> 510
   <211> 22
   <212> RNA
   <213> mouse
<400> 510
   gcagcagggu gaaacugaca ca 22
<210> 511
   <211> 22
   <212> RNA
   <213> mouse
<400> 511
   acugcccuaa gugcuccuuc ug 22
<210> 512
   <211> 23
   <212> RNA
   <213> mouse
<400> 512
   ugugugugua cauguacaug uga 23
<210> 513
   <211> 22
   <212> RNA
   <213> mouse
<400> 513
   auaagugugu gcauguauau gu 22
<210> 514
   <211> 25
   <212> RNA
   <213> mouse
<400> 514
   agucccagga ugcacugcag cuuuu 25
<210> 515
   <211> 26
   <212> RNA
   <213> mouse
<400> 515
   uggguuaaag gauuuaccug aggcca 26
<210> 516
   <211> 22
   <212> RNA
   <213> mouse
<400> 516
   auaaagcuag auaaccgaaa gu 22
<210> 517
   <211> 22
   <212> RNA
   <213> mouse
<400> 517
   acuccauuug uuuugaugau gg 22
<210> 518
   <211> 23
   <212> RNA
   <213> mouse
<400> 518
   uuaaugcuaa uugugauagg ggu 23
<210> 519
   <211> 22
   <212> RNA
   <213> mouse
<400> 519
   aacuggccua caaaguccca gu 22
<210> 520
   <211> 22
   <212> RNA
   <213> mouse
<400> 520
   uucccuuugu cauccuaugc cu 22
<210> 521
   <211> 21
   <212> RNA
   <213> mouse
<400> 521
   ugagaugaag cacuguagcu c 21
<210> 522
   <211> 22
   <212> RNA
   <213> mouse
<400> 522
   ugagguagua gguuguaugg uu 22
<210> 523
   <211> 22
   <212> RNA
   <213> mouse
<400> 523
   ugagguagga gguuguauag uu 22
<210> 524
   <211> 22
   <212> RNA
   <213> mouse
<400> 524
   uagcaccauc ugaaaucggu ua 22
<210> 525
   <211> 22
   <212> RNA
   <213> mouse
<400> 525
   uggcaguguc uuagcugguu gu 22
<210> 526
   <211> 22
   <212> RNA
   <213> mouse
<400> 526
   aaaagcuggg uugagagggc ga 22
<210> 527
   <211> 21
   <212> RNA
   <213> mouse
<400> 527
   ugguagacua uggaacguag g 21
<210> 528
   <211> 22
   <212> RNA
   <213> mouse
<400> 528
   uagguaguuu ccuguuguug gg 22
<210> 529
   <211> 22
   <212> RNA
   <213> mouse
<400> 529
   ucaggcucag uccccucccg au 22
<210> 530
   <211> 16
   <212> RNA
   <213> mouse
<400> 530
   augguggcac ggaguc 16
<210> 531
   <211> 21
   <212> RNA
   <213> mouse
<400> 531
   cccagauaau agcacucuca a 21
<210> 532
   <211> 18
   <212> RNA
   <213> mouse
<400> 532
   gcgugugcuu gcuguggg 18
<210> 533
   <211> 18
   <212> RNA
   <213> mouse
<400> 533
   aucucgcugg ggccucca 18
<210> 534
   <211> 21
   <212> RNA
   <213> mouse
<400> 534
   aacauccugg uccuguggag a 21
<210> 535
   <211> 19
   <212> RNA
   <213> mouse
<400> 535
   ugcacugaag gcacacagc 19
<210> 536
   <211> 22
   <212> RNA
   <213> mouse
<400> 536
   aauccuuugu cccuggguga aa 22
<210> 537
   <211> 22
   <212> RNA
   <213> mouse
<400> 537
   agcgggcaca gcugugagag cc 22
<210> 538
   <211> 19
   <212> RNA
   <213> mouse
<400> 538
   ggugcucaca uguccuccu 19
<210> 539
   <211> 22
   <212> RNA
   <213> mouse
<400> 539
   ugugaguugu uccucaccug ga 22
<210> 540
   <211> 22
   <212> RNA
   <213> mouse
<400> 540
   auuccuggaa auacuguucu ug 22
<210> 541
   <211> 22
   <212> RNA
   <213> mouse
<400> 541
   acucaaaaug gaggcccuau cu 22
<210> 542
   <211> 22
   <212> RNA
   <213> mouse
<400> 542
   uaugugggac gguaaaccgc uu 22
<210> 543
   <211> 22
   <212> RNA
   <213> mouse
<400> 543
   acuuaaacgu gguuguacuu gc 22
<210> 544
   <211> 22
   <212> RNA
   <213> mouse
<400> 544
   ucucugggcc ugugucuuag gc 22
<210> 545
   <211> 22
   <212> RNA
   <213> mouse
<400> 545
   uuauaaagca augagacuga uu 22
<210> 546
   <211> 22
   <212> RNA
   <213> mouse
<400> 546
   uggagacgcg gcccuguugg ag 22
<210> 547
   <211> 22
   <212> RNA
   <213> mouse
<400> 547
   aacacaccug uucaaggauu ca 22
<210> 548
   <211> 23
   <212> RNA
   <213> mouse
<400> 548
   agguuacccg agcaacuuug cau 23
<210> 549
   <211> 21
   <212> RNA
   <213> mouse
<400> 549
   uccgguucuc agggcuccac c 21
<210> 550
   <211> 22
   <212> RNA
   <213> mouse
<400> 550
   aacugugucu uuucugaaua ga 22
<210> 551
   <211> 22
   <212> RNA
   <213> mouse
<400> 551
   auguaugugu gcauguacau gu 22
<210> 552
   <211> 22
   <212> RNA
   <213> mouse
<400> 552
   ugugugcaug ugcuugugug ua 22
<210> 553
   <211> 22
   <212> RNA
   <213> mouse
<400> 553
   uaagugcgcg cauguauaug cg 22
<210> 554
   <211> 20
   <212> RNA
   <213> mouse
<400> 554
   auguauaaau guauacacac 20
<210> 555
   <211> 22
   <212> RNA
   <213> mouse
<400> 555
   ugaacuauug caguagccuc cu 22
<210> 556
   <211> 22
   <212> RNA
   <213> mouse
<400> 556
   acuugugugu gcauguauau gu 22
<210> 557
   <211> 22
   <212> RNA
   <213> mouse
<400> 557
   ugucuugugu gugcauguuc au 22
<210> 558
   <211> 21
   <212> RNA
   <213> mouse
<400> 558
   uaggacacau ggucuacuuc u 21
<210> 559
   <211> 24
   <212> RNA
   <213> mouse
<400> 559
   agggagaugc ugguacagag gcuu 24
<210> 560
   <211> 22
   <212> RNA
   <213> mouse
<400> 560
   ugggaaaguu cucaggcuuc ug 22
<210> 561
   <211> 23
   <212> RNA
   <213> mouse
<400> 561
   ugggacgaga ucaugaggcc uuc 23
<210> 562
   <211> 25
   <212> RNA
   <213> mouse
<400> 562
   aagggagcug gcucaggaga gaguc 25
<210> 563
   <211> 22
   <212> RNA
   <213> mouse
<400> 563
   acgcccuucc cccccuucuu ca 22
<210> 564
   <211> 21
   <212> RNA
   <213> mouse
<400> 564
   uuccugucag ccgugggugc c 21
<210> 565
   <211> 24
   <212> RNA
   <213> mouse
<400> 565
   agucaggcug cuggcuauac acca 24
<210> 566
   <211> 22
   <212> RNA
   <213> mouse
<400> 566
   ccauagcaca gaagcacucc ca 22
<210> 567
   <211> 22
   <212> RNA
   <213> mouse
<400> 567
   cagugggccg ugaaagguag cc 22
<210> 568
   <211> 22
   <212> RNA
   <213> mouse
<400> 568
   ucaacaaaau cacugaugcu gg 22
<210> 569
   <211> 22
   <212> RNA
   <213> mouse
<400> 569
   guuccugcug aacugagcca gu 22
<210> 570
   <211> 22
   <212> RNA
   <213> mouse
<400> 570
   auauacauac acacccauau ac 22
<210> 571
   <211> 22
   <212> RNA
   <213> mouse
<400> 571
   uguggacacc gugggagguu gg 22
<210> 572
   <211> 21
   <212> RNA
   <213> mouse
<400> 572
   cugugacaca cccgcuccca g 21
<210> 573
   <211> 21
   <212> RNA
   <213> mouse
<400> 573
   agucaggcuc cuggcaggag u 21
<210> 574
   <211> 21
   <212> RNA
   <213> mouse
<400> 574
   cucuacuccc ugccccagcc a 21
<210> 575
   <211> 23
   <212> RNA
   <213> mouse
<400> 575
   acgcucugcu uugcuccccc aga 23
<210> 576
   <211> 20
   <212> RNA
   <213> mouse
<400> 576
   cggggcagcu caguacagga 20
<210> 577
   <211> 21
   <212> RNA
   <213> mouse
<400> 577
   acauagaaaa ggcagucugc a 21
<210> 578
   <211> 22
   <212> RNA
   <213> mouse
<400> 578
   gcucuuuuca cauugugcua cu 22
<210> 579
   <211> 22
   <212> RNA
   <213> mouse
<400> 579
   aaccguggcu uucgauuguu ac 22
<210> 580
   <211> 22
   <212> RNA
   <213> mouse
<400> 580
   aggcuacaac acaggacccg gg 22
<210> 581
   <211> 21
   <212> RNA
   <213> mouse
<400> 581
   cuguacagcc uccuagcuuu c 21
<210> 582
   <211> 21
   <212> RNA
   <213> mouse
<400> 582
   ggucaagagg cgccugggaa c 21
<210> 583
   <211> 21
   <212> RNA
   <213> mouse
<400> 583
   ucugucauuc uguaggccaa u 21
<210> 584
   <211> 23
   <212> RNA
   <213> mouse
<400> 584
   uggucgacca gcuggaaagu aau 23
<210> 585
   <211> 22
   <212> RNA
   <213> mouse
<400> 585
   ucaucacgug gugacgcaac au 22
<210> 586
   <211> 23
   <212> RNA
   <213> mouse
<400> 586
   ccccucaggc caccagagcc cgg 23
<210> 587
   <211> 21
   <212> RNA
   <213> mouse
<400> 587
   caaaccacac ugugguguua g 21
<210> 588
   <211> 22
   <212> RNA
   <213> mouse
<400> 588
   uaaggcuccu uccugugcuu gc 22
<210> 589
   <211> 23
   <212> RNA
   <213> mouse
<400> 589
   ugacuggcac cauucuggau aau 23
<210> 590
   <211> 22
   <212> RNA
   <213> mouse
<400> 590
   cggccccacg caccagggua ag 22
<210> 591
   <211> 22
   <212> RNA
   <213> mouse
<400> 591
   agggagagca gggcaggguu uc 22
<210> 592
   <211> 25
   <212> RNA
   <213> mouse
<400> 592
   ugugcaugug uguauaguug ugugc 25
<210> 593
   <211> 20
   <212> RNA
   <213> mouse
<400> 593
   ugugugugug ugugugugug 20
<210> 594
   <211> 20
   <212> RNA
   <213> mouse
<400> 594
   ugguagaccg gugacguaca 20
<210> 595
   <211> 21
   <212> RNA
   <213> mouse
<400> 595
   guuacauggu gaagcccagu u 21
<210> 596
   <211> 23
   <212> RNA
   <213> mouse
<400> 596
   aggucuguag cucaguuggc aga 23
<210> 597
   <211> 22
   <212> RNA
   <213> mouse
<400> 597
   acuggagacg gaagcugcaa ga 22
<210> 598
   <211> 21
   <212> RNA
   <213> mouse
<400> 598
   uuggcagaaa gggcagcugu g 21
<210> 599
   <211> 23
   <212> RNA
   <213> mouse
<400> 599
   ucugggcaga gcugcaggag aga 23
<210> 600
   <211> 23
   <212> RNA
   <213> mouse
<400> 600
   guaagugagg gcaagccuuc ugg 23
<210> 601
   <211> 23
   <212> RNA
   <213> mouse
<400> 601
   aggagguccu ggggccgccc uga 23
<210> 602
   <211> 21
   <212> RNA
   <213> mouse
<400> 602
   ugggcccucc agaccucaug c 21
<210> 603
   <211> 24
   <212> RNA
   <213> mouse
<400> 603
   uggcagcugg caagucagaa ugca 24
<210> 604
   <211> 22
   <212> RNA
   <213> mouse
<400> 604
   cuggcgccaa cugugaccac ug 22
<210> 605
   <211> 22
   <212> RNA
   <213> mouse
<400> 605
   ugagguagua gauuguauag uu 22
<210> 606
   <211> 22
   <212> RNA
   <213> mouse
<400> 606
   gugccuacug agcugaaaca gu 22
<210> 607
   <211> 22
   <212> RNA
   <213> mouse
<400> 607
   uggcucaguu cagcaggaac ag 22
<210> 608
   <211> 22
   <212> RNA
   <213> mouse
<400> 608
   gaggaacuag ccuucucuca gc 22
<210> 609
   <211> 23
   <212> RNA
   <213> mouse
<400> 609
   uggaagacuu gugauuuugu ugu 23
<210> 610
   <211> 29
   <212> DNA
   <213> artificial
<220>
   <223> primer
<400> 610
   ttggatccag ggcctgtatg tgtgaatga 29
<210> 611
   <211> 32
   <212> DNA
   <213> artificial
<220>
   <223> primer
<400> 611
   ttttgctagc acacttgtgc tttagaagtt gc 32
<210> 612
   <211> 32
   <212> DNA
   <213> artificial
<220>
   <223> primer
<400> 612
   ttggatccaa aattactcag cccatgtagt tg 32
<210> 613
   <211> 30
   <212> DNA
   <213> artificial
<220>
   <223> primer
<400> 613
   ttttgctagc ttagccagag aagtgcaacc 30
<210> 614
   <211> 30
   <212> DNA
   <213> artificial
<220>
   <223> primer
<400> 614
   ttggatccat gtgtcggaga agtggtcatc 30
<210> 615
   <211> 32
   <212> DNA
   <213> artificial
<220>
   <223> primer
<400> 615
   ttttgctagc ctccaaagga agagaggcag tt 32
<210> 616
   <211> 23
   <212> DNA
   <213> artificial
<220>
   <223> primer
<400> 616
   tgtaaacatc ctcgactgga agc 23
<210> 617
   <211> 22
   <212> DNA
   <213> artificial
<220>
   <223> primer
<400> 617
   tgtaaacatc ctacactcag ct 22
<210> 618
   <211> 23
   <212> DNA
   <213> artificial
<220>
   <223> primer
<400> 618
   tgtaaacatc ctacactctc agc 23
<210> 619
   <211> 22
   <212> DNA
   <213> artificial
<220>
   <223> primer
<400> 619
   ctttcagtca gatgtttgct gc 22
<210> 620
   <211> 23
   <212> DNA
   <213> artificial
<220>
   <223> primer
<400> 620
   tgtaaacatc cttgactgga agc 23
<210> 621
   <211> 17
   <212> DNA
   <213> artificial
<220>
   <223> primer
<400> 621
   ctcgcttcgg cagcaca 17
<210> 622
   <211> 20
   <212> DNA
   <213> artificial
<220>
   <223> primer
<400> 622
   aacgcttcac gaatttgcgt 20